# EUROPEAN PATENT SPECIFICATION

(11) **EP 0 876 494 B1**
(45) Date of publication and mention of the grant of the patent: **05.03.2008**
(21) Application number: 97901640.9
(22) Date of filing: 24.01.1997
(51) Int. Cl.: C12N 15/80, C12N 9/24

(54) **PRODUCTION AND SECRETION OF ACTINOMYCETE XYLANASES IN A FILAMENTOUS TRICHODERMA FUNGUS**
HERSTELLUNG UND SEKRETION VON XYLANASEN VON ACTINOMYCETEN IN FILAMENTÖSEN TRICHODERMA PILZEN
PRODUCTION ET SECRETION DE XYLANASES D'ACTINOMYCETE DANS DES CHAMPIGNONS FILAMENTEUX DE TRICHODERMA

(30) Priority: 26.01.1996 US 590563
(43) Date of publication of application: 11.11.1998
(73) Proprietor: AB Enzymes GmbH, 64293 Darmstadt (DE)
(72) Inventor: MÄNTYLÄ, Arja, FIN-00400 Helsinki (FI); PALOHEIMO, Marja, FIN-01450 Vantaa (FI); LANTTO, Raija, FIN-01800 Klaukkala (FI); FAGERSTRÖM, Richard, FIN-02260 Espoo (FI); LAHTINEN, Tarja, FIN-01620 Vantaa (FI); SUOMINEN, Pirkko, Maple Grove, Minnesota 55311 (US); VEHMAANPERÄ, Jari, FIN-01800 Klaukkala (FI)
(74) Representative: Lönnqvist, Gunnel Solveig Kristina
(86) International application number: PCT/FI1997/000037
(87) International publication number: WO 1997/027306

(56) References cited:
- EP-A- 0 215 594
- EP-A- 0 238 023
- WO-A-89/01969
- WO-A-95/12668
- WO-A1-90/15860
- CA-A- 2 129 171
- DIALOG INFORMATION SERVICES, File 154, MEDLINE, Dialog Accession No. 07407541, Medline Accession No. 91207693, PUNT P.J. et al., "Intracellular and Extracellular Production of Proteins in Aspergillus under the Control of Expression Signals of the Highly Expressed Aspergillus Nidulans gdpA Gene"; & J. BIOTECHNOL. (NETHERLANDS), Jan. 1991, 17(1), p19-33.
- NYYSSÖNEN E. ET AL: 'Efficient production of antibody fragments by the filamentous fungus Trichoderma reesei' BIOTECHNOLOGY vol. 11, May 1993, pages 591 - 595
- VAN DEN HONDEL C. ET AL: 'Development of a system for analysis of regulation signals in Aspergillus', 1985, ALAN R. LISS, INC.
- DATABASE EMBL [Online] U08894, 05 May 1994 DERY C. ET AL: 'Actinomadura sp. FC7 xylanase II gene'
- HOLTZ C. ET AL: 'Production and properties of xylanases from thermophilic actinomycetes' ANTONIE VAN LEEUWENHOEK vol. 59, 1991, pages 1 - 7
- PALOHEIMO ET AL: 'High-yield production of a becterial xylanase in the filamentous fungus Trichoderma reesei requires a carrier polypeptide with an intact domain structure' APPL. ENVIRONMENT. MICROBIOL. vol. 69, no. 12, December 2003, pages 7073 - 7082
- GWYNNE ET AL: 'Genetically engineered secretion of active human interferon and a bacterial endoglucanase from Aspergillus nidulans' BIOTECHNOLOGY vol. 5, July 1987, pages 713 - 719, XP009061601
- NYYSSÖNEN ET AL: 'Protein production by the filamentous fungus Trichoderma reesei: secretion of active antibody molecules' CAN. J. BOT. vol. 73, no. 1, 1995, pages S885 - S890, XP000575039
- GOUKA ET AL: 'Efficient production of secreted proteins by Aspergillus: progress, limitations and prospects' APPL. MICROBIOL. BIOTECHNOL vol. 47, January 1997, pages 1 - 11

## Description

The present invention provides recombinant expression vectors for producing actinomycete xylanases in filamentous fungi *Trichoderma,* by transforming said filamentous fungus hosts with an expression vector containing a DNA sequence encoding one or more functional domains of a secretable fungal protein, wherein the functional domain is a tail domain of a secretable fungal cellulase or a core and hinge domain of a secretable fungal mannanase derived from the host *Trichoderma.* Also provided are hosts transformed with said expression vectors. Said transformed hosts produce xylanase preparations useful e.g. for modifying plant biomass properties, especially to reduce the lignin content in enzyme aided bleaching.

The aim of kraft pulp bleaching is to remove the residual lignin that is left in pulp after kraft cooking. Traditionally, this has been done by using chlorine-containing chemicals. Due to environmental concerns and consumer demands, alternative bleaching technologies have been desired.

The first biotechnical approach to this problem was to attack the lignin directly with lignin degrading enzymes. However, the chemistry of enzymatic lignin degradation seems to be very complicated and difficult to control.

Lignin can be degraded, if the whole microorganism that produces ligninolytic enzymes is used. However, treatment times are relatively long. For example, treatment times may take days, and the microorganisms need supplemental nutrients to work. It can also be difficult to control the growth of other, undesired, microbes. The use of lignin degradation by isolated ligninolytic enzymes or by microorganisms is the subject of much research. (see, for example, Farrell, R.L. et al., Lignocellulosics 305-315 (1992); Jurasek, L., Lignocellulosics 317-325 (1992)).

In addition to cellulose and lignin, wood pulp contains hemicellulose. Another approach to reduce the lignin content of pulp is to attack hemicellulose - the third main component of wood. The hemicellulose in native hardwood is mainly xylan, while in softwood the hemicellulose is mainly glucomannans and some xylan. During kraft cooking, part of the xylan is dissolved into the cooking liquor. Towards the end of the cooking period when the alkali concentration decreases, part of the dissolved and modified xylan reprecipitates back onto the cellulose fibre.

In 1986, it was noticed that xylanase pretreatment of unbleached kraft pulp results in a lessened need for chemicals in the bleaching process (Viikari, L. et al.. Proceedings of the 3rd Int. Conf. on Biotechnology in the Pulp Paper Ind., Stockholm (1986), pp. 67-69). Xylanase pretreatment of kraft pulp partially hydrolyses the xylan in kraft pulp. The mechanism of how hydrolysis of xylan results in better lignin removal is not fully understood. One frequently suggested possibility is that the pulp structure becomes more porous and this enables more efficient removal of lignin fragments in the subsequent bleaching and extraction stages. Also hydrolysis of the xylan located in the inner parts of the fibre and possibly linked to lignin may have a role. Later, in several laboratories, the xylanase pretreatment was reported to be useful in conjunction with bleaching sequences consisting of Cl₂, ClO₂, H₂O₂, O₂ and O₃. See reviews in Viikari, L. et al., FEMS Microbiol. Rev. 13: 335-350 (1994); Viikari, L. et al., in: Saddler, J.N., ed., Bioconversion of Forest and Agricultural Plant Residues, C-A-B International (1993), pp. 131-182; Grant, R., Pulp and Paper Int. (Sept. 1993), pp. 56-57; Senior & Hamilton, J. Pulp & Paper :111-114 (Sept. 1992); Bajpai & Bajpai, Process Biochem. 27:319-325 (1992); Onysko, A., Biotech. Adv. 11:179-198 (1993); and Viikari, L. et al., J. Paper and Timber 73:384-389 (1991).

As a direct result of the better bleachability of the pulp after such a xylanase treatment, there is a reduction of the subsequent consumption of bleaching chemicals, which when chlorine containing chemicals are used, leads to a reduced formation of environmentally undesired organo-chlorine compounds. Also as a direct result of the better bleachability of pulp after a xylanase treatment, it is possible to produce a product with a higher final brightness where such brightness would otherwise be hard to achieve (such as totally chlorine free (TCF) bleaching using peroxide). Because of the substrate specificity of the xylanase enzyme, cellulose fibers are not harmed and the strength properties of the product are well within acceptable limits.

A xylanase that is active at an alkaline pH would decrease the need to acidify the pulp prior to xylanase treatment. In addition, the temperatures of many modem kraft cooking and bleaching processes are relatively high, well above the 50 °C that is suitable for many of the commercial bleaching enzymes. Accordingly, a need exists for thermostable xylanase preparations that are stable at alkaline pH values for use in wood pulp bleaching processes.

It is known that actinomycetes, e.g. (*Microtetraspora flexuosa* ATCC35864 and *Thermomonospora fusca* KW3, produce thermostable and alkaline stable xylanases (US 5,437,992 and EP 473 545. The cloning of xylanases has been reported from several bacteria (e.g. Ghangas, G.S. et al., J. Bacteriol. 171:2963-2969 (1989); Lin, L.-L., Thomson, J.A., Mol. Gen. Genet. 228:55-61 (1991); Shareck, F. et al., Gene 107:75-82 (1991); Scheirlinck, T. et al., Appl Microbiol Biotechnol. 33:534-541 (1990); Whitehead, T.R., Lee, D.A., Curr. Microbiol. 23:15-19 (1991)); and also from *Actinomadura sp.* FC7 (Ethier, J.-F. et al., in: Industrial Microorganisms: Basic and Applied Molecular Genetics, R. Baltz et al., Eds, (Proc. 5th ASM Conf. Gen. Mol. Biol. Indust. Microorg., Oct 11-15, 1992, Bloomington, Indiana, poster C25)). It has been proposed by some researchers that the former genus *Actinomadura* should be divided into two genera. *Actinomadura* and *Microtetraspora,* the latter including, e.g. the former *A. flexuosa* (Kroppenstedt et al., System. Appl. Microbiol. 13: 148-160 (1990).

The use of hemicellulose hydrolyzing enzymes in different bleaching sequences is discussed in WO 89/08738, EP 383 999, WO 91/02791, EP 395 792, EP 386 888, EP 473 545, EP 489 104 and WO 91/05908, WO 95/34662, WO 95/18219, WO 95/27779, WO 95/34662, WO 95/18219, WO 92/04664 and WO 92/03540. The use of hemicellulolytic enzymes for improved water removal from mechanical pulp is discussed in EP 262 040, EP 334 739 and EP 351 655, DE 4,000,558, WO 92/04664, WO 92/03540. WO 94/21785 and EP 513 140. When the hydrolysis of biomass to liquid fuels or chemicals is considered, the conversion of both cellulose and hemicellulose is essential to obtain a high yield (Viikari et al., "Hemicellulases for Industrial Applications," In: Bioconversion of Forest and Agricultural Wastes, Saddler, J., ed., CAB International, USA (1993)). Also, in the feed industry, there is a need to use a suitable combination of enzyme activities to degrade the high β-glucan and hemicellulose containing substrate.

The efficient and cost-effective production of thermostable xylanases is a problem, because thermostable xylanases originate mainly from relatively unstudied bacteria, which often produce only minimal or very small amounts of xylanase. Further, there is little or no experience of cultivating these microbes in a fermentor or no fermentation processes available. Furthermore, these microbes may be unsuitable for industrial scale production. On the other hand, filamentous fungi like *Aspergillus* and *Trichoderma* are known to produce large quantities of proteins, on an industrial scale. In particular, these fungi have been shown to be suitable for production of homologous or heterologous proteins of fungal origin.

There are very few reports related to the production of proteins or enzymes of bacterial origin in filamentous fungi: the production of endoglucanase from *Cellulomonas fimi* (Gwynne et al. , Bio/Technology 5: 713-719 (1987); and β-glucuronidase from *E. coli* (Punt et al. , J. Biotechnol. 17: 19-34 (1991) have been reported in *A. nidulans.* Of these enzymes, endoglucanase was secreted into the culture medium by *Aspergillus nidulans* in the range of 10-15 mg protein per liter. β-glucuronidase was only detectable intracellularly.

Many of the studies on heterologous gene expression have concerned mammalian genes (van den Hondel et al., Heterologus gene expression in filamentous fungi, Ed. Bennett and Lasure. More Gene Manipulations in Fungi Academic Press, San Diego, U.S.A., pp. 396-428 (1991); Gouka et al., efficient production of secreted proteins by Aspergillus: progress, limitations and prospects, Appl. Microbiol. Biotechnol. 47:1-11(1997) . So far, the initial yields of eucaryotic enzymes in filamentous fungi have been in a range of tens of mg per liter in shake flask cultivations. In the International patent publication WO 90/15860 secretion of chymosin by *A. niger* var. *awamori* was described using a fusion to the homologous glucoamylase gene. Nyyssönen et al., Bio/Technology 11: 591-595 (1993) and Nyyssönen et al., Can. J. Bot. 73 (Suppl.1): S885-S890 describe the production of antibody fragments in *Trichoderma reesei.* The best yield of antibody fragments when produced as a fusion to the cellobiohydrolase 1 gene of *T. reesei* was in the range of 40 mg per liter in a shake flask cultivation.

The invention is directed *inter alia,* to recombinant expression vectors for producing, i.e. expressing and secreting xylanases originating from bacteria, especially from actinomycete. The main purpose of the invention is to provide an alternative expression vector for producing xylanases originating from actinomycete in filamentous fungi, *Trichoderma.*

The characteristics of the present invention are as set forth in the claims.

The present invention is related to a recombinant expression vector for production of bacterial proteins or enzymes, especially actinomycete xylanases in a filamentous fungal host *Trichoderma,* most preferably in *T. reesei.* Said vector comprises a promoter operably linked to a DNA sequence encoding one or more functional domains of a filamentous fungus secretable protein, preferably a tail domain of a cellulase or a core and hinge domain of a mannanase derived from *Trichoderma,* which in turn frames the DNA sequence, e.g. SEQ ID NO: 1: or SEQ ID NO: 3:, or equivalents thereof, which encode a bacterial protein, preferably an actinomycetous xylanase, most preferably *Actinomadura flexuosa* 35 kD (AM35) or 50 kD (AM50) xylanase, i.e. SEQ ID NO: 2: or SEQ ID NO: 4: or equivalents thereof.

Also provided are peptide sequences of *A*. *flexuosa* 35 kDa and 50 kDa protein used for identifying and characterizing the protein sequences. Said peptides are assigned SEQ ID NO: 6:, SEQ ID NO: 7:, SEQ ID NO: 8:, SEQ ID NO: 9: and SEQ ID NO: 10:.

The preferred promoters of the present invention are promoters of a filamentous fungus secretable protein, most preferably a *T. reesei cbh1* promoter.

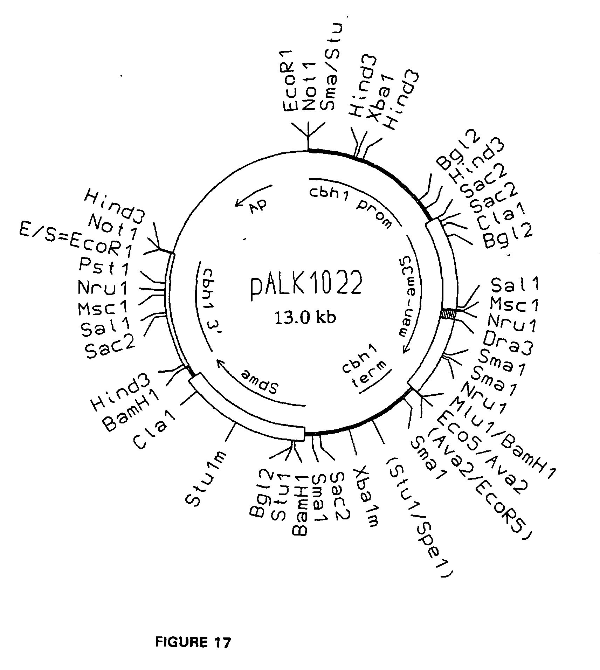

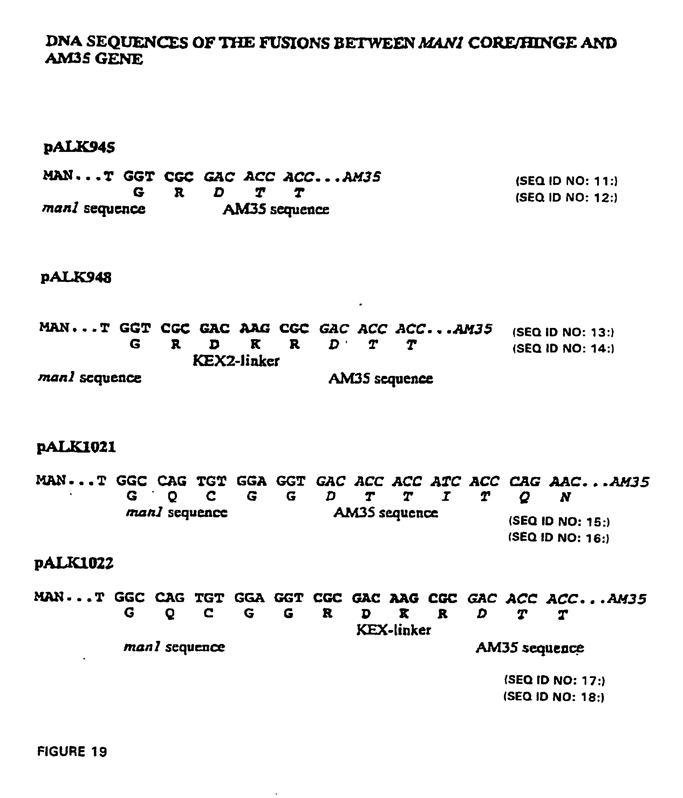

Examples of recombinant expression vector are the plasmids pALK945, pALK948, pALK1021 and pALK1022, which are constructed as shown in figure 17 and contain SEQ ID NO: 11:, SEQ ID NO: 13:, SEQ ID NO: 15: and SEQ ID NO: 17:, respectively and which encode the amino acid sequences comprising SEQ ID NO: 12:, SEQ ID NO: 14:, SEQ ID NO: 16:, and SEQ ID NO: 18: as shown in Figure 19.

All the proteins comprising SEQ ID NO: 12:, SEQ ID NO: 14:, SEQ ID NO: 16:, and SEQ ID NO: 18: have a N-terminal DNA sequence SEQ ID NO: 5:, which is identical with the N-terminal sequence of the wild type *A. flexuosa* xylanase (D-T-T-I-T-Q).

The present invention further provides preparation containing one or more proteins or enzymes, preferably xylanases originating from bacteria, especially from actinomycetes in an essentially cell-free culture medium, which can be subjected to suitable down-stream processing methods.

The preparation containing enzymes, especially xylanases originating from actinomycetes are obtainable by cultivating hosts transformed with the expression vectors of the present invention. These preparations are useful e.g. for enzyme-aided bleaching, because the enzymes are stable at processing temperatures, when the processing temperatures are in the ranges 50-90 °C, preferably 60-85 °C, most preferably 70-80 °C.

Especially, said preparations are useful for treating paper making pulp and for enzymatical treating of plant biomass.

It is therefore the main object of this invention to produce more efficiently xylanases of bacterial origin in filamentous fungi such as *Trichoderma.* Preferably, the host is *Trichoderma reesei* and the xylanases originate from actinomycetes.
Figure 1 shows the effect of pH on *Actinomadura flexuosa* DSM43186 xylanase activity (culture supernatant) at 50 °C.
Figures 2A, 2B and 2C show the effect of temperature on *A. flexuosa* DSM43186 xylanase activity (culture supernatant) at pH values 6.9, 7.8 and 9.0 and at time points of 0, 30, 60 and 120 minutes. The highest xylanase activity of the whole experiment is described as 100 % and all other activity values are proportional to it.

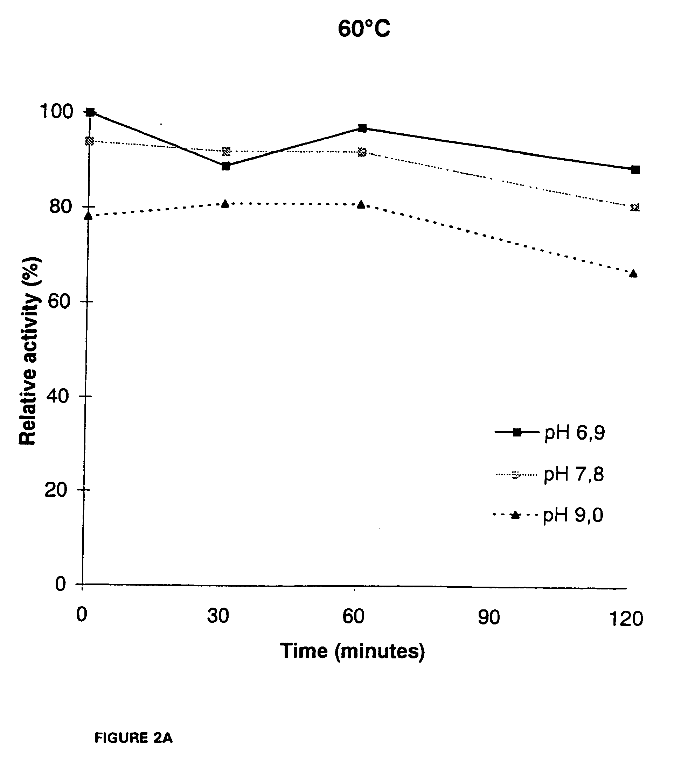

Figure 2A shows the activities in a temperature of 60 °C.

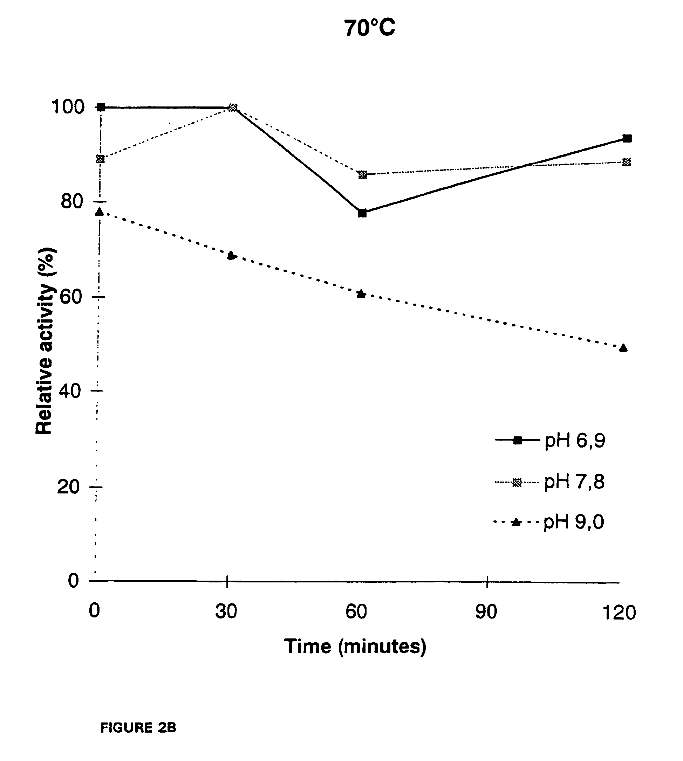

Figure 2B shows the activities in a temperature of 70 °C.

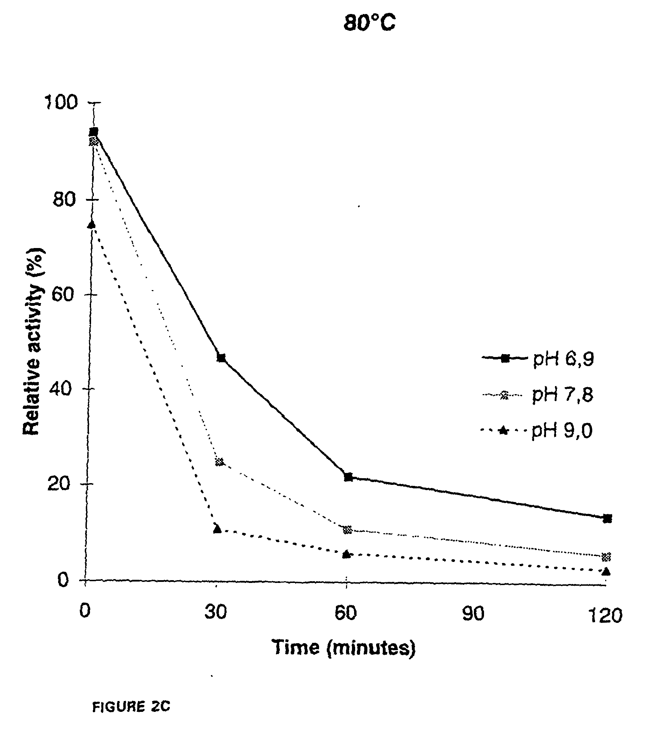

Figure 2C shows the activities in a temperature of 80 °C.

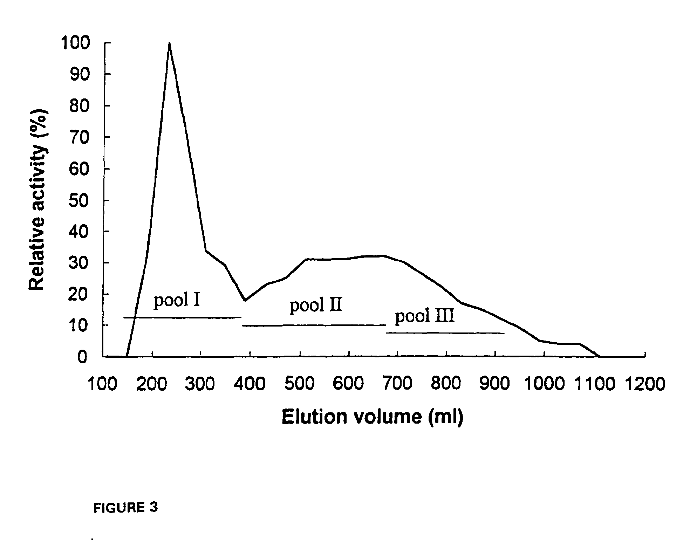

Figure 3 shows the DEAE Sepharose CL-6B chromatography elution profile of *A. flexuosa* DSM43186 xylanases.

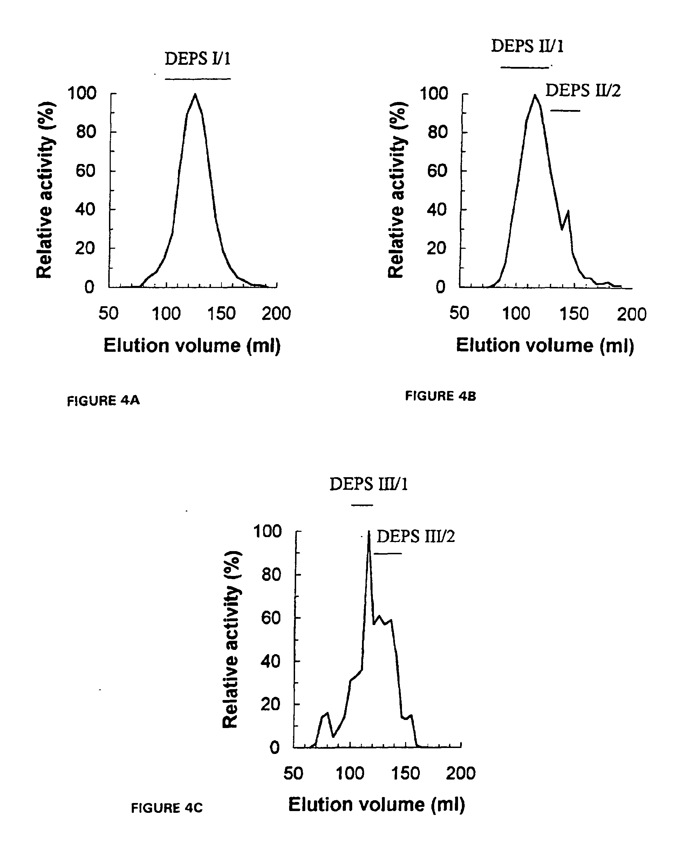

Figure 4A shows the Phenyl Sepharose CL-4B chromatography elution profile of DEAE pool I of Figure 3. The fractions that were combined to provide sample DEPS 1/1 are indicated.
Figure 4B shows the Phenyl Sepharose CL-4B chromatography elution profile of DEAE pool II of Figure 3. The fractions that were combined to provide sample DEPS II/1 and DEPS II/2 are indicated.
Figure 4C shows the Phenyl Sepharose CL-4B chromatography elution profile of DEAE pool III of Figure 3. The fractions that were combined to provide sample DEPS III/1 and DEPS III/2 are indicated.

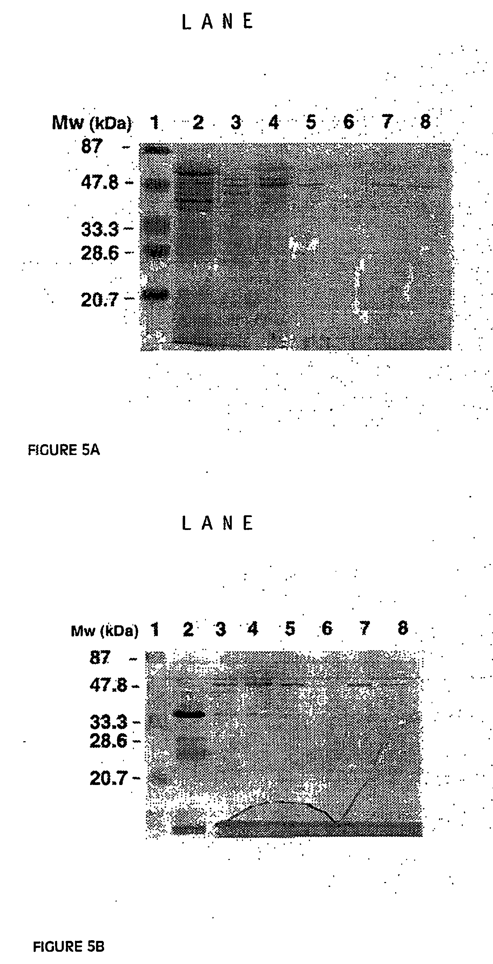

Figure 5A shows the Coomassie Brilliant Blue protein staining pattern of the various chromatographic pools. Lane 1: molecular weight markers; lane 2: medium; lane 3: DEPS (Pool I/1); lanes 4 and 5: DEPS (Pool II/1 and II/2, respectively); lane 6: empty; lanes 7 and 8: DEPS (Pool III/1 and III/2, respectively). DEPS: Fractions after the DEAE chromatography shown in Figure 3 and the Phenyl Sepharose chromatography shown in Figures 4A-4C.
Figure 5B shows the Western blot analysis of the various chromatographic pools stained in Figure 5A. Polyclonal antiserum raised against the *Thermomonospora fusca* XynA xylanase was used for detection. Lane 1: molecular weight markers; lane 2: medium; lane 3: DEPS (Pool I/1); lanes 4 and 5: DEPS (Pool II/1 and II/2, respectively); lane 6: empty; lanes 7 and 8: DEPS (Pool III/1 and III/2, respectively). DEPS: Fractions after the DEAE chromatography shown in Figure 3 and the Phenyl Sepharose chromatography shown in Figures 4A-4C.

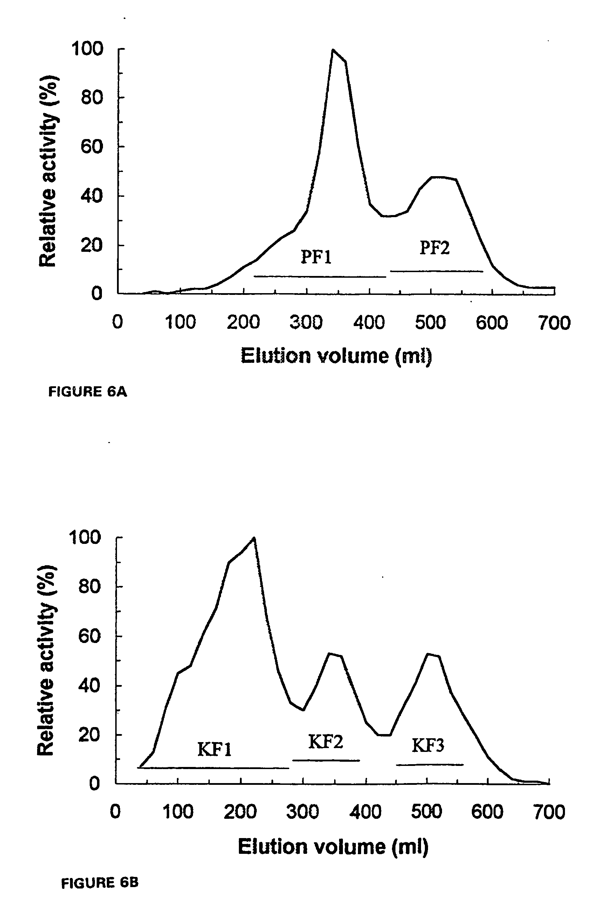

Figure 6A shows the Phenyl Sepharose FF chromatography elution profile of DEAE flow through permeate. The tubes that were combined to provide sample PF1 and PF2 are indicated.
Figure 6B shows the Phenyl Sepharose FF chromatography elution profile of DEAE flow-through concentrate. The tubes that were combined to provide sample KF1, KF2 and KF3 are indicated.

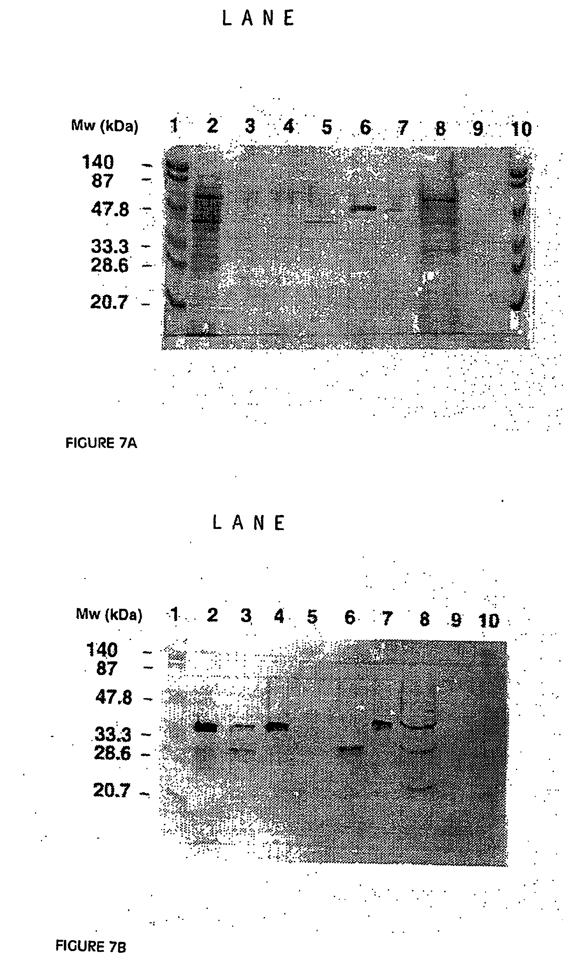

Figure 7A shows the Coomassie Blue protein staining pattern of the various chromatographic pools. Abbreviations are as in Figures 6A and 6B. Lanes 1 and 10: molecular weight markers; lane 2: medium; lane 3: PF1; lane 4: PF2; lane 5: KF1; lane 6: KF2; lane 7: KF3; Lane 8: DEAE flow-through concentrate; Lane 9: Empty.
Figure 7B shows the Western blot analysis of the various chromatographic pools stained for protein in Figure 7A. Polyclonal antiserum raised against the *T. fusca* XynA xylanase was used for detection. Abbreviations are as in Figures 6A and 6B. Lanes 1 and 10: molecular weight markers; lane 2: medium: lane 3: PF1; lane 4: PF2; lane 5: KF1; lane 6: KF2; lane 7: KF3. Lane 8: DEAE flow-through concentrate; Lane 9: empty.

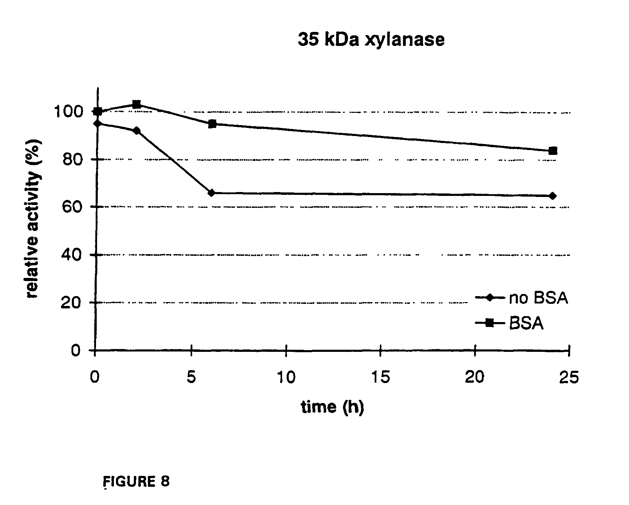

Figure 8 shows the thermostability of purified *A. flexuosa* 35 kDa xylanase (AM35) at 70 °C, pH 6 with and without added bovine serum albumin (BSA). The enzyme sample was incubated in Mcllvain's buffer +/-BSA (100 µg/ml). Samples were collected after 0. 2. 6, and 24 hours of incubation and enzyme activity assay was done at pH 6.5, 60 °C.

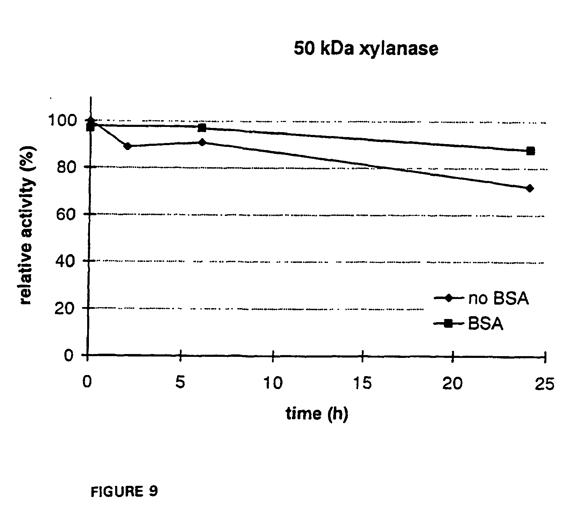

Figure 9 shows the thermostability of purified *A. flexuosa* 50 kDa xylanase (AM50) at 70 °C, pH 6 with and without added BSA. The enzyme sample was incubated in Mcllvain's buffer +/- BSA (100 µg/ml). Samples were collected after 0, 2, 6 and 24 hours of incubation and enzyme activity assay was done at pH 6.5, 60°C.

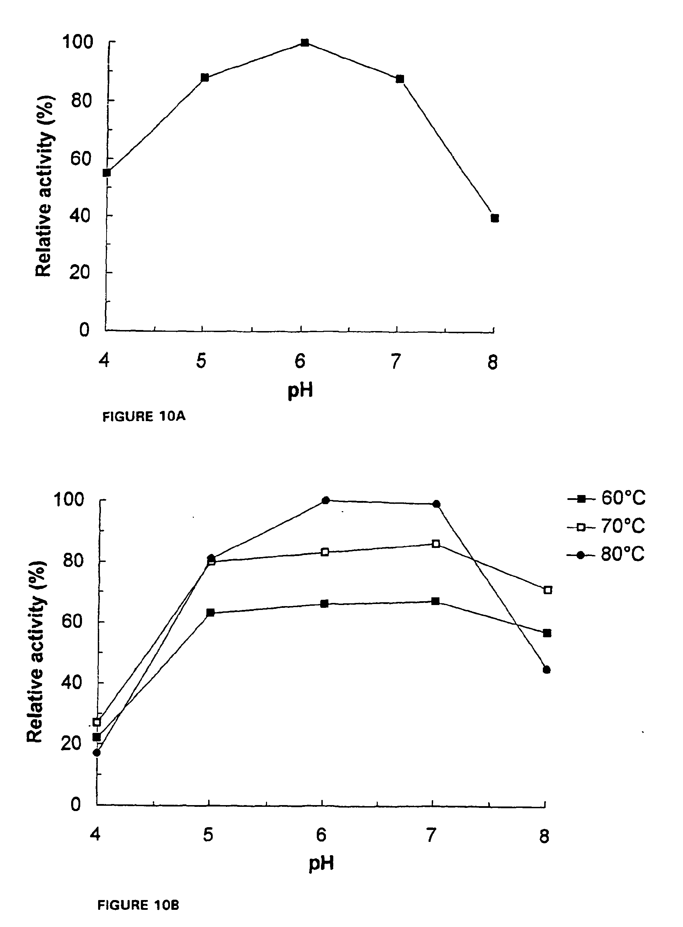

Figure 10A shows the effect of pH on the activity of the 35 kDa xylanase at 80 °C.
Figure 10B shows the effect of pH on the activity of the 50 kDa xylanase at 60 °C, (closed squares), 70 °C (open squares) and 80 °C (closed circles).

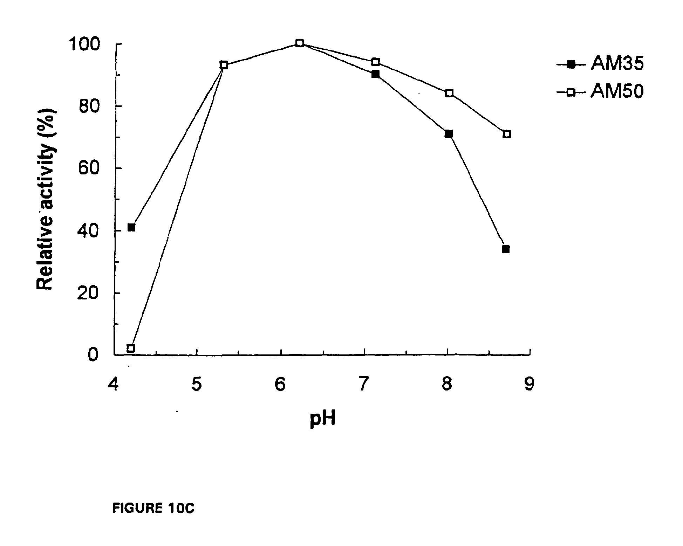

Figure 10C shows the effect of pH on the activity of the 35 kDa (closed squares) and the 50 kDa (open squares) xylanases at 60 °C with 60 minutes incubations.

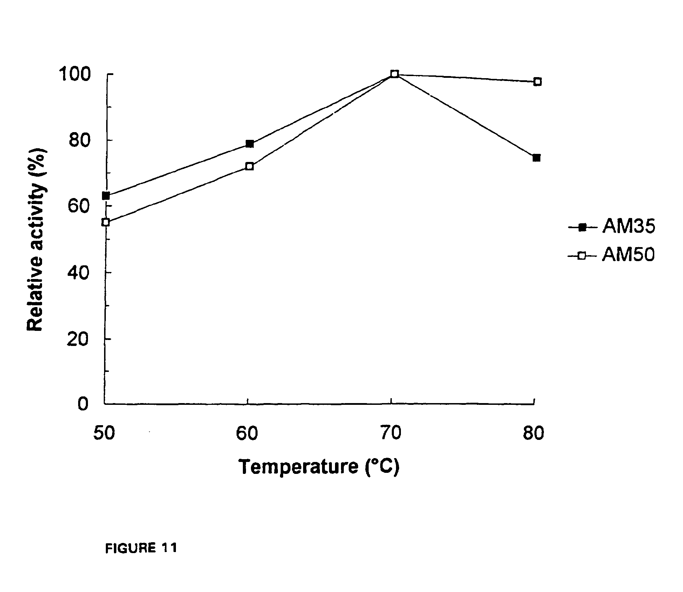

Figure 11 shows the effect of temperature on the activity of the 35 kDa (closed squares) and the 50 kDa (open squares) at pH 7 with 60 minutes incubations.

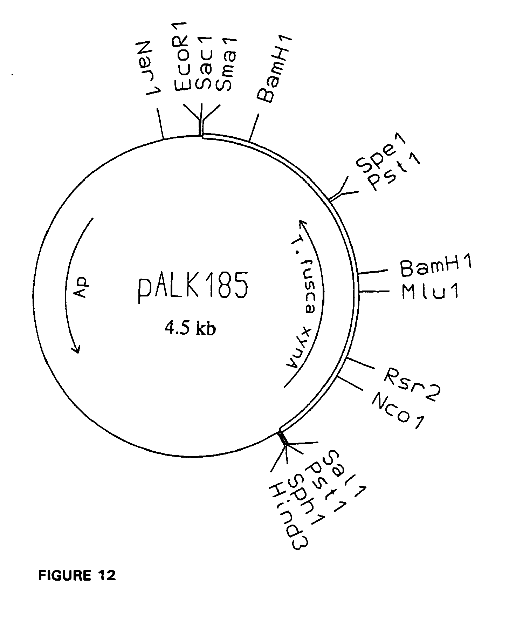

Figure 12 is a map of plasmid pALK185 (4.5 kb), containing *T. fusca* xylanase gene (*xyn*A).

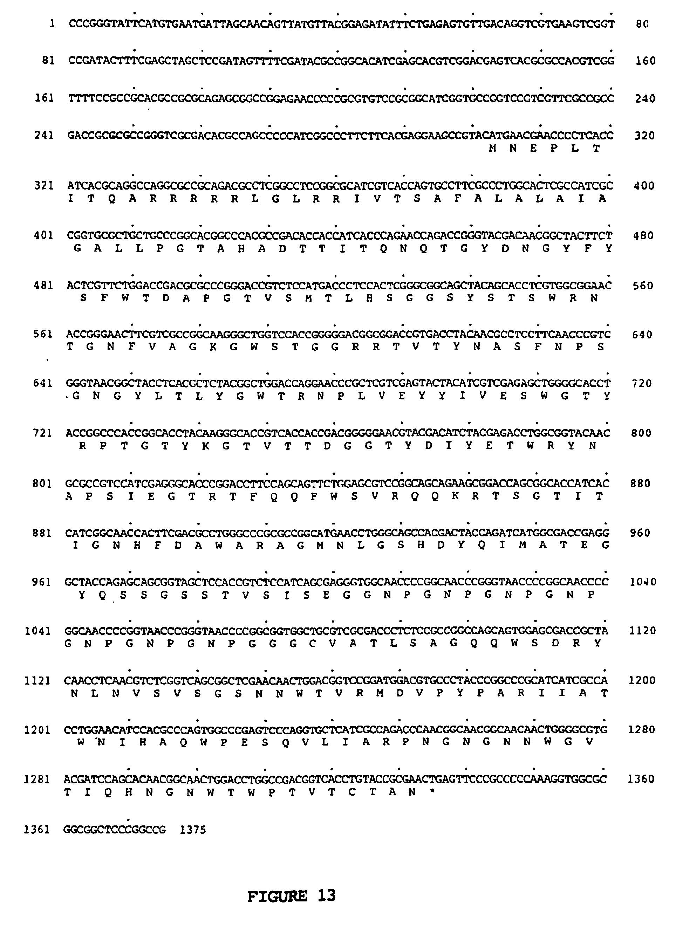

Figure 13 shows the DNA sequence and the amino acid sequence of 1375 bps of *A. flexuosa* DSM43186 35 kDa xylanase.

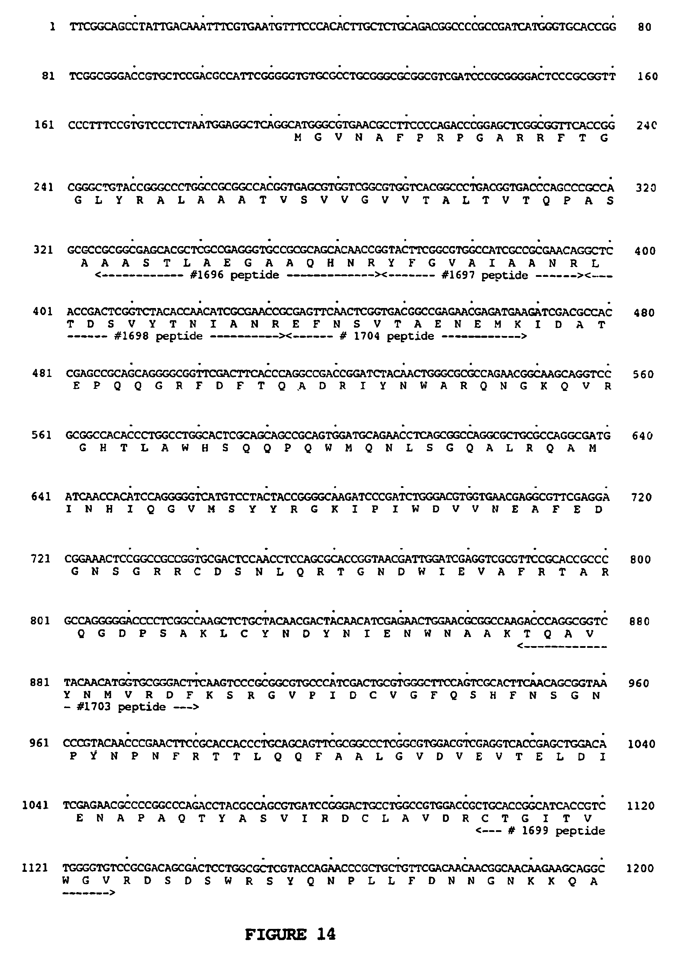

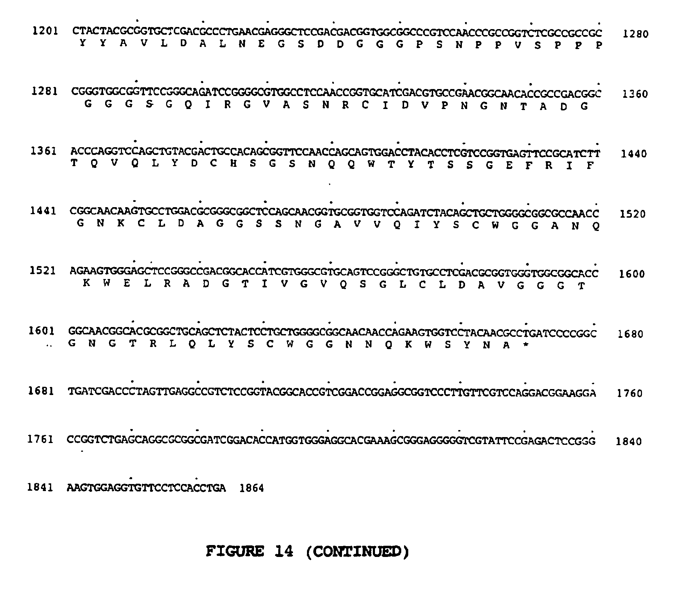

Figure 14 shows the DNA sequence and amino acid sequence of 1864 bps of *A. flexuosa* DSM43186 50 kDa xylanase.

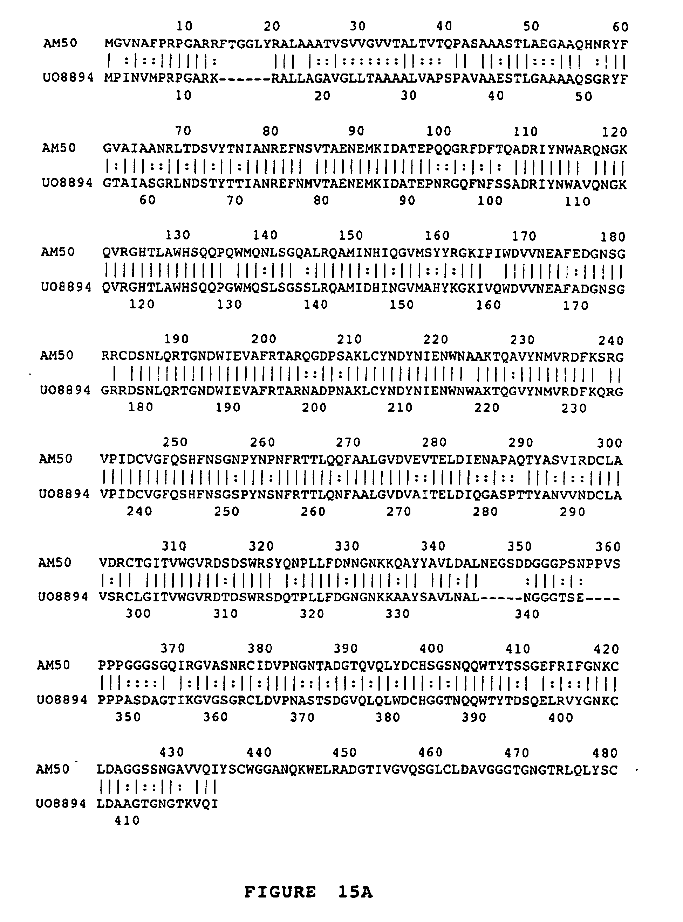

Figure 15A shows a homology comparison at the amino acid level between the AM50-peptide derived from the 1864 bps insert and the *Actinomadura* sp. FC7 xylanase II (accession no. U08894) gene. The figure shows that there was 70.7% identity in a 434 amino acid overlap.

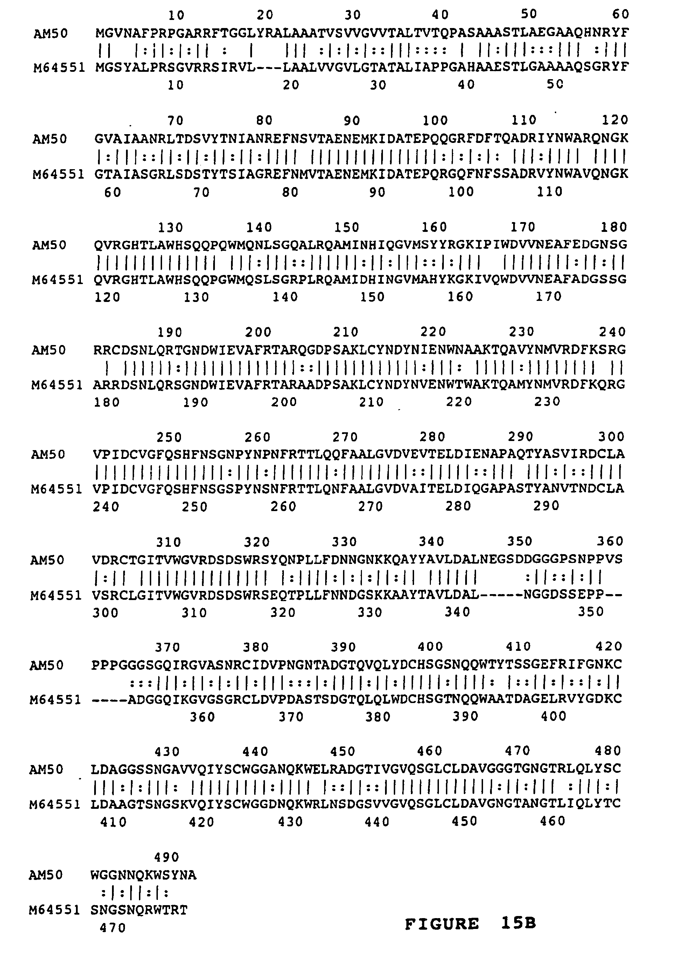

Figure 15B shows a homology comparison at the amino acid level between the AM50-peptide derived from the 1864 bps insert and the *Streptomyces lividans* xylanase A *(xlnA)* gene (accession no. M64551). The figure shows that there was 70.3% identity in a 489 amino acid overlap.

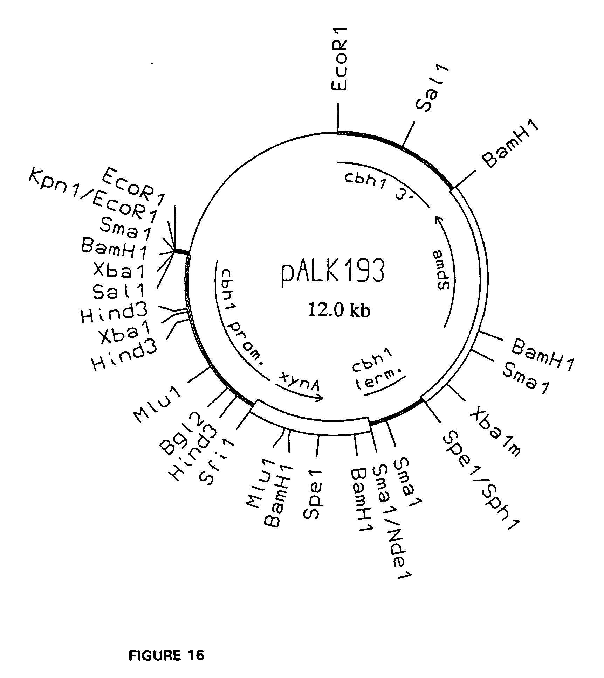

Figure 16 shows the expression cassette pALK193 (12 kb) containing the expression cassette for production of *T. fusca* xylanase XynA in *T. reesei.*
Figure 17 is a map of plasmid pALK1022 (13 kb) containing the expression cassette for production of *A. flexuosa* 35 kDa xylanase in *T. reesei.*

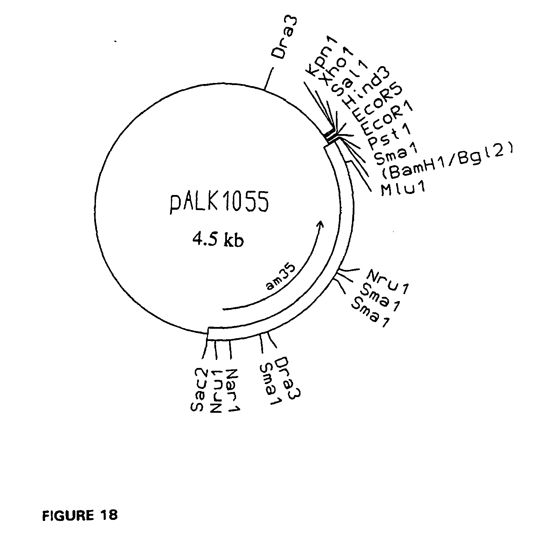

Figure 18 is a map of plasmid pALK1055 (4.5 kb) containing the gene for *A. flexuosa* 35 kDa xylanase.
Figure 19 shows the DNA sequence SEQ ID NO: 11:, SEQ ID NO: 13:. SEQ ID NO: 15: and SEQ ID NO: 17 as well as the corresponding amino acid sequences SEQ ID NO: 12:, SEQ ID NO: 14:, SEQ ID NO: 16: and SEQ ID NO: 18:, which comprise the fusions between the *man1* core/hinge and the *am35* gene for pALK945, pALK948, pALK1021 and pALK1022.

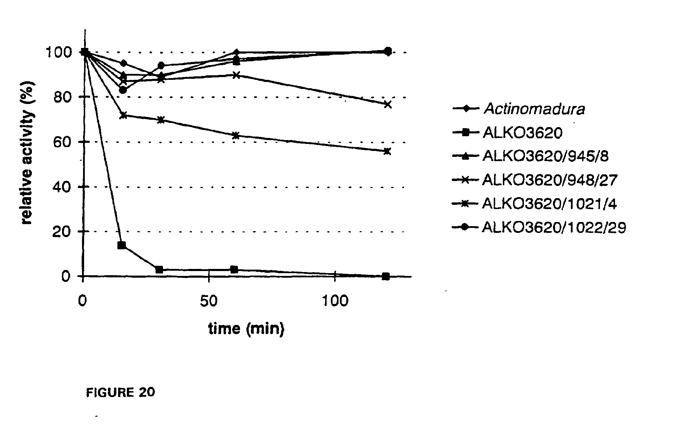

Figure 20 shows the thermal stability of xylanase activity from culture supernatants of a *A. flexuosa* DSM43186 and chosen *T. reesei* transformants producing *A. flexuosa* 35 kDa xylanase. Samples from the culture supernatants were incubated at 70 °C, pH 7 in McIIvain's buffer. (BSA was added to 100 µg/ml) for 0, 15, 30, 60 and 120 minutes after which xylanase activities from the sample were determined at 70°C, pH 7 (5 minutes incubation).

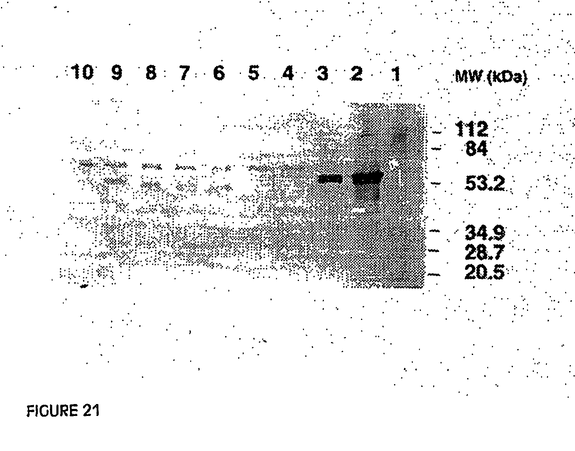

Figure 21 shows a Western blot analysis of culture supernatants from transformants producing *A. flexuosa* AM35 xylanase. Polyclonal antiserum raised against the purified β-mannanase (pI 5.4) of *T. reesei* RutC30 was used for detection. Lane 1: prestained low molecular weight marker (Bio-Rad, U.S.A.); Lanes 2-3: purified 53 kDa β-mannanase protein sample; Lane 4: culture medium of MANI core producing strain, ALKO 3620/pALK1010/24. Lane 5: culture medium of the transformation host strain ALKO3620; Lanes 6-10: Culture media of the transformants ALKO3620/pALK945/8, ALKO3620/pALK945/6, ALKO3620/pALK948/7, ALKO3620/pALK1022/29 and ALKO3620/pALK1021/4, respectively.

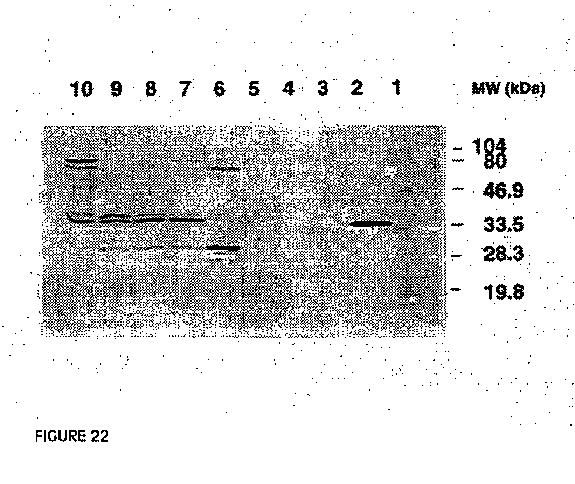

Figure 22 shows a Western blot analysis of culture supernatants from transformants producing *A. flexuosa* xylanase. Polyclonal antiserum raised against the purified 35 kDa xylanase of *A. flexuosa* was used for detection. Lane 1: prestained low molecular weight marker (Bio-Rad, U.S.A.); Lane 2: purified 35 kDa xylanase of *A. fleruosa* DSM43186; Lane 3: 53 kDa β-mannanase protein sample; Lane 4: culture medium of the MANI core producing strain, ALKO3620/pALK1010/24; Lane 5: culture medium of the transformation host ALKO3620; Lanes 6-10: culture media of the transformants ALKO3620/pALK945/8, ALK03620/pALK945/6, ALKO3620/pALK948/27, ALKO3620/pALK1022/29 and ALKO3620/pALK1021/4, respectively.

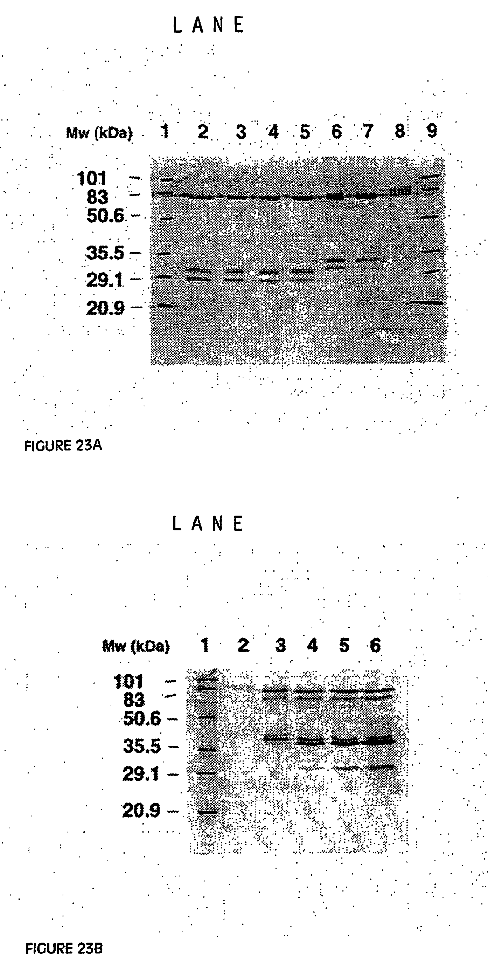

Figure 23A: Western-blot of growth medium samples from a Fed-batch fermentation of ALKO3620/pALK945/8. Polyclonal antibody raised against the purified *A. flexuosa 35* kDa xylanase. Lane 1 and 9: prestained molecular mass standards (LMW, Bio-Rad); Lane 3 to 8: growth medium samples after 7, 6, 5, 4, 3 and 2 days fermentation times, respectively; Lane 2: final sample after 7.3 days fermentation.
Figure 23B: Western-blot of growth medium samples from a laboratory fermentation of ALKO3620/pALK945/6. Polyclonal antibody raised against the purified *A. flexuosa* 35 kDa xylanase. Lane 1: prestained molecular mass standards (LMW, Bio-Rad); Lane 2 to 6: growth medium samples after 1, 2, 3, 4 and 5 days fermentation times, respectively.

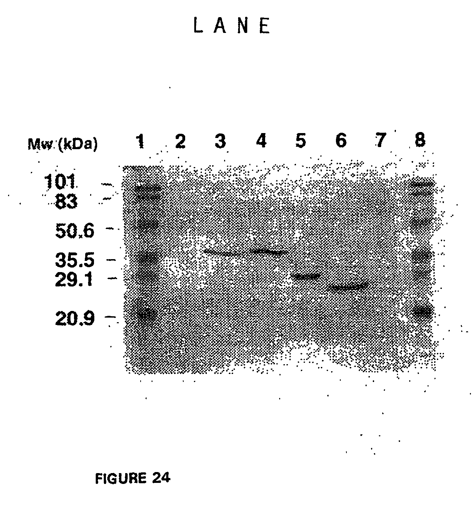

Figure 24: SDS-PAGE of purified recombinant *A. flexuosa* 35 kDa xylanases and wild-type *A. flexuosa* 35 kDa xylanase. Lanes 1 and 8: Prestained molecular mass standards (Bio-Rad); Lane 3: purified wild-type 35 kDa xylanase; Lane 4: purified 33.4 kDa xylanase; Lane 5: purified 23.8 kDa xylanase; Lane 6: purified 22 kDa xylanase; Lane 2 and 7: empty.

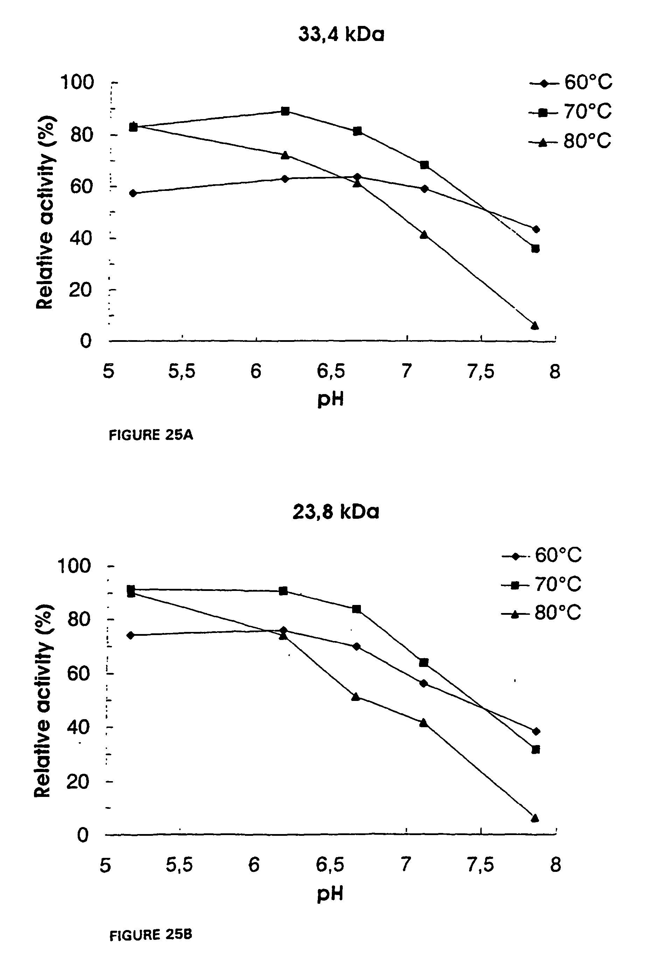

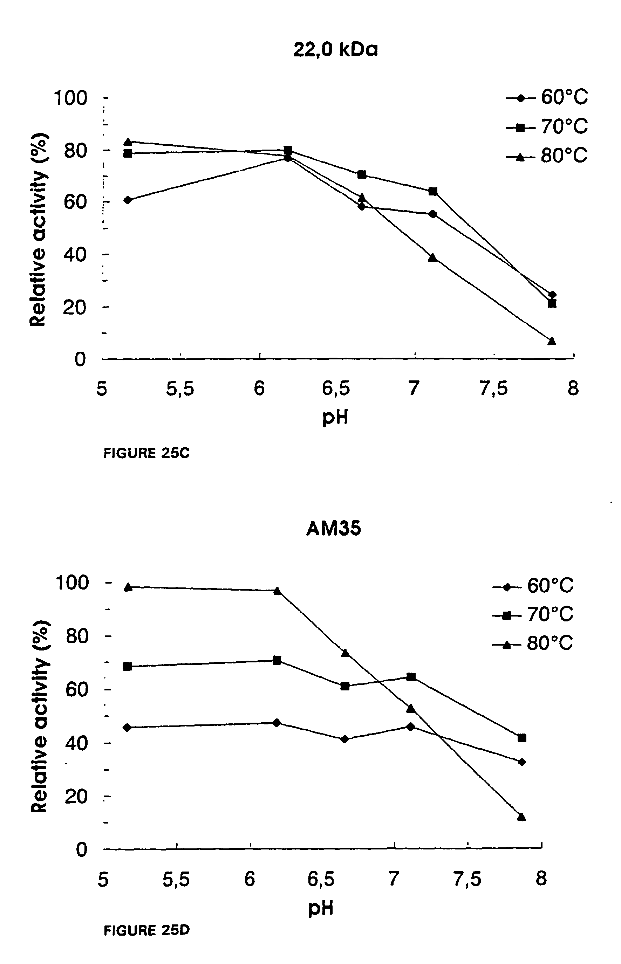

Figure 25: Temperature and pH dependence of purified recombinant and wild-type *A. flexuosa* 35 kDa xylanases. Incubations were performed at temperatures and pH values indicated for 60 min with 1 % (w/v) birch xylan (Roth 7500) as substrate.

### Deposits

Plasmid pALK923, pALK938, pALK939, pALK940, pALK941 and pALK1056 were deposited at the Deutsche Sammlung von Mikroorganismen und Zellkulturen GmbH, Mascheroder Weg 1 b, D-38124 Braunschweig, Germany assigned accession numbers DSM9322, DSM9899, DSM9900, DSM9901, DSM9902 and DSM9903, respectively. pALK923 was deposited on July 27, 1994, and pALK938-941 and pALK1056 were deposited on April 3, 1995.

Plasmids pALK927 and pALK928 were deposited at the DSM on September 27, 1994, and assigned accession numbers DSM9447 and DSM9448. respectively.

### Detailed Description of the Preferred Embodiments

### I. Definitions

In the description that follows, a number of terms used in recombinant DNA technology are extensively utilized. In order to provide a clearer and consistent understanding of the specification and claims, including the scope to be given such terms, the following definitions are provided.

***Cloning vehicle.*** A plasmid or phage DNA or other DNA sequence (such as a linear DNA) which provides an appropriate nucleic acid environment for the transfer of a gene of interest into a host cell. The cloning vehicles of the invention may be designed to replicate autonomously in prokaryotic and eukaryotic hosts. In fungal hosts such as *Trichoderma,* the cloning vehicles generally do not autonomously replicate and instead, merely provide a vehicle for the transport of the gene of interest into the *Trichoderma* host for subsequent insertion into the *Trichoderma* genome. The cloning vehicle may be further characterized by one or a small number of endonuclease recognition sites at which such DNA sequences may be cut in a determinable fashion without loss of an essential biological function of the vehicle, and into which DNA may be spliced in order to bring about replication and cloning of such DNA. The cloning vehicle may further contain a marker suitable for use in the identification of cells transformed with the cloning vehicle. Suitable marker genes are for example antibiotic resistance providing marker genes, but other useful markers exist as well, e.g. biocide resistance providing genes as well as heavy metals, such as copper resistance providing genes. Alternatively, such markers may be provided on a cloning vehicle which is separate from that supplying the gene of interest using the so called cotransformation system. The word "vector" is sometimes used for "cloning vehicle. "

***Expression vehicle.*** An expression vehicle or vector is similar to a cloning vehicle but it is capable of expressing a gene of interest, after transformation into a desired host. When a fungal host is used, the gene of interest is preferably provided to a fungal host as part of a cloning or expression vehicle that integrates into the fungal chromosome. Sequences which derive from the cloning vehicle or expression vehicle may also be integrated with the gene of interest during the integration process. For example, in *T. reesei,* the gene of interest can be directed to the *cbh1* locus.

The gene of interest may preferably be placed under the control of (i.e., operably linked to) certain control sequences such as promoter sequences provided by the vector (which integrate with the gene of interest). If desired, such control sequences may be provided by the host's chromosome as a result of the locus of insertion.

Expression control sequences on an expression vector will vary depending on whether the vector is designed to express a certain gene in a prokaryotic or eukaryotic host (for example, a shuttle vector, may provide a gene for selection in different microbial hosts) and may additionally contain transcriptional elements such as, enhancer elements, termination sequences, and/or translational initiation and termination sites.

***Homologous**.* By an enzyme *"**homologous**"* to a host of the invention is meant that an untransformed strain of the same species as the host species naturally produces some amount of the native protein; by a gene "homologous" to a host of the invention is meant a gene found in the genome of an untransformed strain of the same species as the host species. By an enzyme *"**heterologous**"* to a host of the invention is meant that an untransformed strain of the same species as the host species does not naturally produce some amount of the native protein; by a gene "heterologous" to a host of the invention is meant a gene not found in the genome of an untransformed strain of the same species as the host species.

***Xylanase.*** As used herein, a xylanase is a hemicellulase that cuts the β-1,4 bonds within the xylosic chain of xylan, (xylan is a polymer of D-xylose residues that are joined through β-1,4 linkages). Xylanase activity is synonymous with xylanolytic activity. More specifically xylanolytic activity means an activity similar with or identical to the xylanolytic activity of *A. flexuosa* 35 kDa (AM35) and *A.flexuosa* 50 kDa (AM50), the characteristics, e.g. the thermostability are more specifically described and defined in the detailed description and examples.

By an amino acid sequence that is an *"**equivalent**"* of a specific amino acid sequence is meant an amino acid sequence that is not identical to the specific amino acid sequence, but rather contains at least some amino acid changes (deletion, substitutions, inversions, insertions, etc.) that do not essentially affect the biological activity of the protein as compared to a similar activity of the specific amino acid sequence, when used for a desired purpose. Preferably, an "equivalent" amino acid sequence contains at least 85%-99% identity at the amino acid level to the specific amino acid sequence, most preferably at least 90% and in an especially highly preferable embodiment, at least 95% identity, at the amino acid level. In the case of larger deletions, e.g. the removal of the tail region, the comparison is performed to the amino acid sequence of the corresponding area in the original sequence.

***Functional Domains.*** The term in relation to a secretable fungal protein includes e.g. the secretion signal sequence or signal sequence and prosequence of the secretable protein or part of the protein, which contains sequences that permit the proteins or fusion constructions to be secreted. In other words, the term "functional domain" means a region of a DNA sequence which encodes a specific region of a protein. In this invention the term "functional domain" means the regions of the DNA sequence encoding the amino acid sequence responsible for the catalytic or enzymatic function (the core domain) or the binding function (the tail domain). The binding function is responsible for the binding of respective protein or enzyme, e.g. to xylan or cellulose. The specific domain can also be a folding domain, which is responsible for the tertiary structure of the protein, e.g. it encodes an α-helical or β-sheet structure of a protein or a combination thereof. The functional domain can also be responsible for the immunological activity of the protein. Thus, the "functional domain" might comprise a secretion signal or the core sequence or a sequence responsible for the folding of the protein as set forth above. Said functional domains can be totally separate from each other and are responsible for the "**biological**" activity of the protein. By the "biological" activity of an amino acid sequence of the invention is meant the enzymatic, functional, folding or binding activity or a combination of said activities of the secretable fungal protein. In this invention the term "funxtional domain" specifically relates to a tail domain of a cellulase or a core and hinge domain of a mannanase, which domains are derived from *Trichoderma.*

***Preparation or enzyme preparation.*** By "preparation or enzyme preparation" is meant a composition containing proteins or enzymes which are present in the culture medium and from which the host cells have been removed after the cultivation or fermentation has been completed. The preparation or enzyme preparation can be further processed by down-stream-processing methods, which are appropriate for the application of the protein or enzyme. The proteins or enzymes can be either partially or completely isolated and purified. For bulk use the preparation or enzyme preparation is usually subjected to filtration and/or centrifugation to remove the host cells from the spent culture medium. Thus the term "preparation or enzyme preparation" in the present invention means crude enzyme preparations for bulk use, but also actinomycete xylanases, which can be highly purified for special reasons.

By a host that is *"**substantially incapable**"* of synthesizing one or more enzymes is meant a host in which the activity of one or more of the listed enzymes is depressed, repressed, deficient, or absent when compared to the wild-type.

***Enzyme-aided bleaching**.* By "enzyme-aided bleaching" is meant the extraction of residual lignin from paper making pulp after the action of hemicellulose degrading enzymes with or without lignin degrading enzymes. The removal or extraction of lignin may be restricted by hemicelluloses either physically (through reprecipitation onto the fibre surface during cooking) or chemically (through lignin-carbohydrate complexes). The hemicellulase activity partially degrades the hemicellulose, which enhances the extractability of lignins by conventional bleaching chemicals (like chlorine, chlorine dioxide, peroxide, etc.) (Viikari et al., "Bleaching with Enzymes" in Biotechnology in the Pulp and Paper Industry, Proc. 3rd Int. Conf., Stockholm, pp. 67-69 (1986); Viikari et al., "Applications of Enzymes in Bleaching" in Proc. 4th Int. Symp. Wood and Pulping Chemistry, Paris, Vol. 1, pp. 151-154 (1987); Kantelinen et al., "Hemicellulases and their Potential Role in Bleaching" in International Pulp Bleaching Conference, Tappi Proceedings, pp. 1-9 (1988)). The advantage of this improved bleachability is a lower consumption of bleaching chemicals and lower environmental loads or higher final brightness values. In the past, this has often been referred to as biobleaching.

### II. Genetic Engineering of the Hosts of the Invention

The problem of producing bacterial proteins, preferably actinomycete xylanases in a cost-effective manner in a large scale is solved by producing the proteins in filamentous fungi, *Trichoderma.* The process for efficiently producing bacterial proteins in filamentous fungi is facilitated through the cloning of genetic sequences that encode the desired bacterial protein activity and through the expression of such genetic sequences in filamentous fungi. As used herein, the term "genetic sequences" is intended to refer to a nucleic acid molecule (preferably DNA). Genetic sequences that encode the desired proteins include actinomycetous DNA, cDNA, synthetic DNA and combinations thereof, preferably actinomycetous DNA, cDNA, synthetic DNA and combinations thereof encoding xylanase, most preferably *Actinomadura* genomic DNA, cDNA, synthetic DNA and combinations thereof. Vector systems may be used to produce hosts for the production of the enzyme preparations of the invention. Such vector construction (a) may further provide a separate vector construction (b) which encodes at least one desired gene to be integrated to the genome of the host and (c) a selectable marker coupled to (a) or (b). Alternatively, a separate vector may be used for the marker.

A nucleic acid molecule, such as DNA, is said to be "capable of expressing" a polypeptide if it contains expression control sequences which contain transcriptional regulatory information and such sequences are "operably linked" to the nucleotide sequence which encodes the polypeptide.

An operable linkage is a linkage in which a sequence is connected to a regulatory sequence (or sequences) in such a way as to place expression of the sequence under the influence or control of the regulatory sequence. Two DNA sequences (such as a protein encoding sequence and a promoter region sequence linked to the 5' end of the encoding sequence) are said to be operably linked if induction of promoter function results in the transcription of the protein encoding sequence mRNA and if the nature of the linkage between the two DNA sequences does not (1) result in the introduction of a frame-shift mutation, (2) interfere with the ability of the expression regulatory sequences to direct the expression of the mRNA, antisense RNA, or protein, or (3) interfere with the ability of the template to be transcribed by the promoter region sequence. Thus, a promoter region would be operably linked to a DNA sequence if the promoter were capable of effecting transcription of that DNA sequence.

The precise nature of the regulatory regions needed for gene expression may vary between species or cell types, but shall in general include, as necessary, 5' non-transcribing and 5' non-translating (non-coding) sequences involved with initiation of transcription and translation respectively.

Expression of the protein in the transformed hosts requires the use of regulatory regions functional in such hosts. A wide variety of transcriptional and translational regulatory sequences can be employed. In eukaryotes, where transcription is not linked to translation, such control regions may or may not provide an initiator methionine (AUG) codon, depending on whether the cloned sequence contains such a methionine. Such regions will, in general, include a promoter region sufficient to direct the initiation of RNA synthesis in the host cell.

As is widely known, translation of eukaryotic mRNA is initiated at the codon which encodes the first methionine. For this reason, it is preferable to ensure that the linkage between a eukaryotic promoter and a DNA sequence which encodes the protein, or a functional derivative thereof, does not contain any intervening codons which are capable of encoding a methionine. The presence of such codons results either in a formation of a fusion protein (if the AUG codon is in the same reading frame as the protein encoding DNA sequence) or a frame-shift mutation (if the AUG codon is not in the same reading frame as the protein encoding sequence).

In a preferred embodiment, a desired protein is secreted into the surrounding medium due to the presence of a secretion signal sequence. If a desired protein does not possess its own signal sequence, or if such signal sequence does not function well in the host, then the protein's coding sequence may be operably linked to a signal sequence homologous or heterologous to the host. The desired coding sequence may be linked to any signal sequence which will allow secretion of the protein from the host. Such signal sequences may be designed with or without specific protease sites such that the signal peptide sequence is amenable to subsequent removal. Alternatively, a host that leaks the protein into the medium may be used, for example a host with a mutation in its membrane.

If desired, the non-transcribed and/or non-translated regions 3' to the sequence coding for a protein can be obtained by the above-described cloning methods. The 3'-non-transcribed region may be retained for its transcriptional termination regulatory sequence elements; the 3'-non-translated region may be retained for its translational termination regulatory sequence elements, or for those elements which direct polyadenylation in eukaryotic cells.

The vectors of the invention may further comprise other operably linked regulatory elements such as enhancer sequences.

In a preferred embodiment, genetically stable transformants are constructed whereby a desired protein's DNA is integrated into the host chromosome. Such integration may occur *de novo* within the cell or, in a most preferred embodiment, be assisted by transformation with a vector which functionally inserts itself into the host chromosome, for example, DNA elements which promote integration of DNA sequences in chromosomes.

Cells that have stably integrated the introduced DNA into their chromosomes are selected by also introducing one or more markers which allow for selection of host cells which contain the expression vector in the chromosome, for example the marker may provide biocide resistance, e.g., resistance to antibiotics, or heavy metals, such as copper, or the like. The selectable marker gene can either be directly linked to the DNA gene sequences to be expressed, or introduced into the same cell by co-transfection.

Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Once the vector or DNA sequence containing the construct(s) is prepared for expression, the DNA construct(s) is introduced into an appropriate host cell by any of a variety of suitable means, including transformation as described above. After the introduction of the vector, recipient cells are grown in a selective medium, which selects for the growth of transformed cells. Expression of the cloned gene sequence(s) results in the production of the desired protein, or in the production of a fragment of this protein. This expression can take place in a continuous manner in the transformed cells, or in a controlled manner.

Accordingly, the bacterial protein encoding sequences may be operably linked to any desired vector and transformed into a selected filamentous fungi host, preferably *Trichoderma* host, so as to provide for expression of such proteins in that host.

To facilitate secretion of the bacterial protein, the bacterial protein encoding sequences may be fused in frame to other sequences so as to construct DNA encoding a fusion protein. For example, a recombinant vector encoding a xylanase gene from actinomycetous origin is fused with the sequence of one or more functional domains of *Trichoderma* cellulase or mannanase as described in US 5,298,405, WO 93/24622 and in Stålbrand et al., Appl. Environ. Microbiol. 61:1090-1097 (1995). The functional domains are selected from the group consisting of cellobiohydrolases (CBHI and CBHII), endoglucanases (EGI and EGII) and mannanase (MANI). MANI has the same domain structure as that of the cellulases: a core domain, containing the active site, a hinge domain containing a serine-threonine rich region, and a tail, containing the binding domain.

Fusion peptides can be constructed that contain an N-terminal mannanase core and the hinge domains fused to the *Actinomadura* xylanase sequence. The result is a protein that contains N-terminal mannanase core and hinge regions, and a C-terminal *Actinomadura* xylanase. The fusion protein contains both the mannanase and xylanase activities of the various domains as provided in the fusion construct. A further alternative is to use a gene coding for a modified or inactive mannanase core and hinge domains fused to *Actinomadura* xylanase sequences. The resulting fusion protein then contains the modified or inactive enzyme domain fused to a desired bacterial sequence.

Fusion proteins can also be constructed such that the cellobiohydrolase or endoglucanase tail is included, placed before the *Actinomadura* xylanase sequence, especially so as to allow use of a nonspecific protease site in the tail as a protease site for the recovery of the xylanase sequence from the expressed fusion protein. Alternatively, fusion proteins can be constructed that provide for a protease site in a linker that is placed before the *Actinomadura* xylanase sequence.

Accordingly, this invention results in the production and secretion of actinomycete xylanases, in *Trichoderma* hosts. The actinomycete xylanase is encoded as a fusion to a DNA sequence encoding a tail domain of a *Trichoderma* cellulase, or a core and hinge domain of a *Trichoderma* mannanase, thereby resulting in a high level of expression and secretion. The improvement in secretion of actinomycete xylanases is more than ten-fold compared to the production and secretion of actinomycete xylanases without a fusion of the actinomycete xylanase encoding gene to a fungal DNA sequence encoding a domain of a secretable protein. When a mammalian protein like chymosin is produced as a fusion protein in *Aspergillus,* the level of production has been only 10-20 % of the production levels of this invention (WO 90/15860). Similarly, the production level of immunoglobulins in *Trichoderma* has been only 10-20% of the production levels of this invention (WO 92/01797).

There are very few reports related to the production of proteins or enzymes of bacterial origin in filamentous fungi: the production of endoglucanase from *Cellulomonas fimi* (Gwynne et al., Bio/Technology 5: 713-719 (1987); and β-glucuronidase from *E. coli* (Punt et al., J. Biotechnol. 17: 19-34(1991) have been reported in *A. nidulans.* Of these enzymes, endoglucanase was secreted into the culture medium in the range of 10-15 mg protein per liter. β-glucuronidase was only detectable intracellularly.

Many of the studies on heterologous gene expression have concerned mammalian genes (van den Hondel et al., Heterologous gene expression in filamentous fungi, Ed. Bennett and Lasure. More Gene Manipulations in Fungi Academic Press, San Diego, U.S.A., pp. 396-428 (1991). So far, the initial yields of eucaryotic enzymes in filamentous fungi have been in a range of tens of mg per liter in shake flask cultivations. In the International patent publication WO 90/15860 secretion of chymosin by *A. niger* var. *awamori* was described using a fusion to the homologous glucoamylase gene. Nyyssönen et al., Bio/Technology 11: 591-595 (1993) describes the production of antibody fragments in *Trichoderma reesei.* The best yield of antibody fragments when produced as a fusion to the cellobiohydrolase 1 gene of *T. reesei* in the range of 40 mg per liter in a shake flask cultivation.

### III. The Enzyme Preparations of the Invention

According to the invention, there is provided a recombinant expression vector for producing actinomycete xylanases. These enzymes are synthesized as fusion proteins. The carrier protein in the fusion is a tail domain of a homologous cellulase, or a core and hinge domain of a homologous mannanase that is readily secreted from the *Trichoderma* host.

The enzyme compositions of the invention satisfy, e.g. requirements of specific needs in various applications in the pulp and paper industry. These applications include, e.g. enzyme-enhanced bleaching of paper making pulp, enzymatic fiberization during beating, enzymatic increase of drainage rates and ink removal of secondary fibre as well as enzymatic pitch removal.

The invention is applied to produce enzyme preparation partially or completely deficient in cellulolytic activity (that is, in the ability to degrade cellulose) and enriched in xylanases desirable for enzyme-enhanced bleaching of chemical pulp. By deficient in cellulolytic activity is meant a reduced, lowered, depressed, or repressed capacity to degrade cellulose. Such cellulolytic deficient preparations, and the making of same by recombinant DNA methods, are described in US 5,298,405, incorporated herein by reference. As described herein, xylanases may be provided directly by the hosts of the invention (the hosts themselves are placed in the wood processing medium). Alternatively, used medium from the growth of the hosts, or purified enzymes therefrom, can be used. Further, if desired activities are present in more than one recombinant host, such preparations may be isolated from the appropriate hosts and combined prior to use in the method of the invention.

To obtain the enzyme preparations of the invention, the recombinant *Trichoderma* hosts described above having the desired properties (that is, for example, hosts capable of expressing large quantities of the actinomycete xylanase enzymes and optionally, those which are substantially incapable of secreting one or more cellulase enzymes) are cultivated under suitable conditions, the desired actinomycete xylanases are secreted from the *Trichoderma* hosts into the culture medium, and the enzyme preparation is recovered from said culture medium by methods known in the art.

The enzyme preparation is the culture medium with or without the native or transformed host cells, or is recovered from the same by the application of methods well known in the art. However, because the xylanase enzymes are secreted into the culture media and display activity in the ambient conditions of the hemicellulolytic liquor, it is an advantage of the invention that the enzyme preparations of the invention may be utilized directly from the culture medium with no further purification. If desired, such preparations may be lyophilized or the enzymatic activity otherwise concentrated and/or stabilized for storage. The enzyme preparations of the invention are very economical to provide and use because (1) the enzymes may be used in a crude form; isolation of a specific enzyme from the culture fluid is unnecessary and (2) because the enzymes are secreted into the culture medium, only the culture medium need be recovered to obtain the desired enzyme preparation; there is no need to extract an enzyme from the hosts.

If desired, an expressed xylanase may be further purified in accordance with conventional conditions, such as extraction, precipitation, chromatography, affinity chromatography, electrophoresis, or the like.

### IV. Identification and Isolation of Actinomadura flexuosa Xylanases

Two xylanases have been identified, purified and cloned from *Actinomadura flexuosa.* Both of these xylanases have a pH optimum and thermostability that are desirable for the enzyme-aided bleaching of wood pulp. One of these xylanases has a molecular weight of about 35 kDa (AM35) and the other has a molecular weight of about 50 kDa (AM50).

The optimal temperature range for *A. flexuosa* xylanases in crude preparations is 70-80 °C at pH 6-7. At pH 8, the optimum temperature range of this xylanase preparation is 60-70 °C. This is useful in kraft pulp bleaching because after kraft cooking, the pH of the pulp is alkaline.

In purified preparations, AM35 retains 80% of its activity, and AM50 retains 90% of its activity after 24 hours when incubated in the presence of BSA at pH 6.0 and 70 °C. At 80 °C, both AM35 and AM50 are most active at pH 6 but both exhibit a broad activity plateau between pH 5 - pH 7, wherein about 80% of the activity is retained.

For the isolation of AM35 and AM50, the host *A. flexuosa* is available as depository accession number DSM43186 from Deutsche Sammlung von Mikroorganismen und Zellkulturen GmbH, Mascheroder Weg 1b, D-38124 Braunschweig, Germany. Both forms can be purified by passage through a series of chromatographic columns. A first purification step by DEAE Sepharose CL-4B retains about half of the xylanase activity when the sample is applied at pH 8.6-9 in 12.5 mM Na₂HPO₄; the other half is found in the flow through.

Elution of the bound xylanase activity with a salt gradient results in an elution of a sharp, earlier eluting peak of activity and a broad, later eluting peak of activity. The sharp, earlier eluting peak retains its homogeneity when subjected to phenyl Sepharose CL-4B chromatography. Samples taken from the later, broad peak of activity separate into at least two peaks when subjected to Phenyl Sepharose CL-4B chromatography. There is only weak cross-reactivity of these later eluting xylanases with a polyclonal antibody directed against *Thermomonospora fusca* xylanase.

By SDS-PAGE, the molecular weight of the xylanase in these pools from the DEAE retentate was about 50 kDa, while the molecular weights of the xylanases in the DEAE flow through was 30, 35, 40 and 50 kDa. Thus, *A. flexuosa* contains four to five xylanase protein bands.

### V. Enzyme-aided Bleaching using the Actinomadura flexuosa Xylanases

The present invention comprehends a method for enzymatically treating plant biomass under conditions of high temperature of 50-90 °C and pH 5-8, and especially 60-85 °C, pH 6-7 and most preferably 70-80 °C and pH 7.0 for one to two hours. In a preferred embodiment, plant biomass is treated with xylanases that are able to hydrolyze xylan chains in wood pulp at neutral or moderately alkaline pH and high temperature. Alternatively, enzyme treatment can be combined to chelating stages (metal removal stages) in which high temperatures but low pH values (4-6) are typically used.

Wood pulp is a composite material consisting primarily of a matrix of cellulose, hemicellulose, and lignin. A common procedure for wood pulp production is chemical pulping. One typical mode of chemical pulping is alkaline sulphate cooking, so called kraft cooking. Under the process conditions (high temperatures and high alkalinity), the cooking chemicals extract lignin out of the pulp. However, not all of the lignin is removed during cooking, but part of it, (about 5%), remains in the pulp. This residual lignin has to be removed in order to get pulp suitable for paper production.

Many processes have been developed for the removal of residual lignin. Typically, the wood pulp is treated with chlorine or other toxic chemicals in order to remove the lignin component and provide a bleached pulp. However, the toxic by-products of this chemical treatment have a negative impact upon the health and stability of the environment into which they are released. Consequently, there is a great need for developing alternative, more environmentally protective techniques for pulp bleaching. Treatment of the cooked pulp with enzymes that partially degrade hemicellulose, e.g., xylan, in the pulp, modifies the pulp so that the lignin becomes easier to extract or remove. This leads to improved bleachability which in turn gives the advantages of lower bleaching chemical consumption and lower environmental loads and/or higher final brightness.

Use of the recombinant expression vector of the present invention for production of actinomycete xylanases provides an enzyme-aided bleaching technique whereby thermostable and neutral xylanases can be used in such conditions that the need to adjust the pH and temperature after the cooking step is decreased or eliminated.

In a preferred embodiment, said technique is carried out *in vitro* in wood pulp. The technique involves placing the enzyme preparation, culture medium, or concentrated mixture containing xylanase into contact with the wood pulp. Routine calculations enable those in the art to determine the dosage of the xylanase enzyme used, treatment pH and temperature and other parameter variables.

Said enzyme-aided bleaching technique may be applied alone or as a supplement to other treatments that improve the removal of lignin from wood pulp. In a preferred embodiment, the present invention is used to enhance the bleachability of wood pulps, especially chemical pulps.

In a preferred embodiment, the xylanases used in said enzyme-aided bleaching are preferably those of *A. flexuosa,* and especially the 35 kDa and/or 50 kDa xylanases of *A. flexuosa.* Especially useful is the culture medium that can be provided by a recombinant host that has been transformed with the expression vectors of the invention.

### VI. Preferred and Further Embodiments and Applicability of the Invention

The invention describes an improved method of expressing and secreting xylanases originating from bacteria, especially from actinomycetes in filamentous fungi by using fusion protein techniques. More than 50-fold greater production and secretion levels than those observed in the original actinomycete strains have been obtained. The production and secretion levels were more than 10-fold higher than those previously observed when producing heterologous (mammalian or bacterial) enzymes in filamentous fungi. The main purpose of the invention is to provide a method for producing xylanases originating from bacteria, especially actinomycete in filamentous fungi, *Trichoderma.*

Thus, the invention is related to a recombinant expression vector for production of actinomycete xylanases in a *Trichoderma* host. Said vector comprises a promoter operably linked to a DNA sequence of one or more functional domains of a filamentous fungus secretable protein, which is fused in frame with a DNA sequence encoding an actinomycete xylanase.

Generally, the filamentous fungus secretable protein encoding DNA sequence encodes an enzyme, such as cellulases or hemicellulases. The enzymes are homologous to the secreting filamentous fungus, e.g. *Trichoderma.* In the most preferred embodiments of the invention the DNA sequence encoding the filamentous fungus secretable protein is an enzyme, which is known to be secreted in significant amounts in *T. reesei.*

Useful proteins or enzymes to provide the objectives of the invention are *Trichoderma* cellulases and *Trichoderma* mannanases but the most preferred filamentous fungus secretable proteins are the following enzymes, which are homologous to *Trichoderma,* i.e. cellobiohydrolases (CBHI and CBHII), endoglucanases (EGI and EGII), and mannanase (MANI). Especially useful for the invention is a tail domain of a *Trichoderma* cellulase, or a core and hinge domain of a *Trichoderma* mannanase.

The amino acid and DNA sequences of the enzymes and domains mentioned above are well known from literature and some are described in the examples of the present invention below. The skilled person can by using said sequences construct a multitude of alternative useful expression vectors according to the principals set forth in this application.

Because the main objective of the invention is the production of xylanases originating from actinomycete, the recombinant expression vector of the present invention comprises a DNA sequence encoding a xylanase originating from an actinomycete.

Examples of such useful DNA sequences are those obtainable from *A. flexuosa* the DNA sequence SEQ ID NO: 1: encoding the amino acid sequence of SEQ ID NO: 2: or the DNA sequence SEQ ID NO: 3: encoding the amino acid sequence of SEQ ID NO: 4: or equivalents of said amino acid sequences, wherein said equivalents have at least 85 % identity at the amino acid level with SEQ ID NO: 2 or SEQ ID NO: 4 and an xylanolytic activity similar to that of SEQ ID NO: 2: or SEQ ID No: 4:. Also the amino acid sequences assigned SEQ ID NO: 12:, SEQ ID NO: 14:, SEQ ID NO: 16:, and SEQ ID NO: 18: are described below in the examples, the sequences representing the fusions between the core and hinge region of MANI and *A. flexuosa* xylanase gene.

Useful DNA sequences can be found e.g. in the plasmids pALK923 (DSM9322), pALK938 (DSM9899), pALK939 (DSM9900, pALK940 (DSM9901), pALK941 (DSM9902) and pALK1056 (DSM9903) that encode the *Actinomadura flexuosa* AM35 xylanase and plasmids pALK927 (DSM9447) and plasmid pALK928 (DSM9448) that encode the *Actinomadura flexuosa* AM50 xylanase.

The preferred promoters in the recombinant DNA expression vectors of the present invention are those of a filamentous fungus secretable protein, most preferably a *T. reesei cbh1* promoter.

Examples of preferred recombinant expression vector are the plasmids pALK945, pALK948, pALK1021 and pALK1022. The plasmid pALK1022 is shown in Figure 17. The constructs of the three other plasmids are essentially the same as plasmid pALK1022 with the exception that the linker sequences are as those described in detail in the examples, e.g. SEQ ID NO: 11:, SEQ ID NO: 13:, SEQ ID NO: 15: and SEQ ID NO: 17:. Said expression vectors are used to transform filamentous fungi, such as *Trichoderma,* most preferably *T. reesei.*

Hosts transformed with said expression vectors produce xylanases which all have the N-terminal amino acid sequence SEQ ID NO: 5:, which is identical with the N-terminal sequence (D-T-T-T-I-T-Q) of wild type *A. flexuosa* xylanase.

The transformed hosts are capable of expressing and secreting one or more proteins or enzymes, preferably xylanases originating from actinomycete into the culture medium during the cultivation or fermentation.

After the cultivation and removal of the host cells by filtration, centrifugation etc., the culture medium as such or concentrated provides a useful protein or enzyme preparation, which can be subjected to further down-stream processing methods for improved stability and storability. Alternatively, the preparation can be subjected to isolation and purification to obtain the desired protein or enzyme originating from bacteria or actinomycete in more purified form.

Examples of useful down-stream processes of the spent culture medium are e.g. filtration, ultrafiltration, precipitation, centrifugation, drying, evaporation, immobilization, granulation etc.

The preparation containing enzymes, especially xylanases originating from actinomycetes and which are obtainable by cultivating hosts transformed with the expression vectors of the present invention are useful e.g. for enzyme-aided bleaching in because the enzymes are stable at processing temperatures, when the processing temperatures are in the ranges 50-90 °C, preferably 60-85 °C, most preferably 70-80 °C.

Said preparations are also useful for treating paper pulp and for enzymatical treating of plant biomass.

Thus, the present invention provides a recombinant expression vector for producing a xylanase containing preparation of actinomycetous origin in a filamentous fungal host *Trichoderma,* in which recombinant expression vectors a promoter is operably linked to a DNA sequence encoding a tail domain of a *Trichoderma* cellulase or a core and hinge domain of a *Trichoderma* mannanase, which in turn contain, fused in frame, a DNA sequence encoding an actinomycete xylanase; then transforming a *Trichoderma* host with the thus constructed vectors and cultivating said transformed hosts in a culture medium and under culture conditions, which are optimal for the secretion of the enzyme. After the cultivation the host cells are separated from the culture medium, which can be used as an enzyme preparation as such or in concentrated form or after subjected to suitable down-stream processing methods.

The invention is described in more detail in the following examples, These examples show only a few concrete applications of the invention. It is self evident for one skilled in the art to create several similar applications.

### Examples

### Example 1

### Actinomadura flexuosa DSM43186 Shake Flask and Fermentor Cultivations

The strain *A. flexuosa* DSM43186 was streaked on rolled oats mineral medium plate (Deutsche Sammlung von Mikroorganismen und Zellkulturen GmbH [German collection of microorganisms and cell cultures], *DSM Catalogue of strains,* 3rd ed., Braunschweig, Germany (1983); 1 liter contains 20 g agar, 20 g rolled oats, 1 ml trace element solution containing 100 mg FeSO₄ x 7 H₂O, 100 mg MnCl₂ x 4 H₂O, 100 mg ZnSO₄ x 7 H₂O / 100 ml; pH 9.0) and incubated at 50 °C until sporulating. A sporulating colony was inoculated in 10 ml of XPYB medium (Greiner-Mai, E. et al., System. Appl. Microbiol. 9:97-109 (1987); Holtz, C. et al., Antonie van Leeuwenhoek 59:1-7 (1991)); 1 liter contains 5 g oats spelt xylan, 5 g peptone from casein, 5 g yeast extract, 5 g beef extract, 0.74 g CaCl₂ x 2 H₂O; pH 9.0) and was incubated at 55 °C in a rotary shaker (250 rpm) for two to three days. An inoculum of 5 ml was then transferred to 250 ml of the same medium and incubated at the same conditions for three days. Xylanase activity obtained was 17 nkat/ml.

The procedure for two 1 L fermentations (Biostat M, B. Braun, Germany) was prepared as above. 10% (v/v) inoculum was used for the fermentations. The pH was maintained at pH 7.8 ± 0.2 by addition of ammonia (12.5% (v/v)) and phosphoric acid (17% (v/v)), the fermentation temperature was 50 °C. The fermentor was stirred at 400 rpm and the air flow was 1 L/min. Xylanase activities obtained were 32 and 58 nkat/ml (measured at pH 6.0 and 60 °C with 5 minutes incubation time). The culture media of the cultivations were used in protein purification and bleaching experiments.

Xylanase activities throughout the examples were measured according to Bailey, M.J. et al., J. Biotechnol. 23:257-270 (1992) using 1% (w/v) birch xylan (Roth no. 7500) as a substrate. The assay conditions were, if not otherwise stated, pH 5.3 and 50 °C with an incubation time of 5 minutes. One xylanase unit (1 nkat) is defined as the amount of enzyme that produces reducing carbohydrates having a reducing power corresponding to one nmole of xylose in one second from birch xylan under assay conditions. The International Unit (IU) is defined as the amount of enzyme that produces one micromole of measured end-product in one minute from the polymeric substrate, then 1 IU = 16.67 nkat.

### Example 2

### Determination of the pH and Temperature Dependencies of Actinomadura flexuosa Xylanase Activity from the Culture Medium

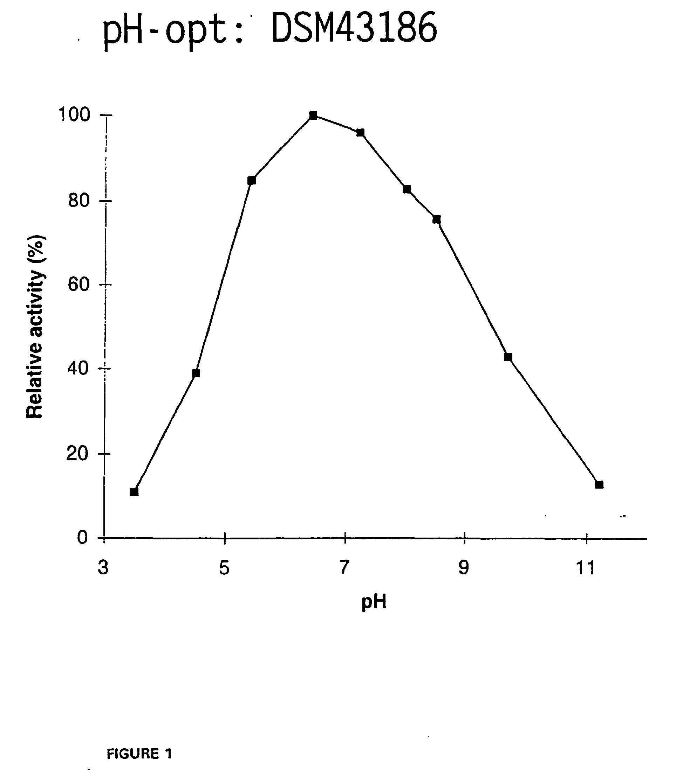

To determine the pH dependency for the *A. flexuosa* xylanase activity, samples of the culture medium from the shake flask cultivation (Example 1) were diluted in 50 mM McIlvains buffers (50 mM citric acid - 100 mM Na₂HPO₄) of pH-range 3.0 - 11.0. The final pH values of the enzyme buffer mixtures were 3.5, 4.5, 5.4, 6.4, 7.2, 8.0, 8.5, 9.7 and 11.2. Xylanase activity was measured at each pH at 50 °C, 5 min reaction. The xylanase activity exhibited 80-100% of its maximum activity in the pH range of about 5.4-8.0, showing maximum activity at about pH 6.4 (Figure 1).

For the thermal stability determination, samples from the culture supernatant were diluted in 50 mM McIlvain's buffers. Bovine serum albumin (BSA) was added to a concentration of 100 µg/ml and pepstatin A 10 µg/ml as well as phenyl methyl sulfonyl fluoride (PMSF) 174 µg/ml were added as protease inhibitors. The final pH values of the enzyme buffer mixtures were 6.9, 7.8 and 9.0. Samples were incubated in the absence of the substrate at 60 °C, 70 °C and 80 °C. Samples were taken at intervals of 0, 30, 60 and 120 minutes and immediately cooled on ice prior to the residual xylanase activity determination at 50 °C (5 min reaction in the corresponding pH). The enzyme was very stable when incubated at 60 °C and 70 °C; after 120 minutes incubation at 70 °C at pH 9 over 60% of xylanase activity was retained (Figure 2A, 2B and 2C).

### Example 3

### Purification of Actinomadura flexuosa Xylanases

Purification of xylanases from *A. flexuosa* growth medium was performed at +4 °C with chromatographic columns coupled to a FPLC apparatus (Pharmacia). Xylanase activity measurements were performed at 50 °C and at pH 6.5. Protein was monitored at 280 nm throughout the purification. Samples were run on polyacrylamide slab gels containing 0.1 % SDS on a Bio-Rad Mini Protean II electrophoresis system and stained with Coomassie Brilliant Blue. A polyclonal antibody prepared against *Thermomonospora fusca* xylanase A XynA, obtained from Prof. David Wilson, Cornell University, New York) was used to detect *A. flexuosa* xylanase(s) in Western blots. In the detection, Promega's ProtoBlot AP System was used.

A growth medium of the two 1 l fermentations described in Example 1 was pooled and centrifuged at 8,000 g for 30 min. The supernatant (1,500 ml) was diluted 1 + 2 with 12.5 mM Na₂HPO₄ pH 9 and adjusted to pH 8.6 with 1 M NaOH. This sample was applied, in two sets, on a DEAE Sepharose CL-6B (Pharmacia) ion-exchanger (2.5 x 29 cm) equilibrated with 12.5 mM Na₂HPO₄, pH 9, at 100 ml/h. The flow-through of both runs was combined and processed separately as described later.

Elution of the bound proteins from the DEAE-column (Figure 3) was accomplished by a linear gradient (400 ml + 400 ml) from 25 mM Na₂HPO₄ pH 9, to 25 mM Na₂HPO₄, pH 9 containing 1 M NaCl at a flow rate of 105 ml/h and fractions of 10 ml were collected. Two xylanase activity containing peaks could be collected (pool I and II), as well as a long "tailing" of the second peak (pool III).

The three pools (each combined from both DEAE runs) were adjusted to contain 2 M sodium chloride each and applied separately on a Phenyl Sepharose CL-4B (Pharmacia) column (2.5 x 15 cm) equilibrated with 25 mM Na₂HPO₄, pH 9 containing 2 M NaCl. Elution was performed at 100 ml/h with a two step gradient of 100% buffer A (25 mM Na₂HPO₄, pH 9) to 35% buffer B (25 mM Na₂HPO₄ containing 60% ethylene glycol) in 60 min followed by a steeper gradient from 35 % B to 100 % B in 60 min. Fractions of 7 ml (pool I) or 5 ml (pools II and III) were collected. The xylanase activity containing fractions of pool I obtained were pooled and named DEPS I (Figure 4A). Both DEAE pools II and III resulted in two xylanase activity containing peaks named DEPS II/1, DEPS II/2 (Figure 4B) and DEPS III/1, DEPS III/2 (Figure 4C), respectively.

Samples of these pools were dialyzed against 25 mM Na₂PO₄, pH 9, over night and run on SDS-PAGE and stained for protein with Coomassie Blue (Figure 5A) as well as analyzed by Western blots with *T. fusca* antibody (Figure 5B). The antibody reacted only with two to three bands of smaller molecular mass (35 kDa or lower) from the growth medium and weakly with the proteins in these pools. The apparent molecular masses of the proteins in these pools were 50 kDa as estimated from SDS-PAGE with molecular mass standards. Pools DEPS 11/2, DEPS III/1 and DEPS III/2 were the most pure.

The flow-through of the DEAE ion-exchanger (see above) was concentrated with a cut-off membrane of 30 kDa. Roughly half of the xylanase activity was found in the concentrate and half in the permeate. Both were adjusted to contain 2 M NaCl and applied on a Phenyl Sepharose 6 FastFlow (low sub; Pharmacia) column (2.5 x 34 cm) equilibrated with 25 mM Na₂HPO₄, pH 9, containing 2 M NaCl. Elution was accomplished at 300 mlh-¹ with the same gradient as was used for DEAE pools on Phenyl Sepharose CL-6B and fractions of 10 ml were collected. Xylanase activity containing peaks obtained were named KF1, KF2 and KF3 (Figure 6B). The permeate from the concentration was subjected to an identical Phenyl Sepharose 6 FastFlow (low sub) run, and the xylanase activity containing fractions were named PF1 and PF2 (Figure 6A). These peaks were dialyzed over night against 25 mM Na₂HPO₄, pH 9, and analyzed on SDS-PAGE as well as on Western blots (Figure 7A and 7B). The first peak, KF₁, from the concentrate showed a band of 40 kDa apparent molecular mass on SDS-PAGE, but no reaction on Western blots. However, this peak had the highest xylanase activity. KF2 showed a band of 50 kDa on SDS-PAGE reacting weakly with the antibody, but a clear band of 30 kDa could be seen on Western blots. The third peak, KF3, showed a band of 35 kDa on Western blots. The concentrate contained xylanases with apparent molecular weights of 50, 40, 35 as well as 30 kDa. The first peak, PF1, from the permeate reacted with *T. fusca* antibody showing two bands of 35 kDa and 30 kDa. respectively. PF2, on the other hand, showed only one band of 35 kDa on Western blots.

As a summary, *A. flexuosa* sp. DSM43186 growth medium contains xylanases with molecular mass of about 50 kDa (represented by pools DEPSII/2,DEPSIII/1 and DEPSIII/2), 40 kDa (represented by pool KF1), 35 kDa (represented by pool PF2 and KF3) and 30 kDa (represented by pool KF2). Of these, the 35 kDa and 50 kDa proteins appear as the major xylanase bands (on SDS-PAGE). It is possible that the 40 kDa xylanase band on SDS-PAGE is a degradation product of the 50 kDa band on SDS-PAGE and that the 30 kDa band on SDS-PAGE is a degradation product of 35 kDa xylanase band on SDS-PAGE.

### Example 4

### Production and Sequencing of Peptides from the Purified 35 kDa and 50 kDa Xylanases

A sample (12 ml) of pool I from the DEAE Sepharose CL-6B (Fig. 3) run was subjected to gel exclusion chromatography on a HighLoad 26/60 Superdex G75 column (Pharmacia) equilibrated with 25 mM Na₂HPO₄, pH 9 at 120 ml/h. A sample (25 ml) of the xylanase activity containing peak fraction obtained was diluted (1 + 1) with water and applied on a mono Q (Pharmacia) ion-exchanger equilibrated with 12.5 mM Na₂HPO₄, pH 9. Elution was performed at 30 ml/h with a linear gradient from 12.5 mM Na₂HPO₄, pH 9 to 12.5 mM Na₂HPO₄, pH 9 containing 0.5 M NaCl in 50 min. The xylanase activity containing peak (1 ml) was concentrated on a Centricon micro concentrator (cut-off 30 kDa) and eluted with 1% ammonium bicarbonate. This concentrated sample containing a single 50 kDa protein band on SDS-PAGE was evaporated and alkylated with vinylpyridin. The alkylated sample was digested with trypsin (modified trypsin, sequenal grade, Promega V5111). The digest was applied on a reverse phase column coupled to an HPLC, and peaks absorbing at 214 nm were collected manually. Each of the collected fractions were subjected to Edman degradation in a gas-pulsed-liquid-phase sequencer (Kalkkinen & Tilgmann, J. Protein Chem. 7:242-243 (1988)) and the released PTH amino acids were analyzed on-line by using narrow bore reverse phase HPLC.

A sample of purified 35 kDa xylanase (purified essentially as described in Example 3) was subjected to Edman Degradation as above.

Peptides obtained from the purified 50 kDa xylanase and the N-terminal sequence of the purified 35 kDa xylanase are listed in Table 1.

**Table 1: Peptides from the purified 50 kDa xylanase and the N-terminal sequence of the purified 35 kDa xylanase**

| **Peptide** | **Sequence** |
|---|---|
| # 1696 | Ala-Ala-Ser-Thr-Leu-Ala-Glu-Gly-Ala-Ala-Gln-His-Asn-Arg |
| # 1697 | Tyr-Phe-Gly-Val-Ala-Ile-Ala-Ala-Asn-Arg |
| # 1698 | Leu-Asn-Asp-Ser-Val-Tyr-Thr-Asn-Ile-Ala-Asn-Arg |
| # 1699 | Asn/Gly/X-Thr-Gly-Ile-Thr-Val-X-Gly-Val |
| # 1703 | His/Glu/Thr-Glu/Phe-Leu/Asn-Val/Ser-Tyr/Val-Asn/Thr-Met /Ala-Val/ Glu-Asn/X-Glu/X-Met/X |
| # 1704 | Glu-Phe-Asn-Ser-Val-Thr-Ala-Glu-Asn-Glu-Met-(Lys) |
| 35 kDa N-term | Asp-Thr-Thr-Ile-Thr-Gln |

The combination of the 50 kDa xylanase peptide sequences #1696 (SEQ ID NO: 6), #1697 (SEQ ID NO: 7), #1698 (SEQ ID NO: 8) and #1704 (SEQ ID NO: 10) corresponds with 75 % similarity to amino acids 42-89 in *Streptomyces lividans* xylanase A (accession number M64551). The peptide #1703 has not been assigned any SEQ ID NO, because the multitude of alternatives present in the sequence and because it is not claimed. In addition, peptide #1699 (SEQ ID NO: 9:) shows 78% similarity to amino acids 301-309 in *S. lividans* XlnA:

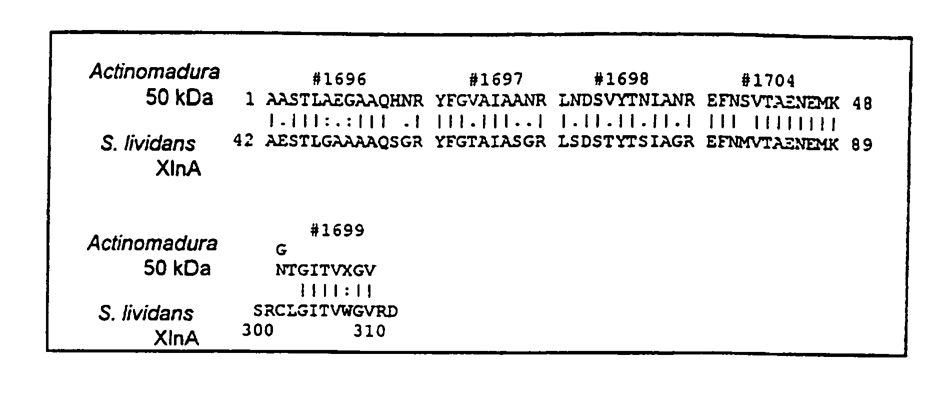

The sequences of *S.lividans* are present for comparison only.

### Example 5

### The pH Properties and Temperature Stability of the Purified 35 kDa and 50 kDa Xylanases

The temperature stability of the purified 35 and 50 kDa enzymes with or without 100 µg/ml BSA was determined by incubating the enzyme samples at 70 °C, pH 6.0 for a period of 0, 2, 6 and 24 hours after which the xylanase activity of the samples was determined (at pH 6.5, 60 °C, 20 min reaction). In the samples into which BSA had been added, over 80% of the original activity could be measured even after 24 h of incubation (Figures 8 and 9 for the 35 kDa and the 50 kDa xylanases, respectively). When BSA was not added, still about 60% (35 kDa) or 70% (50 kDa) of the original activity was measured after 24 h of incubation (Figures 8 and 9).

The pH dependence was determined by incubating the enzyme samples at different pH values (pH 4-8) and at temperatures of 80 °C (35 kDa) and 60, 70 and 80 °C (50 kDa) for 20 minutes (35 kDa) or 10 minutes (50 kDa). At 80 °C, the 35 kDa xylanase showed a pH optimum of around pH 6 having nearly 90% of its activity from about pH 5 to 7 (Figure 10A). At 60 °C and 70 °C, the 50 kDa xylanase showed a pH optimum of pH 5-7 and at 80 °C, a pH optimum of pH 6-7. The enzyme was very stable from pH 5-7 under these conditions (Figure 10B). Incubation of both 35 kDa and 50 kDa xylanases at 60 °C for 60 minutes at pH values from 4.2 to 8.7 showed similar stability as found in the above experiment, except that the 50 kDa xylanase seems to be less stable at pH 4.2 under these conditions (Figure 10C). Temperature dependence experiments at pH 7 with 60 minute incubations of the 35 kDa and 50 kDa xylanases with substrate at temperatures of 50, 60, 70 and 80 °C showed maximal activity at 70 °C for both enzymes (Figure 11). The 50 kDa xylanase seemed from these results to be slightly more stable at 80 °C and pH 7 than the 35 kDa xylanase. On the other hand, the 35 kDa xylanase showed more activity and stability in the pH range of 4-5 (Figures 10A-10C).

### Example 6

### Bleaching Experiments Using Actinomadura flexuosa Culture Medium

Bleaching experiments were done to determine the usefulness of *A. flexuosa* xylanase activity in both ECF (elementary chlorine free) and TCF (totally chlorine free) bleaching of kraft pulp.

### ECF Bleaching

Growth medium containing *A. flexuosa* xylanase activity (Example 1) was added to Finnish oxygen delignified softwood kraft pulp (kappa number 15) in the amount of 50 or 100 nkat/g pulp dry matter. Xylanase activity was measured at pH 6 and 60 °C with 5 minutes incubation time. Cellulase activity of the growth medium was very low. The enzyme treatments were done at pH 7 and 70 °C for one hour. Reference pulp was kept under the same conditions without enzyme addition.

After the enzyme treatments pulps were bleached in two stages: chlorine dioxide stage and alkaline extraction. The absorbance of the filtrate at 280 nm was determined to estimate the amount of dissolved lignin.

As can be seen in Table 2, after the pretreatment with the xylanase preparation more residual lignin was removed from the pulps as evidenced by the increase of A₂₈₀ of the filtrates and reduction of kappa numbers in the final pulps. The final pulps had also 3-4 units higher brightness compared with the reference. The strength of the pulps was not affected, because the viscosity values stayed inside the normal variation of the method.

**Table 2**

| | **Reference** | **50 nkat/g** | **100 nkat/g** |
|---|---|---|---|
| **Enzyme treatment** | | | |
| Consistency, % | 3 | 3 | 3 |
| Retention time, hours | 1 | 1 | 1 |
| Enzyme dosage, nkat/g | 0 | 50 | 100 |
| Temperature, °C | 70 | 70 | 70 |
| pH, start/end | 7.0/7.1 | 7.0/7.2 | 7.2/7.4 |
| Absorbance, 280 nm | 0.22 | 0.49 | 0.65 |
| **ClO₂ stage** | | | |
| Consistency, % | 3 | 3 | 3 |
| Retention time, hours | 1 | 1 | 1 |
| ClO₂ dosage, % | 2.3 | 2.3 | 2.3 |
| Temperature, °C | 60 | 60 | 60 |
| pH at the end | 2.4 | 2.5 | 2.5 |
| **Extraction stage** | | | |
| Consistency, % | 10 | 10 | 10 |
| Retention time, hours | 1 | 1 | 1 |
| NaOH dosage, % | 1.5 | 1.5 | 1.5 |
| Temperature, °C | 70 | 70 | 70 |
| pH at the end | 10.9 | 10.9 | 10.9 |
| **Final Pulp** | | | |
| Brighmess, % ISO | 56.7 | 59.9 | 60.6 |
| Kappa number | 6.6 | 5.6 | 5.4 |
| Viscosity, ml/g | 920 | 910 | 900 |

### TCF Bleaching

Finnish oxygen delignified softwood kraft pulp (kappa number 15) was treated with *A. flexuosa* xylanase preparation using enzyme dosages of 50 and 100 nkat/g pulp dry matter. Xylanase activity was measured at pH 6 and 60 °C with 5 minutes incubation time. Cellulase activity of the preparation was very low. The enzyme treatments were done at pH 7 and 70 °C for one hour. Reference pulp was kept under the same conditions without enzyme addition.

After the enzyme treatments the pulps were bleached using QP sequence. Metals were first removed by chelating with 0.2 % EDTA (chelating stage, Q) and the pulps were then bleached with hydrogen peroxide (peroxide stage, P). Bleaching chemicals were the following: 3 % H₂O₂, 3 % NaOH 0,2 % DPTA (diethylene triamine pentaacetic acid) and 0.5 % MgSO₄. The absorbance of the filtrate at 280 nm was determined to estimate the amount of dissolved lignin. The results are shown in Table 3.

**Table 3**

| | **Reference** | **50 nkat /g** | **100 nkat /g** |
|---|---|---|---|
| **Enzyme treatment** | | | |
| Consistency, % | 3.5 | 3.5 | 3.5 |
| Retention time, hours | 1 | 1 | 1 |
| Enzyme dosage, nkat/g | 0 | 50 | 100 |
| Temperature, °C | 70 | 70 | 70 |
| pH. start/end | 7.0/7.4 | 7.0/7.3 | 7.0/7.3 |
| Absorbance, 280 nm | 0.27 | 0.43 | 0.57 |
| **Chelation stage, Q** | | | |
| Consistency, % | 3.0 | 3.0 | 3.0 |
| Retention time, hours | 1 | 1 | 1 |
| EDTA. % of dry matter | 0.2 | 0.2 | 0.2 |
| Temperature, °C | 70 | 70 | 70 |
| pH at the end | 5.5 | 5.6 | 5.8 |
| Absorbance, 280 nm | 0.24 | 0.44 | 0.64 |
| **Peroxide stage, P** | | | |
| Consistency, % | 10 | 10 | 10 |
| Retention time, hours | 3 | 3 | 3 |
| Temperature, °C | 80 | 80 | 80 |
| pH at the end | 10.6 | 10.6 | 10.6 |
| Peroxide dosage, % | 3.0 | 3.0 | 3.0 |
| Peroxide consumed, % | 2.1 | 2.2 | 2.1 |
| **Final Pulp** | | | |
| Brightness, % | 71.9 | 72.9 | 73.0 |
| Kappa number | 9.0 | 8.3 | 7.9 |
| Viscosity, ml/g | 870 | 890 | 890 |

Table 3 shows that according to the measured A₂₈₀ values and kappa numbers, significantly more lignin was removed after the xylanase treatments compared with the reference. The viscosity values of the pulps remained inside the normal variation of the method, which means that the strength of the pulps was not affected. Also brightness values were higher than reference, but as expected the increase was slighter than in chlorine dioxide bleaching.

### Example 7

### Bleaching Experiments Using the Purified 35 kDa and 50 kDa Xylanases

The purified larger 50 kDa (AM50) xylanase and the smaller 35 kDa (AM35) xylanase (including also the 30 kDa xylanase) were tested in a three stage peroxide bleaching. The purified enzyme preparations were the same as used in the determination of the pH and temperature properties of the purified enzymes (Example 5).

The purified enzyme preparations were added to Finnish oxygen delignified softwood kraft pulp (kappa number 13.5 and brightness 37 %) in the amount of 100 nkat/g pulp dry matter. Xylanase activity was measured at pH 6.5 and 60 °C with 5 minutes incubation time. The enzyme treatments were done at pH 6.5 and 60 °C for one hour. Reference pulp was treated in the same conditions but without enzyme addition. Bleaching was performed with QP₁P₂P₃ sequence. The chelation stage (Q) was performed by adding EDTA to 0.2 % of pulp dry matter. The three hydrogen peroxide stages (P₁P₂P₃) were all carried out the same way. The results are shown in Table 4.

**Table 4**

| | **Reference** | **AM50** | **AM35** |
|---|---|---|---|
| **Enzyme treatment** | | | |
| Consistency, % | 3.5 | 3.5 | 3.5 |
| Retention time, hours | 1 | 1 | 1 |
| Enzyme dosage, nkat/g | 0 | 100 | 100 |
| Temperature, °C, start/end | 60/59 | 58/58 | 60/59 |
| pH, start/end | 6.6/6.6 | 6.8/6.8 | 6.7/6.7 |
| **Chelation stage, Q** | | | |
| Consistency, % | 3.0 | 3.0 | 3.0 |
| Retention time, hours | 1 | 1 | 1 |
| EDTA, % of dry matter | 0.2 | 0.2 | 0.2 |
| Temperature at the | | | |
| end, °C | 50 | 49 | 51 |
| pH at the end | 4.6 | 5.4 | 4.8 |
| **P₁ stage** | | | |
| Consistency, % | 10 | 10 | 10 |
| Retention time, hours | 3 | 3 | 3 |
| Temperature, °C | 80 | 80 | 80 |
| pH. start/end | 12.0/11.7 | 12.1/12.0 | 11.9/11.7 |
| Peroxide dosage, % | 3.0 | 3.0 | 3.0 |
| Peroxide consumed, % | 2.7 | 2.7 | 2.6 |
| Brightness, % | 59.6 | 62.3 | 63.7 |
| Kappa number | (5.9) | 6.3 | 5.3 |
| **P₂ stage** | | | |
| Consistency, % | 10 | 10 | 10 |
| Retention time, hours | 3 | 3 | 3 |
| Temperature, °C | 80 | 80 | 80 |
| pH, start/end | 12.2/11.7 | 12.2/11.7 | 12.1/11.6 |
| Peroxide dosage, % | 3.0 | 3.0 | 3.0 |
| Peroxide consumed, % | 2.2 | 2.4 | 2.2 |
| Brightness, % | 67.2 | 69.7 | 70.7 |
| Kappa number | 6.8 | 4.8 | 4.9 |
| **P₃ stage** | | | |
| Consistency, % | 10 | 10 | 10 |
| Retention time, hours | 3 | 3 | 3 |
| Temperature, °C | 80 | 80 | 80 |
| pH, start/end | 11.9/12.0 | 12.0/11.6 | 12.0/11.8 |
| Peroxide dosage, % | 3.0 | 3.0 | 3.0 |
| Peroxide consumed, % | 2.1 | 2.2 | 2.0 |
| Brightness, % | 71.3 | 74.0 | 74.4 |
| Kappa number | 5.2 | 4.1 | 2.2 |
| Total peroxide | | | |
| consumption, % | 7.0 | 7.3 | 6.8 |
| | | | |

The use of AM50 and AM35 clearly increased the brightness without significantly increasing the amount of peroxide that was consumed. Also lignin content of pulps was reduced which is evidenced by the reduction of kappa numbers of pulps treated with these enzymes.

### Example 8

### Isolation of the Chromosomal DNA and Construction of the Genomic Library

*Actinomadura flexuosa* DSM43186 was cultivated in 50 ml of medium consisting of 10% (w/v) sucrose, 0.5% (w/v) oat spelt xylan, 0.5% (w/v) peptone from casein, 0.5% (w/v) yeast extract, 0.5% (w/v) beef extract, 0.074% (w/v) CaCl₂ x 2H₂O, pH 7.4-7.5, in baffled shake flask for 2.5 days at 52 °C with shaking at 200 rpm. 2.5 ml of this culture was transferred to 50 ml of fresh medium supplemented with 0.8% glycine, and grown for 2 days at 50 °C, 200 rpm. Cells were pelleted by centrifugation and washed with 10% sucrose-25mM Tris-HCl (pH 8.0)-25mM EDTA.

The chromosomal DNA was isolated according to Hopwood et al., Genetic manipulation of Streptomyces: A laboratory manual, The John Innes Foundation, Norwich, UK (1985). Briefly, the mycelium was lysed with lysozyme and 2 x Kirby mixture (2 g sodium triisopropylnaphthalene sulphonate, 12 g sodium 4-amino-salicylate, 5 ml 2 M Tris-HCl (pH 8.0), 6 ml of Tris-HCl saturated phenol, made up to 100 ml with water). The DNA was precipitated with isopropanol and dissolved into TE (10 mM Tris-HCl, 1 mM EDTA, pH 8.0). RNA was digested with RNase.

The chromosomal DNA was partially digested with *Sau*3A (Boehringer, Germany) and size-fractionated in sucrose gradient (10-40% (w/v) sucrose in 1 M NaCl, 20 mM Tris-HCl, pH 8.0, 5 mM EDTA) run at 55 000 rpm for 6h at 22 °C in the Beckman TL-100 ultracentrifuge in the TLS-55 rotor. The gradient was divided in fractions, and those containing DNA of mainly 7-10 kb in size were used to construct a genomic *Actinomadura* library.

The predigested ZAP Express^{™} *Bam*HI/CIAP Vector Cloning Kit (Stratagene; U.S.A.) was used to construct the library and the instructions of the manufacturer were followed in all the subsequent steps. Briefly, about 200 ng of size-fractionated DNA was ligated into 1 µg of ZAP Express^{™} prepared arms, and packaged using Gigapack II packaging extract (Stratagene, U.S.A.). The titer of the library was determined by infecting *E. coli* XL1-Blue MRF cells with serial dilutions of the packaged phage and plating on NZY plates. The total titer of the ligation mixture was approximately 3 x 10⁷ pfu/ml, with over 96% insert frequency. The library was used for screening without amplification.

### Example 9A

### Isolation of the Gene Encoding the 35 kDa Xylanase on the Basis of Hydrolyzing Activity on RBB-Xylan Plates

The genomic library of *Actinomadura flexuosa* DSM43186 DNA in ZAP Express^{™} vector was screened for xylanolytic activity, as follows. The host, Stratagene *E. coli* XL-Blue MRF' cells were grown in LB + 0.2 % (w/v) maltose + 10 mM MgSO₄ and adjusted to OD₆₀₀=0.5. The cells were infected with the recombinant library for 15 min at 37 °C and plated with NZY top agar on the NZY plates. The plates were incubated for 4 hrs at 42 °C, overlaid with nitrocellulose filters saturated with 10 mM IPTG to induce the *lac*Z-fusion protein expression, and incubated over night at room temperature.

The filters were washed with 50 mM K-phosphate buffer (pH 6.8), and transferred onto RBB-xylan + kanamycin (Km) plates. The plate has two layers; lower layer of 15 ml of regular LB + Km (40 µg/ml) and upper layer of 5 ml of RBB xylan (0.5% (w/v) RBB xylan, 1% (w/v) oats spelts xylan in LB + Km, buffered with 50 mM K-phosphate, pH 6.8). The plates were transferred to 50 °C for a second night to determine xylanolytic activity. Filters were removed, and the clear halo on the RBB-xylan + Km plates revealed the clones having xylanase activity. 22 positive plaques from the original NZY-plates were picked in SM buffer/chloroform.

The ZAP Express^{™} vector has been designed to allow simple, efficient *in vivo* excision and recircularization of any cloned insert contained within the lambda vector to form a phagemid containing the cloned insert. Briefly, the positive clones were incubated with XL1 Blue MRF cells with the ExAssist helper phage. After heat denaturation (70°C, 15 min), and centrifugation, the excised phagemid pBK-CMV is packaged as filamentous phage particles in the supernatant. The rescued phagemid was mixed with XLOLR cells, and plated on LB/kanamycin (50 µg/ml) according to the manufacturer.

*E. coli* XLOLR cells transformed with the rescued phagemid DNAs were retested on RBB-xylan + Km. From the 22 originally positive clones 12 retained the xylanase activity. The phagemid DNAs were digested with *Eco*RI*-Pst*I, electrophoresed, blotted onto a nylon membrane, and hybridized with a digoxigenin-labeled 1.15 kb *T. fusca* xylanase fragment from pALK185 (Fig. 12). The plasmid pALK185 contains the *T. fusca xynA* gene from pTX101 (Ghangas, G.S. et al., J. Bact. 171:2963-2969 (1994)). Four phagemids hybridized with the *T. fusca* DNA probe, indicating that they carried gene(s) sharing some homology with the *T. fusca* fragment. These phagemids were designated pALK938, pALK939, pALK940 and pALK941. From the *A. flexuosa* DSM43186 chromosomal DNA, the *T. fusca xynA* probe hybridized to about a 4 kb *EcoRl-Pstl* fragment.

### Example 9B

### Isolation of the Gene Encoding for the 35 kDa Xylanase on the Basis of Hybridizing to the Thermomonospora fusca xynA Gene

The genomic library of *Actinomadura flexuosa* DSM43186 DNA in ZAP Express vector was screened with a digoxigenin-labeled 1.15 kb *T. fusca* xylanase fragment from pALK185 (Fig. 12), according to supplier's instructions. 17 positive clones were picked. The phagemids were excised *in vivo,* as described above in example 9A. The *E. coli* clones harboring the positive phagemids were tested for xylanolytic activity on RBB-xylan, as described above in example 9A. 11 clones showed xylanolytic activity. One of the clones was chosen, and the plasmid was designated pALK1056.

### Example 10

### Isolation of the Gene Encoding the 35 kDa Xylanase on the Basis of Production of Polypeptide recognized by the Thermomonospora fusca XynA Antibody

The polyclonal antibody against *T. fusca* 32 kDa xylanase, XynA (See Example 3), was used to screen the *Actinomadura flexuosa* DSM 43186 genomic library. Stratagene XL1-Blue MRF' cells were grown in LB + 0.2% maltose + 10 mM MgSO₄ and diluted to OD₆₀₀=0.5. The cells were infected with the recombinant library for 15 min at 37 °C and plated with NZY top agar on the NZY plates. Plates were incubated for 3.5 hours at 42 °C, overlaid with nitrocellulose filters saturated with 10 mM IPTG, and incubated overnight at room temperature. Detection was performed with the 1:1500 diluted *T. fusca* XynA antibody using Promega's ProtoBlot AP System. Twelve positive clones, of which the clone 1.1 clearly gave the strongest signal, were picked in SM buffer/chloroform, and purified with a second round of screening.

The phagemids were excised *in vivo,* as described above in example 9A. The phagemids were then digested with *Eco*RI and *Pst*I*,* electrophoresed, blotted onto a nylon membrane and hybridized with a digoxigenin-labeled 1.15 kb *T. fusca* xylanase fragment from pALK185 (Fig. 12). Of the *A. flexuosa* DSM43186 chromosomal DNA, the *T. fusca xynA* probe hybridized to about a 4 kb *Eco*RI*-Pst* fragment. The clones were also tested for xylanolytic activity on RBB-xylan, as described above in example 9A. One clone (clone 1.1) was positive in both screens. The phagemid carried by this clone was designated pALK923.

### Example 11

### Restriction Enzyme Analysis and Sequencing of the Xylanase Gene Coding for the 35 kDa Protein

The plasmids pALK938 (DSM9399), pALK939 (DSM9900), pALK940 (DSM9901), pALK941 (DSM9902), pALK1056 (DSM9903) and pALK923 (DSM9322) were analyzed by restriction enzyme analysis, and were used for sequencing of the xylanase gene. The DNA was sequenced by using ABI (Applied Biosystems, U.S.A.) kits based on fluorescent-labeled T3 and T7 primers, or sequence-specific primers with fluorescent-labelled dideoxynucleotides, by the Taq dye primer cycle sequencing protocol in accordance with the supplier's instructions. Because of the high GC content in the *A. flexuosa* DNA, the sequencing reactions were performed with 10% (v/v) DMSO. at annealing temperature of 58-60 °C. Sequencing reactions were analyzed on ABI 373A sequencer, and the sequences obtained were characterized by using the Genetics Computer Group Sequence Analysis Software Package, version 7.2. The DNA sequence encoding the 35 kDa xylanase is presented in Figure 13. The sequence shows an ORF (open reading frame) of 1035 bp, predicting a polypeptide of 344 amino acids, and corresponding to a protein with a molecular weight of about 37.5 kDa. A putative signal processing site is found after alanine 43, and the predicted mature protein has a calculated molecular weight of about 32.9 kDa. The sequence data is thus in good agreement with the 35 kDa xylanase purification and sequencing results described in Examples 3 and 4. The 35 kDa gene sequence appeared identical in all the tested clones, except in the pALK923 DNA. pALK923 contained 93 bp of unknown sequence at the N-terminus of the insert, after which the *A. flexuosa* 35 kDa xylanase gene sequence started at the location corresponding to base pair 411 in Figure 13.

The sequence shows high homology towards xylanases from different organisms. At amino acid level, the gene shows about 76 % identity towards the *T. fusca* XynA. In addition, the *A. flexuosa* 35 kDa xylanase shows 81 % identity with xylan binding domain of the *T. fusca* XynA (Irwing et.al., Appl. Env. Microbiol.60: 763-770 (1995)). Thus, *A. flexuosa* 35 kDa xylanase contains a separate xylan binding domain separated from the catalytic domain by a linker region showing only approximately 40 % identity with the linker region of *T. fusca* XynA.

### Example 12

### Isolation of the 50 kDa Actinomadura flexuosa Xylanase Gene

The genomic library of *A. flexuosa* DSM43186 DNA in ZAP Express^{™} vector was screened using a DNA probe.

Oligonucleotide primers were designed based on the peptide sequences derived from the purified 50 kDa protein. The primer sequences are presented in Table 5. Because the combination of peptide sequences #1696 (SEQ ID NO: 6), #1697 (SEQ ID NO: 7), #1698 (SEQ ID NO: 8) and #1704 (SEQ ID NO: 10) corresponds with 75% similarity to amino acids 42-89 in *Streptomyces lividans* xylanase A, a 39 bp antisense oligo was synthesized, from bases 331 to 369 in the *S. lividans xlnA* sequence. The *S*. *lividans xlnA* 331-369as probe and the primers #1704 (SEQ ID NO: 10:, #1703as, #1696s were labeled with digoxigenin and terminal transferase, and used as probes in hybridization at 50 °C according to Boehringer, DIG DNA Labeling and Detection Nonradioactive, Applications Manual.

The #1704as and the *S*. *lividans xlnA* 331-339as probe recognized the same 1.0 kb *Eco*RI*-Pst*I fragment in *A. flexuosa* DNA. The fragment is different from the 4 kb fragment recognized by the *T. fusca xynA* probe (See Example 9A). Based on these results, the S. *lividans xlnA* 331-369as probe was used to screen the *A. flexuosa* library for the 50 kDa xylanase coding gene.

Three positive plaques were picked after an overnight detection. These clones were named Act.xyl.50/13, Act.xyl.50/14 and Act.xyl.50/15.

The phagemids containing the cloned *A. flexuosa* insert were excised as described in Example 9A. To determine the xylanase activity, the *E. coli* clones were streaked on RBB-xylan + Km plates as described in Example 9A, using the strain producing the *A. flexuosa* 35 kDa xylanase (from plasmid pALK923) as a positive control. The clones Act.xy1.50/13 and Act.xyl.50/14 showed xylanase activity, giving a clear halo around the colony.

**Table 5: Oligonucleotide primers used in the detection of the gene coding for the A. flexuosa 50 kDa xylanase**

| **Primer** | **DNA sequence** |
|---|---|
| *Actinomadura* sp. DSM43186 | |
| #1696s | GCA/C/G/TGCA/C/G/TCAA/G/CAC/TAAC/TA/CG |
| #1703as | ACCATA/GTTA/GTAA/C/G/TACA/C/G/TA |
| #1704as | TTCATC/TTCA/GTTC/TTCA/C/G/TGC |
| *S. lividans xlnA* 331-369as | CGTGAGTTCAACATGGTGACGGCCGAGAACGAGATGAAG |
| *S. lividans xlnA* 257-284s | AGAGCGGCCGCTACTTCGGCACCGCCAT |
| *S. lividans xlnA* 530-561as | CACGCCGTTGATGTGGTCGATCATCGCCTGGC |

| | |
|---|---|
| s = sense; as = antisense | |

### Example 13

### Sequencing the Gene for 50 kDa Actinomadura flexuosa Xylanase Protein

The phagemid DNAs from the Act.xyl.50/13 and Act.xyl.50/14 were named pALK927 and pALK928, respectively. The *S. lividans xlnA* 331-369as oligomer was used to sequence the *A. flexuosa* insert. In addition, two oligomers corresponding to nucleotides 257-284 and 530-561 in the S. *lividans xlnA* sequence, as well as sequence-specific primers, were synthesized to obtain sequence from the cloned insert. The sequencing reactions were performed with 10% (v/v) DMSO, at the annealing temperature of 58 °C. The sequencing was performed as described in Example 11. The sequence of the 1864 bps of the *A. flexuosa* DSM43186 50 kDa xylanase gene is presented in Figure 14. Peptide sequences obtained from the purified 50 kDa protein are indicated by underlining of the derived amino acid sequence. The derived peptide sequence shows 70-71 % identity towards *Actinomadura* sp. FC7 xylanase II (Figure 15A) and *S*. *lividans* xylanase A (Figure 15B) proteins. The sequence shows an ORF of 1479 bps, predicting a polypeptide of 492 amino acids, corresponding to a protein with a molecular weight of about 53.5 kDa.

### Example 14

### Production of Bacterial (Actinomycetous) Enzymes in Trichoderma reesei:Production of Thermomonospora fusca Xylanase without a Fusion

The expression cassette pALK193 (Fig. 16) was constructed for expression of the *T. fusca* xylanase gene, *xyn*A (Ghangas et al., J. Bacteriol. 171:2963-2969 (1989); Irwin et al., Appl. & Environ. Microbiol. 60:763-770 (1994)) in *T. reesei.* In the expression cassette, the *T. fusca* xylanase gene is fused to the *T. reesei* cellobiohydrolase 1 *(cbh1)* signal sequence that is preceeded by the *cbh1* promoter. The 9.4 kb pALK193 expression cassette was cut from the vector backbone by *Eco*RI restriction. It was then isolated, purified and transformed into *T. reesei* ALKO2221 strain.

The expression fragment pALK193 contains:
** T. reesei cbh1* promoter and signal sequence: The approximately 2.2 kb promoter sequence was derived from the plasmid pAMH110 (EP 244 234 - Fig. 15) and was originally isolated from *T. reesei* strain VTT-D-80133 (Teeri et al., Bio/Technol. 1: 696-699 (1983)). The sequence of the signal sequence and the promoter area preceeding the ATG was published by Shoemaker et al., Bio/Technology 1: 691-696 (1983)). In the *T. reesei* strain VTT-D-80133 the sequence preceeding the ATG is CCGCGGACTGCGCATC (a *Sac*II site is underlined, an additional cytosine in the DNA sequence, compared to the sequence by Shoemaker et al. Bio/Technology 1: 691-696 (1983) is bolded).
   To make an exact fusion of the *T. fusca xynA* gene to the *cbh1* signal sequence, the 12 nucleotides after the *Sfi*I site in the *cbh1* signal sequence and the 5'-end of the *T. fusca* xylanase gene (to the internal *Mlu*I site, see Fig. 12 and 16) were synthesized by using polymerase chain reaction (PCR).
** T. fusca* xylanase gene (xynA): The cloning of the *xynA* gene is published in Ghangas et al., J. Bacteriol. 171:2963-2969 (1989), and the sequence of the gene is published in Irwin et al., Appl. & Environ. Microbiol. 60:763-770 (1994). The sequence coding for the mature enzyme was fused (exact fusion) to the *cbh1* signal sequence. About a 0.7 kb *xynA* terminator region, to the *Sma*I site after the STOP codon of the *xynA* coding region, precedes the *cbh1* terminator fragment in the construction.
** T. reesei cbh1* terminator: The 0.7 kb *Ava*II terminator fragment starting 113 bp before the STOP of the *cbh1* gene was added after the *T. fusca xynA* gene, to ensure termination of transcription. The terminator fragment derived from the plasmid pAMH 110 (originally isolated from *T. reesei* strain VTT-D-80133; Teeri et al., Bio/Technol. 1: 696-699 (1983)) and it contains three TAA codons in all reading frames preceding the terminator fragment (from *Nde*I site, see EP 244 234).

*A. nidulans amdS* gene: The gene has been isolated from *Aspergillus nidulans* VH1-TRSX6. It encodes acetamidase (Hynes et al., Mol. Cell. Biol. 3: 1430-1439 (1983)). Acetamidase enables the strain to grow by using acetamide as the only nitrogen source and this characteristic was used for selecting the transformants. The 3.1 kb fragment (*Spe*I *- Xba*I) from the plasmid p3SR2 (Kelly and Hynes, EMBO J. 4: 475-479 (1985)) is used in the plasmids. The fragment contains 1007 bps of the promoter area, 1897 bps of the coding region (introns included) and the 183 bps terminator area of the *amdS* gene.

*T. reesei cbhl* 3'-fragment:The fragment was isolated from *T. reesei* ALKO2466 by using plasmid rescue (1.7 kb, *Bam*HI *- Eco*RI*,* starting 1.4 kb after the gene's STOP codon, Suominen et al., "High frequency one-step gene replacement in Trichoderma reesei II. Effects of deletions of individual cellulase genes," Mol. Gen Genet. 241: 523-530 (1993)).
Strain ALKO2466 derives from the strain ALKO233 (Harkki et al., Enzyme Microb. Technol. 13: 227-233 (1991)). The 3'-fragment is used together with the promoter area (described above) to target the *T. fusca xynA* gene to the *cbh1* locus by homologous recombination.

Standard DNA methods were used for construction of the vectors (Sambrook et al., "Molecular Cloning: A Laboratory Manual", 2nd ed. Cold Spring Harbor Laboratory Press, Cold Spring Harbor, N.Y., 1989). The restriction enzymes. T4 DNA ligase, Klenow fragment of the DNA polymerase I, T4 DNA polymerase, polynucleotide kinase and *Taq* polymerase were from Boehringer (Germany) and New England Biolabs (U.S.A.). Each enzyme was used according to the supplier's instructions. Plasmid DNA was isolated by using Qiagen columns (Qiagen GmbH, Germany) or Promega Magic Minipreps (Promega, U.S.A.) according to the manufacturer's protocols. The oligonucleotides used in the PCR-reactions and in sequencing reactions were synthesized by a ABI (Applied Biosystems, U.S.A.) 381A DNA Synthesizer. DNA sequencing was done using ABI kits based on fluorescence-labelled primers, or when sequence-specific primers were used, on fluoresence-labelled dideoxynucleotides, by the *Taq* cycle sequencing method according to the supplier's instructions. Sequencing reactions were analyzed on an ABI 373A sequencer.

DNA fragments for cloning or transformations were isolated from low-melting- point agarose gels (FMC Bioproducts, U.S.A.) by freeze-thaw-phenol method (Benson, Biotechniques 2:66-58 1984) or by using Qiaex II Gel Extraction Kit (Qiagen GmbH, Germany), the GeneClean^{R} or Mermaid Kits^{™} (Bio 101 Inc., U.S.A.) according to the supplier's instructions.

*T. reesei* ALKO2221 was transformed with the isolated 9.4 kb *EcoRI* expression cassette of pALK193 (Fig. 16) as described by Penttilä et al., Gene 61: 155-164 (1987)) with the modifications described in Karhunen et al., Mol. Gen. Genet 241: 515-522 (1993). *T. reesei* transformants were transferred on a selective medium and purified through conidia. Transformants were stabilized by growing them on selective slants for two generations prior to sporulating on potato dextrose agar.

The culture supernatants of the transformants were analyzed by measuring the xylanase activity produced, by running samples on SDS-PAGE and by performing Western blots. Polyclonal antibody against *T. fusca* xylanase and purified *T. fusca* xylanase used in the Western blots were obtained from Prof. David Wilson (Cornell University, New York).

The xylanase activity from the culture supernatants was measured at 6.5, 60 °C and with 5 minutes incubation time. Prior to the assay the supernatants were incubated at 60 °C for 20 hours in 50 mM Mcllvain's buffer with 200 µg/ml BSA to destroy *T. reesei's* own xylanase activity. *T. fusca* supernatant's xylanase activity was unaffected by the 20 hours' incubation used prior to the reaction but the incubation used did destroy host's xylanase activity. The xylanase activity produced by the best pALK193 transformants was about 900 nkat/ml.

The *T. fusca* xylanase produced by the *T. reesei* transformants was not detectable on SDS-PAGE but was detected on the Western blots. The amount of the *T. fusca* xylanase produced by the transformants, based on the estimation from the Western blots where purified *T. fusca* xylanase was used as a control, was about 50-100 mg/l.

### Example 15

### Production of Bacterial (Actinomycete) Enzymes in Trichoderma reesei:Production of Trichoderma reesei β-mannanase and Actinomadura flexuosa Xylanase Fusions

The *Trichoderma reesei* strains were constructed for *Actinomadura flexuosa* xylanase production (35 kDa xylanase, AM35). Strains overproduce *A. flexuosa* xylanase and are unable to produce *T. reesei's* endoglucanase II and cellobiohydrolase I. Such cellulolytic activity-deficient preparations, and the making of same by recombinant DNA methods, are described in US 5,298,405 or Suominen et al., Mol. Gen. Genet. 241: 523-530 (1993). For the overproduction of *A. flexuosa* xylanase, the *am35* gene was fused to the *T. reesei* mannanase 1 gene's core/hinge region and the gene fusion was expressed from the strong *cbhl* promoter. Different protease cleavage sites were added between the mannanase and xylanase encoding sequences.

The plasmids pALK945, pALK948, pALK1021 and pALK1022 (Fig. 17) containing the sequences SEQ ID NO: 11:, SEQ ID NO: 13:, SEQ ID NO: 15 and SEQ ID NO: 17:, respectively. The plasmids were used in the construction of the *A. flexuosa* xylanase overproducing strains and are otherwise identical to each other, except that the fusion between the *manl* core/hinge and *am35* sequences differs (see Fig. 19, and below).

The plasmids pALK945, pALK948, pALK1021 and pALK1022 contain the following elements:
* *cbh1* (cellobiohydrolase 1) promoter: The promoter is from *Trichoderma reesei* VTT-D-80133 (Teeri et al., Bio/Technology 1: 696-699 (1983)). The 2.2 kb *EcoRI - Sac*II fragment (Karhunen et al. , Mol. Gen. Genet. 241: 515-522 (1993)) is used in the constructs. The sequence preceeding the ATG was published by Shoemaker et al., Bio/Technology 1: 691-696 (1983)). In the *T. reesei* strain VTT-D-80133 the sequence preceeding the ATG is CCGCGGACTGCGCATC (the SacII site is underlined, an additional cytosine in the DNA sequence, compared to the sequence by Shoemaker et al. Bio/Technology 1: 691-696 (1983), is bolded).
To make an exact fusion, the 10 nucleotides of the promoter, from the SacII site to the ATG, and the 5'-end of the *man1* gene (to the internal *Cla*I site, see Fig. 17) were synthesized by using polymerase chain reaction (PCR).
* the *man1* gene's core/hinge region: The *man1* gene codes for β-mannanase that degrades mannans/glucomannans (Stålbrand et al., Appl. Environ. Microbiol. 61: 1090-1097 (1995)). The gene has been isolated from *T. reesei* QM6a and its sequence is known (Stålbrand *et al.,* 1995). The 1.35 kb DNA fragment from nucleotides 1 to 1346 coding for the *man1* core/hinge region (amino acids from 1 to 379) was used in plasmids pALK945 and pALK948. The DNA fragment from nucleotides 1 to 1359 (amino acids 1 to 383) was used in plasmids pALK1021 and pALK1022. The *manl* core/hinge region was linked, from its C-terminal end, to the *am35* gene by using the PCR method to obtain four different fusions.
* the *am35* gene: The nucleotide sequence and deduced amino acid sequence of the am35 gene encoding a 35 kDa xylanase is presented in Fig. 13. The gene was cloned from a genomic library of *Actinomadura flexuosa* DSM43186 by using a plate activity assay (Example 9A). A 1.3 kb fragment from nucleotide 542 (the N-terminal Asp44) to the *Mlu*I site about 250 bps after the end of the gene (pALK1055, Fig. 18) was used in all plasmids. The gene was linked, from its N-terminal end, to the *man1* core/hinge sequence by using four different fusions.
* the *manl* core/hinge - am35 fusions: *manl* core/hinge was fused to the AM35 with or without a KEX-linker sequence, marked as ...KR..., representing ...Lys-Arg... in the list below. The fusion was done by PCR and the following amino acid sequences were formed (see Fig. 19 for the DNA sequences):
*man1* core/hinge + **synthetic sequence** + *am35 sequence*

| | | |
|---|---|---|
| pALK945 | ...PLYG**R***DTT*... | = additional R |
| pALK948 | ...PLYG**RDKR***DTT*... | = KEX2-linker added |
| pALK1021 | ...PLYGQCGG*DTT*... | = no new amino acids |
| pALK1022 | ...PLYGQCGG**RDKR***DTT*... | = KEX2-linker added |

A *Nru*I restriction site (TCGCGA) was introduced to pALK945 and pALK948 linkers to aid construction of the linkage between the two sequences. This was done by changing the native codon encoding glycine 379 in the *man1* core/hinge region (GGC) to a synthetic codon (GGT) and selecting the codon **CGC** for arginine. The N-temiinal Asp44 of the am35 is encoded by **GAC**.

The fusion sequences were sequenced to ensure that no unwanted alterations had taken place.

The linker sequence used in the plasmid pALK945 has been used in the production of murine anti-2-phenyloxazolone IgG1 antibody from *T. reesei* as a fusion to the cellobiohydrolase I core/hinge region (WO 92/01797; Nyyssönen et al., Bio/Technology 11: 591-595 (1993)). The fusions were cleaved at a low frequency by an extracellular, hitherto uncharacterisized *T. reesei* protease. The cleavage was made after the tyrosine residue in the CBHI linker region, two amino acids before the authentic N-terminus of the heavy chain Fd chain.

The linker sequences in the plasmids pALK948 and pALK1022 carried a synthetic spacer peptide, containing a KEX2-like protein processing signal, preceding the mature *Actinomadura* xylanase.
* the *cbh1* terminator: The 739 bp *Ava*II fragment (Karhunen et al., Mol. Gen. Genet. 241: 515-522 (1993)) starting 113 bp before the STOP of the *cbh1* gene was added after the am35 gene to ensure termination of transcription.
* the *amdS* gene: The fragment containing the amdS gene was the same as used in the construction of *T. fusca xynA* expression plasmid, see Example 14.
* the *cbh1* 3'-fragment: The fragment was the same as used in the construction of *T. fusca xynA* expression plasmid, see Example 14. The 3'-fragment is used together with the promoter area (described above) to target the *man1-am35* gene fusion to the *cbh1* locus by homologous recombination.

Standard DNA methods used in the construction of vectors pALK945, pALK948, pALK1021 and pALK1022 are described in Example 16. The 10.3 kb expression cassette was cut from the vector backbone by *Eco*RI restriction. The expression cassettes were then isolated, purified and transformed into ALKO3620 as described in Example 16, but other *Trichoderma* strains can be used as hosts as well.

In the host strain ALKO3620 the endoglucanase 2 *(egl2)* gene has been replaced by the 3.3 kb BgIII - XbaI fragment from the plasmid pAN8-1 (Mattern et al., Fungal Genet. Newlett. 35: 25 (1988)). This fragment contains a transformation marker gene, *ble* from *Streptoalloteichus hindustanus* (Drocourt et al., Nucl. Acids Res. 18: 4009 (1990)). The *ble* gene confers resistance to several antibiotics, e.g. phleomycin and it is, in the construct, expressed from *Aspergillus nidulans gpdA* (glyseraldehyde-3-phosphate-dehydrogenase) promoter, *A. nidulans trpC* terminator is used to terminate the transcription. The replacement was done by using the recombinant DNA methods described in US 5,298,405.

### Example 16

### Characteristics of the Actinomadura flexuosa Xylanase Producing Transformants

Several purified *amdS* transformants were grown in shake flasks in a medium containing 4% whey, 1.5 % complex nitrogen source derived from grain, 1.5 % KH₂PO₄ and 0.5% (NH₄)₂SO₄. Cultures were maintained at 30 °C and 250 rpm for 7 days. *A. flexuosa* was cultivated in 1 1 fermentor as described in Example 1.

The culture supernatants were subjected to SDS-polyacrylamide gel electrophoresis (PAGE). CHBI was detected by Western blotting and immunostaining using a CBHI specific monoclonal antibody (CI-258 (Aho et al., Eur. J. Biochem. 200: 643-649 (1991)) and the ProtoBlot Western blot AP system (Promega, U.S.A.) according to the recommendations of the manufacturer.

Some of the CBHI negative transformants were chosen for further characterization: e.g. the recombinant *T. reesei* strains ALKO3620/pALK945/8, ALKO3620/pALK945/6, ALKO3620/pALK948/27, ALKO3620/pALK1021/4 and ALKO3620/pALK1022/29 do not contain the *cbh1* gene. The *cbh1* gene is replaced by the *amdS* marker gene and the *manl-am35* fusion construct in pALK945, pALK948, pALK1021, pALK1022 expression cassettes. The *cbh1* gene replacement was verified in Southern hybridisations. The host strain ALK03620 used in the transformations is deficient of the *egl2* gene (see Example 15) Thus, the strains do not produce *Trichoderma's* cellulase components EGII and CBHI.

Xylanase activities were measured at pH 5.3 and 50 °C, with an incubation time of 5 minutes in 50 mM Na₂HPO₄ buffer and pH 7 and 70 °C, with an incubation time of 5 minutes in 50 mM Mcllvain's buffer, containing 100 µg/ml of BSA. The xylanase activity of one transformant per each transformation is presented in Table 6. The corresponding production levels of the transformants as estimated from the specific activity of purified *A*. *flexuosa* AM35 xylanase (18 000 BXU/mg at pH 7.0 and 70 °C) are shown in Table 6. Another shake flask cultivation was performed which contained two *cbh1* negative transformants obtained from the pALK945 transformation. Xylanase activities were measured at pH 7, 70 °C with an incubation time of 60 minutes. Results are presented in Table 6.

**Table 6:**

| **The xylanase activity and estimated production level of *T. reesei* transformants producing A. *flexuosa* 35 kDa xylanase.** | | | | |
|---|---|---|---|---|
| **xylanase** | **BXU/ml (pH 5.3,50°C, 5 min)** | **BXU/ml (pH 7, 70°C, 60 min)** | **BXU/ml pH 7, 70°C, 5 min)** | **AM35 g/l (estimate)** |
| *A. flexuosa* culture medium | 190 | ND | 440 | 0.02* |
| ALKO3620 | 3560 | 10 | 360 | 0 |
| ALKO3620/ pALK945/6 | ND | 6230 | ND | ND |
| ALKO3620 pALK945/8 | 10970 | 8350 | 14970 | 0.83 |
| ALKO3620/ pALK948/27 | 6690 | 7010 | 11920 | 0.66 |
| ALKO3620/ pALK1021/4 | 8400 | 6630 | 9800 | 0.54 |
| ALKO3620/ pALK1022/29 | 7940 | 7410 | 14870 | 0.83 |

| | | | | |
|---|---|---|---|---|
| * Total xylanase activity in *A. flexuosa* culture medium. | | | | |

The xylanase activity of the *T. reesei* host strain ALKO3620 is about ten times lower at optimum conditions for the AM35 protein (pH 7 and 70°C) than it is under the conditions optimal for the *T. reesei* xylanase (pH 5.3, 50 °C). The lowest activity and production level was obtained with a transformant containing a fusion construct without any protease processing site (ALO3620/pALK1021/4).

For the thermal stability determination, samples from the culture supernatants were diluted in 50mM McIlvain's buffers. BSA was added as a carrier protein to the concentration of 100 µg/ml. The xylanase activity was measured by incubating the enzyme samples at 70°C, pH 7.0 for a period of 0, 15, 30 and 120 minutes after which the xylanase activity of the samples was determined at pH 7, 70°C, using a 5 minutes reaction time. The results are shown in Figure 20. The thermal stability of the AM35 protein produced by recombinant *T. reesei* strains transformed with the pALK945 and pALK1022 plasmids was identical with *A. flexuosa* xylanase. The AM35 protein produced by pALK948 and pALK1021 transformants was less stable (Figure 20).

Samples from the culture supernatants were run on polyacrylamide slab gels containing 0.1% SDS on Bio-Rad Mini Protean II electrophoresis system. A polyclonal antibody prepared against the purified β-mannanase (pI 5.4) of *T. reesei* RutC30 (Stålbrand et al., Appl. Environ. Microbiol. 61: 1090-1097 (1995)) was used to detect the mannanase in Western blots. In the detection, Promega's ProtoBlot AP System was used. The Western result is shown in Fig. 21. The molecular weight of the β-mannanase protein in the culture medium of the host strain ALKO3620 (lane 5) and of all the transformants (lanes 6-10) is somewhat larger than that of the purified 53 kDa β-mannanase protein sample (Stålbrand et al., J. Biotechnol. 29: 229-242 (1993)), (lanes 2 and 3). In addition to the native β-mannanase, the transformants ALKO3620/pALK945/8 ALKO3620/pALK945/6, ALKO3620/pALK948/27 and ALK03620/pALK1022/29 (lanes 6-9) produce a smaller protein (about 50 kDa) reacts with the polyclonal mannanase antibody. This band represents the shortened mannanase protein obtained from the fusion constructs, and shows that the extracellular proteases have processed the fusion. In the strain ALK03620/pALK1021/4 (lane 10) two bands with molecular weights of about 70 and 80 kDa are obtained. These bands originate from the unprocessed fusion protein (MANI core/hinge + AM35 mature protein).

The culture supernatants were analysed also with a polyclonal antibody raised against the purified 35 kDA xylanase of *A.flexuosa* (Example 18). The result is presented in Figure 22. Transformants ALKO3620/pALK945/6, ALKO3620/pALK948/27 and ALKO3620/pALK10 22/29 showed one to two major bands on the Western blots. These bands were estimated to be 37-39 kDa and migrated parallel to the purified 35 kDa xylanase of *A. flexuosa.* In another transformant obtained from the transformation with pALK945 expression cassette the major band had a molecular weight of 31 kDa. Transformant ALK03620/pALK1021/4 showed two protein bands of about 70-80 kDa which correlated with the molecular weight of the unprocessed fusion protein. The transformant also produced the processed form of the fusion protein.

When plain actinomycetes xylanase is expressed in *T. reesei* as a fusion of a homologous gene, high production levels of the heterologous protein can be achieved. When *Actinomadura am35* gene was expressed under the *cbh1* promoter as a mannanase fusion, the level of xylanase produced was about 500-800 mg/l. When *T. fusca xynA* was expressed in *T. reesei* by linking it to the same promoter (*cbh1*), without a fusion to homologous gene, only 50-100 mg/l of xylanase was produced. Identity between the two actinomycetes xylanases, *Actinomadura* xylanase AM35 and *T. fusca* xylanase A is 76% at amino acid level.

### Example 17

### Bleaching Experiments Using the Actinomadura flexuosa Xylanase Synthesized in Trichoderma reesei as a Mannanase Fusion Protein

Bleaching experiments were done to determine the usefulness of the *A. flexuosa* AM35 xylanase activity synthesized in *Trichoderma reesei* as a mannanase fusion protein in TCF (totally chlorine free) bleaching of kraft pulp.

The culture media of transformants ALKO3620/pALK945/8, ALKO3620/pALK948/27 and ALKO3620/pALK1022/29 (Example 16) were added to Finnish oxygen delignified softwood kraft pulp (kappa number 16) in the amount of 100 nkat/g pulp dry matter. Xylanase activity was measured at pH 7 and 70 °C with 60 minutes incubation time. The enzyme treatments were done at pH 7 and 80 °C for one hour. Reference pulp was treated in the same way but without enzyme addition. Bleaching was performed using QP sequence. Metals were first removed by chelation with EDTA (chelation stage, Q) and the pulps were then bleached with hydrogen peroxide (peroxide stage, P). The results are shown in Table 7.

**Table 7**

| | **Reference** | **ALKO3620 pALK945/8** | **ALKO3620 pALK948/27** | **ALKO3620 pALK1022/29** |
|---|---|---|---|---|
| **Enzyme treatment** | | | | |
| Consistency, % | 3.5 | 3.5 | 3.5 | 3.5 |
| Retention time, | | | | |
| hours | 1 | 1 | 1 | 1 |
| Enzyme dosage, | | | | |
| nkat/g | 0 | 100 | 100 | 100 |
| Temperature, °C, | | | | |
| start/end | 81/79 | 82/80 | 80/77 | 83/79 |
| pH, start/end | 7.1/6.9 | 6.9/6.9 | 7.0/7.0 | 7.0/7.3 |
| **Chelation stage, Q** | | | | |
| Consistency, % | 3.0 | 3.0 | 3.0 | 3.0 |
| Retention time, | | | | |
| hours | 1 | 1 | 1 | 1 |
| EDTA, % of dry | | | | |
| matter | 0.2 | 0.2 | 0.2 | 0.2 |
| Temperature at | | | | |
| the end, °C | 75 | 76 | 74 | 76 |
| pH at the end | 5.2 | 5.1 | 5.0 | 4.9 |
| **Peroxide stage, P** | | | | |
| Consistency, | 10 | 10 | 10 | 10 |
| Retention time, | | | | |
| hours | 3 | 3 | 3 | 3 |
| Temperature, °C | 80 | 80 | 80 | 80 |
| pH, start/end | 11.3/10.8 | 11.4/10.8 | 11.4/10.8 | 11.4/10.9 |
| Peroxide dosage, % | 3.0 | 3.0 | 3.0 | 3.0 |
| Peroxide consumed, % | 2.3 | 2.3 | 2.3 | 2.3 |
| Brightness, % | 64.2 | 66.2 | 65.5 | 65.9 |
| Viscosity, ml/g | 840 | 850 | 850 | 870 |

The use of *A. flexuosa* xylanase activity containing culture media of transformants ALKO3620/pALK945/8, ALKO3620/pALK948/27 and ALKO3620/pALK1022/29 as a pretreatment of pulp in peroxide bleaching at 80 °C increased the brightness (2 units at its best) of the pulps obtained without increasing the amount of peroxide that was consumed. Viscosity of the pulps was not reduced because of low contaminating cellulase activity in the culture media.

Bleaching experiment was also performed at pH 7 and 70 °C for one hour using the same culture media as above. The brightness values of the final pulps were similar (1.2 units at its best) than when the enzyme pretreatments were performed at pH 7 and 80 °C.

### Reference Example 18

### Purification and Characterization of the Recombinant Actinomadura flexuosa 35 kDa Xylanase

A 0.5 mg sample of purified 35 kDa xylanase from *A. flexuosa* DSM43186 was sent to Diabor Ltd (Kiviharjuntie 11 A 4, FIN-90220 Oulu, Finland) in order to raise polyclonal antibodies in rabbits. The titer of the produced antibody was good, and a dilution of 1:10.000 was suitable for Western blots.

The protein products of the 35 kDa A. flexuosa DSM43186 xylanase gene in *T. reesei* of both ALK03620/pALK945/8 and ALKO3620/pALK945/6 were identified by running samples of growth medium obtained from fermentor cultivations on SDS-PAGE followed by Western blotting with the wild-type 35 kDa xylanase antibody. Western blotting was performed with the ProtoBlot Western blot AP system (Promega, U.S.A) according to the manufacturer's instructions. Prestained Bio-Rad (LMW-standards) standard proteins were used as molecular mass standards.

The different bands identified from ALKO3620/pALK945/8 depended on growth conditions and growth time. As a whole, five different bands reacted with the 35 kDa xylanase antibody (Figure 23A). The upper band was estimated to be a 80 kDa protein. The four other band were estimated to be 27, 30, 31 and 34 kDa. *A. flexuosa* (wild type) 35 kDa xylanase was on the same gel estimated to be 39 kDa. Thus, the gene of ALKO3620/pALK945/8 did not produce a xylanase band of the same size as the wild-type xylanase. The mass of the upper band, 80 kDa, correlates well with the intact fusion protein. ALKO3620/pALK945/6 showed two to three bands on the western blots, also depending on growth conditions (Figure 23B). The upper band was estimated to about 80 kDa and thus probably the intact fusion protein. The main band on the blots of ALKO3620/pALK945/6 was estimated to 39 kDa and migrated parallel to the wild-type 35 kDa xylanase. Growth medium samples after prolonged growth of the ALKO3620/pALK945/6 strain showed also a faint 31 kDa band on Western blots. The occurrence of smaller size xylanases after prolonged growth of the fungus suggests that post translational modifications, e.g. proteolysis, are present.

The samples of ALKO3620/pALK945/8 and ALKO3620/pALK945/6 above were rerun on SDS-PAGE and blotted on PVDF membrane. The bands obtained were cut out from the PVDF blot and subjected to Edman degradation as described in Example 4. All bands showed the same amino terminal sequence i.e. SEQ ID NO: 5: as the wild-type 35 kDa xylanase (D-T-T-I-T-Q-). Thus, C-terminal modifications must account for the observed differences in molecular masses.

In order to further characterize the gene products of the recombinant 35 kDa *A. flexuosa* xylanase, the different molecular mass xylanases were purified from suitable growth mediums of ALKO3620/pALK945/8 and ALKO3620/pALK945/6. Purification was essentially performed as described in Example 3. Samples of 40 to 150 ml growth medium, depending on the estimated xylanase content, were adjusted to pH 9.1 with 1 M NaOH. The samples were centrifuged at 11.000 g (+4 °C for 20 min). The supernatants were adjusted to 3.7 mS/cm and separately applied on a 5 x 18 cm DEAE-Sepharose FF (Pharmacia) column equilibrated with 20 mM Na₂HPO₄, pH 8.6, at a flow rate of 30 ml/min. Most of the *T. reesei* proteins bound to the DEAE, but the xylanase activity was found in the flow-through.

The flow-through fractions containing xylanase activity were pooled and NaCl was added to 2 M. The pool was applied on a 5 x 13 cm Phenyl-Sepharose FF (Pharmacia) equilibrated at a flow rate of 20 ml/min with 40 mM Na₂HPO₄ , pH 8.6, containing 2 M NaCl. Elution was performed at a flow rate of 20 ml/min with a gradient from 100% equilibration buffer to 100% 40 mM Na₂HPO₄,pH 8.6, in 20 min. The column was further washed with this buffer for 10 min. Elution was continued for 30 min with a gradient from 100 % 40 mM Na₂HPO₄ pH 8.6, to 100 % 40 mM Na₂HPO₄, pH 8.6, containing 60 % ethylene glycol. Fractions of 10 ml were collected and assayed for xylanase activity, on SDS-PAGE as well as on Western blots. Fractions containing purified xylanases were pooled and stored at -20 °C. Wild-type 35 kDa xylanase was purified as above from a 840 ml growth medium sample of *A. fleruosa* (DSM43186).

The purified recombinant *A. flexuosa* xylanases and the wild-type 35 kDa xylanase were run on SDS-PAGE (Figure 24). From ALKO3620/pALK945/6 a purified 39 kDa xylanase was obtained and from ALK03620/pALK945/8 a 30 kDa and a 27 kDa xylanase. These, as well as the wild-type 35 kDa xylanase, were subjected to mass spectrometric analysis on a Brucker Biflex Reflector MALDI-TODF (Brucker-Franzeen GmbH. Germany) mass spectrometer. The wild-type 35 kDa xylanase showed a mass of 32 857. in well agreement to the mass calculated from the *A. flexuosa* 35 kDa gene (32 876), Example 11. Thus, wild-type 35 kDa xylanase seems to be unglycosylated. The 39 kDa xylanase from ALKO3620/pALK945/6 gave a mass of 33 429 by mass spectrometry. Since the N-terminal of this sample was identical with the wild-type xylanase, probably either a C-terminal extension or glycosylation accounted for the 572 Da deference in mass. There are no stop codons in the gene sequence, following the wild-type stop codon, which could account for the larger mass of the recombinant xylanase. Thus, probably one or more of the five potential N-glycosylation sites (Asn-X-Ser/Thr, X=/Pro) have been glycosylated in *T. reesei.* The two 30 and 27 kDa xylanases purified from ALKO3620/pALK945/8 had the same N-terminal sequence as the wild-type 35 kDa xylanase. On the mass spectrometer their masses were determined to 23 974 and 21 974 respectively. The estimated molecular masses from SDS-PAGE were thus larger than the masses determined by mass spectrometry. The SDS-PAGE 39 kDa xylanase was renamed to 33.4 kDa and the 30 kDa xylanase to 23.8 kDa and the 27 kDa xylanase to 22 kDa. The wild-type *A. flexuosa* xylanase name was kept as 35 kDa xylanase.

The protein concentration of the purified xylanases was determined at A₂₀₅ by the method of Scopes (Scopes, Anal. biochem. 59: 277-287, 1974). The Kₘ values and the k_{cat} values (based on protein concentration and molecular mass) of the purified xylanases were determined at pH 7 and 70 °C in the substrate range from 2 to 25 mg/ml (birch xylan, Roth 7 500). The k_{cat} values obtained were slightly smaller for both the 35 and 33.4 kDa xylanases as compared to the 22 and 23.8 kDa xylanases (Table 8). However, the Kₘ values for the 22 and 23.8 kDa xylanases (approximately 10 mg/ml) were 2.5 x higher than the corresponding Kₘ values of the 33.4 and 35 kDa xylanases (approximately 4 mg/ml). As judged from the protein sequence of the 35 kDa gene, the 22 and 23.8 kDa xylanase products miss the C-terminal xylan binding domain (see Example 11) resulting in less efficient binding to xylan. Roughly estimated the 22 kDa form contains only the core domain without the linker region. The 23.8 kDa form contains in addition part of the linker region.

Determination of the pI of the purified xylanases was performed by running samples on a 0.5 x 20 cm mono P chromatofocusing column (Pharmacia) equilibrated with 0.075 mM Tris-HCl pH 9.3. Elution was performed with polybuffer (10 ml polybuffer in 100 ml water and adjusted to pH 6 with 1 M acetic acid) at a flow rate of 0.5 ml/min and fractions of 0.5 ml were collected. The pH and xylanase activity of the fractions were determined, and the xylanase activity was found in the fraction corresponding to its pl (Table 8).

**Table 8**

| **Xylanase Strain (kDa)** | | **mass (kDa)** | **Km (mg/ml)** | **kcat (1/s)** | **pI** |
|---|---|---|---|---|---|
| 33.4 | ALKO3620/pALK945/6 | 33.429 | 3.8 | 1397 | 8.6 |
| 23.8 | ALKO3620/pALK945/8 | 23.769 | 9.8 | 1525 | 7.6 |
| 22 | ALKO3620/pALK945/8 | 21.974 | 10.0 | 1682 | 8.2 |
| 35 | DSM43186 | 32.857 | 4.5 | 1135 | 8.5 |

The temperature and pH dependence of the purified xylanases were determined by incubating samples for 60 min with substrate in the pH range of 5.1 to 7.9 and at temperatures of 60, 70 and 80 °C, essentially as described in Example 5. From the results, it seems that the full length xylanases, 35 and 33.4 kDa, are slightly more active at higher temperatures and pH than the shorter, 22 and 23.8 kDa, xylanases (Figure 25).

The purified xylanases were tested for thermal stability by incubating samples at 80 °C and both pH 5 and pH 7 (in 50 mM McIlvains buffer). Samples were withdrawn at suitable time intervals, and the residual activity was measured at pH 7 and 70 °C (5 min incubation time). The half-lives of the xylanases are shown in Table 9. Both 22 and 23.8 kDa xylanases shoved a longer half-life than the full length 33.4 and 35 kDa xylanases under the conditions tested. This stability difference could be a result of the presence of the separate binding domain in the full length xylanases, destabilizing the structure at high temperatures in the absence of substrate. In the temperature dependence experiments (giving slightly different results), the presence of substrate binding to the xylan-binding domain may, on the contrary, stabilize the full length xylanases.

**Table 9**

| **Xylanase (kDa)** | **t_{1/2} (min) (80°C, pH 5)** | **t_{1/2} (min) (80°C, pH 7)** |
|---|---|---|
| 33.4 | 13 | 17 |
| 23.8 | 157 | 95 |
| 22 | 123 | 63 |
| 35 | 32 | 31 |

Apparently the truncations of the 35 kDa xylanase from the C-terminal end has some, but not severe, effects on the kinetic features of this xylanase. A 22 kDa fragment of the 35 kDa xylanase is still active and shows similar kinetics as its longer counterparts.

### Reference Example 19

### Bleaching Experiments Using Purified 22.0 kDa, 23.8 kDa and 33.4 kDa Forms of AM35 Xylanase from Actinomadura flexuosa

The bleach boosting effect of purified forms 22.0 kDa, 23.8 kDa and 33.4 kDa of AM35 xylanase (Example 18) was tested in one stage peroxide bleaching.

The purified forms were added to Finnish oxygen delignified softwood kraft pulp in the amount of 100 nkat / pulp dry matter as such without a protecting agent (results in Table 10) and with *T. reesei* culture medium suitable for protective background (results in Table 11). The xylanase activity of the purified forms as well as the *T. reesei* culture medium were measured at pH 7 70 °C with 5 minutes incubation time. The enzyme treatments were done at pH 8 80 °C for one hour. Reference pulps were treated in the same way but without enzyme addition. Bleachings were performed using QP sequence. Metal ions were first removed by adding EDTA 0.2 % of pulp dry matter (chelation stage, Q). The pulps were then bleached with hydrogen peroxide (P) using the following chemicals: 3 % H₂O₂, 3 % NaOH, 0.2 % DTPA and 0.5 % MgSO₄. The conditions of Q and P stages are shown in Tables 10 and 11.

**Table 10**

| | **Reference** | **22.0 kDa** | **23.8 kDa** | **33.4 kDa** |
|---|---|---|---|---|
| **Enzyme treatment** | | | | |
| Consistency, % | 3.5 | 3.5 | 3.5 | 3.5 |
| Retention time, hours | 1 | 1 | 1 | 1 |
| Enzyme dosage, nkat/g | 0 | 100 | 100 | 100 |
| Temperature, °C, | | | | |
| start/end | 80/79 | 80/79 | 79/79 | 81/80 |
| pH, start/end | 8.3/8.2 | 8.1/8.0 | 8.2/8.1 | 8.2/8.1 |
| **Chelation stage, Q** | | | | |
| Consistency, % | 3.0 | 3.0 | 3.0 | 3.0 |
| Retention time, hours | 1 | 1 | 1 | 1 |
| EDTA, % of dry matter | 0.2 | 0.2 | 0.2 | 0.2 |
| Temperature at the | | | | |
| end, °C | 73 | 73 | 72 | 74 |
| pH at the end | 5.8 | 5.6 | 5.7 | 5.8 |
| **Peroxide stage, P** | | | | |
| Consistency, % | 10 | 10 | 10 | 10 |
| Retention time, hours | 3 | 3 | 3 | 3 |
| Temperature, °C | 80 | 80 | 80 | 80 |
| pH, start/end | 11.3/10.9 | 11.31/10.8 | 11.3/10.7 | 11.3/10.7 |
| Peroxide dosage, % | 3.0 | 3.0 | 3.0 | 3.0 |
| Peroxide consumed, % | 2.3 | 2.3 | 2.3 | 2.3 |
| Brightness, % | 64.7 | 65.0 | 65.6 | 65.5 |

The 33.4 kDa and 23.8 kDa forms of AM35 xylanase seemed to boost the bleaching effect in peroxide bleaching when pH was 8, temperature 80 °C, time one hour and enzyme dosage 100 nkat/g of dry pulp. The 22.0 kDa form did not enhance bleachability probably because of the missing substrate binding domain and incomplete linker region. Generally the increase of brightness was quite small, 0.9 units at its best. This might be due to the fact that the purified enzymes were added as such to 80 °C pulp without any protecting agent or carrier protein, such as *T. reesei* culture medium or BSA.

The second bleaching experiment was carried out the same way as the first one. Only the purified enzyme forms were first mixed with a *T. reesei* culture medium and then added to the pulps. This culture medium was similar to the culture medium from which the enzyme forms were purified. The results are shown in Table 11.

The result of the second bleaching experiment was similar to the first one. The treatments with the 33.4 kDa and 23.8 kDa forms increased brightness about 1.5 units compared with the treatment where only *T. reesei* culture medium was used. Also 22 kDa form gave about 1 unit brightness increase. The results show that the *T. reesei* culture medium worked as a protecting background and 33.4 kDa and 23.8 kDa protein forms, whose linker regions between catalytic and binding domains are intact, increased brightness values more than the 22 kDa form.

**Table 11**

| | **Reference** | **22.0 kDa** | **23.8 kDA** | **33.4 kDa** | **T.reesei culture medium used as a protection background** |
|---|---|---|---|---|---|
| **Enzyme** | | | | | |
| **treatment** | | | | | |
| Consistency, % | 3.5 | 3.5 | 3.5 | 3.5 | 3.5 |
| Retention | | | | | |
| time, hours | 1 | 1 | 1 | 1 | 1 |
| Enzyme dosage, | 0 | 100 | 100 | 100 | 100* |
| nkat/g | | (+100*) | (+100*) | (+100*) | |
| Temperature, | | | | | |
| °C. start/end | 81/80 | 80/80 | 80/80 | 82/82 | 81/80 |
| pH. start/end | 7.9/7.8 | 7.6/7.7 | 7.6/7.7 | 7.6/7.6 | 7.6/7.6 |
| **Chelation stage, Q** | | | | | |
| Consistency, % | 3.0 | 3.0 | 3.0 | 3.0 | 3.0 |
| Retention | | | | | |
| time, hours | 1 | 1 | 1 | 1 | 1 |
| EDTA, % of | | | | | |
| dry matter | 0.2 | 0.2 | 0.2 | 0.2 | 0.2 |
| Temperature at | | | | | |
| the end, °C | 76 | 75 | 75 | 76 | 77 |
| pH at the end | 5.2 | 5.2 | 5.2 | 5.2 | 5.1 |
| **Peroxide stage, P** | | | | | |
| Consistency, % | 10 | 10 | 10 | 10 | 10 |
| Retention | | | | | |
| time, hours | 3 | 3 | 3 | 3 | 3 |
| Temperature, °C | 80 | 80 | 80 | 80 | 80 |
| pH, start/end | 11.4/ | 11.4/ | 11.5/ | 11.5/ | 11.6/ |
| | 10.7 | 10.7 | 10.8 | 10.8 | 10.8 |
| Peroxide | | | | | |
| dosage, % | 3.0 | 3.0 | 3.0 | 3.0 | 3.0 |
| Peroxide | | | | | |
| consumed, % | 2.2 | 2.3 | 2.3 | 2.3 | 2.3 |
| Brightness, % | 62.1 | 64.1 | 64.6 | 64.5 | 63.0 |

| | | | | | |
|---|---|---|---|---|---|
| *) The xylanase activity is originated in the *T. reesei* culture medium consisting *T. reesei's* own xylanases. | | | | | |

### Reference Example 20

### Production of 23.8 kDa form of Actinomadura flexuosa 35 kDa xylanase in Trichoderma reesei

*T. reesei* strains are constructed for the production of xylanolytically active but shortened, still catalytically active fragments of the full-length actinomycete enzymes. Especially, *T*. *reesei* strains for the production of the 23.8 kDa form (see example 18) of *A. flexuosa* 35 kDa xylanase are constructed. The expression of the shortened form can lead to enhanced xylanase activity in the culture medium.

A recombinant vector encoding a xylanase or a desired domain of it is prepared by fusing the sequence encoding xylanase or a desired domain of it with the sequence of a secretable fungal protein or one or more functional domains of said protein. Especially, the sequence encoding the 23.8 kDa form of the *A. flexuosa* 35 kDa xylanase is fused to *T. reesei* cellulase or hemicellulase as described in US 5,298,405, WO 93/24621 and Stålbrand et al., Appl.Environ.Microbiol. 61: 1090-1097 (1995). Especially, the enzyme is selected from the group consisting of CBHI, CBHII, EGI, EGII, XYLI, XYLII and MANI, or a functional domain thereof.

Fusion proteins can be constructed that contain an N-terminal mannanase, xylanase, cellobiohydrolase or endoglucanase core domain or the core and the hinge domains from the same, fused to the *A. flexuosa* xylanase sequence encoding the 23.8 kDa form. The result is a protein that contains N-terminal mannanase, xylanase, cellobiohydrolase or endoglucanase core or core and hinge regions and a C-terminal *A. flexuosa* 23.8 kDa form. The fusion protein contains both the mannanase, xylanase, cellobiohydrolase or endoglucanase and xylanase activities of various domains as provided in the fusion construct.

Fusion proteins can also be constructed such that the mannanase or cellobiohydrolase or endoglucanase tail or a desired fragment thereof, is included, placed before the *A. flexuosa* xylanase sequence, especially so as to allow use of a nonspecific protease site in the tail as a protease site for the recovery of the cellulase sequence from the expressed fusion protein. Alternatively, fusion proteins can be constructed that provide for a protease site in a linker that is placed before the *A. flexuosa* xylanase cellulase, with or without tail sequences.

### Reference Example 21

### Production of Thermomonospora fusca Cellulases in Trichoderma reesei

*T. fusca* produces at least six cellulase degrading enzymes, four endoglucanases E1, E2, E4 and E5 and two exocellulases E3 and E6 (Irwin et al., Biotechnol. and Bioeng. 42: 1002-1013 (1993)). *T. reesei* strains are constructed for efficient production of *T. fusca* cellulases. Especially, *T. reesei* strains are constructed that express *T. fusca* endocellulase, E5. The cloning of the E5 gene is described in Lao et al. , J . Bacteriol. 173: 3397-3407 (1991).

A recombinant vector encoding a cellulase is prepared by fusing the sequence encoding a polypeptide with cellolytic activity with the sequence of a secretable fungal protein or at least a functional domain of said protein. Especially the *T. fusca* cellulase encoding sequence is fused to *T. reesei* cellulase or hemicellulase or one or more functional domains of said cellulase or hemicellulase, as described in US 5,298,405, WO 93/24621 and Stålbrand et al., Appl. Environ. Microbiol. 61: 1090-1097 (1995). Especially, the enzyme is selected from the group consisting of CBHI, CBHII, EGI, EGII, XYLI, XYLII and MANI, or a domain thereof, such as secretion signal or the core sequence.

Fusion proteins can be constructed that contain an N-terminal mannanase, xylanase, cellobiohydrolase or endoglucanase core domain or the core and the hinge domains from the same, fused to the *T. fusca* cellulase sequence. The result is a protein that contains N-terminal mannanase, xylanase, cellobiohydrolase or endoglucanase core or core and hinge regions and a C-terminal *T. fusca* cellulase. The fusion protein contains both the mannanase, xylanase, cellobiohydrolase or endoglucanase and cellulase activities of various domains as provided in the fusion construct.

Fusion proteins can also be constructed such that the mannanase or cellobiohydrolase or endoglucanase tail or a desired fragment thereof, is included, placed before the *T. fusca* cellulase sequence, especially so as to allow use of a nonspecific protease site in the tail as a protease site for the recovery of the cellulase sequence from the expressed fusion protein. Alternatively, fusion proteins can be constructed that provide for a protease site in a linker that is placed before the *T. fusca* cellulase, with or without tail sequences.

### Reference Example 22

### Bacterial Protein Expressed in Aspergillus

A recombinant vector encoding a bacterial enzyme is prepared by fusing the enzyme encoding sequence to an Aspergillus secretable protein. The protein is preferably A. niger or A. niger var awamori glucoamylase or α-amylase; or one or more functional domains thereof. (Stoffer et al., Biochem. J. 292:197-202 (1993); Svensson et al., Structure-Function relationship in amylases, Ed. R.B. Friedman. Biotechnology of Amylodextrin Oligosaccharides. ACS Symposium Serium 458:28-43 (1991); Boel et al., EMBO J. 3:1581-1585 (1984), Boel et al., EMBO J. 3: 1097-1102 (1984), Korman et al, Curr.Genet 17: 203-212, (1990)

Fusion proteins can be constructed that contain an N-terminal glucoamylase or α-amylase or one or more functional domains or from the same, fused to a sequence encoding bacterial protein such as Actinomadura xylanase. The result is a protein that contains N-terminal glucoamylase or α-amylase or part of them, and a C-terminal Actinomadura xylanase. The fusion protein contains both the mannanase or glucoamylase or α-amylase and xylanase activities of the various domains as provided in the fusion construct.

Fusion proteins can also be constructed such that e.g. glucoamylase tail or a desired fragment thereof, is included, placed before the *Actinomadura* xylanase sequence, especially so as to allow use of a nonspecific protease site in the tail as a protease site for the recovery of the xylanase sequence from the expressed fusion protein. Alternatively, fusion proteins can be constructed that provide for a protease site in a linker that is placed before the *Actinomadura* xylanase, with or without tail sequences.

The expression vector uses e.g. *A. niger* glucoamylase promoter. (Boel et al., EMBO J. 3:1581-1585 (1984)). The transformation host may be some *Aspergillus niger* strain (Kelly and Hynes, EMBO J. 4:475-479 (1985)) or for example some *Aspergillus niger var awamori* strain (e.g. ATCC 38854). The chosen *Aspergillus* strain is transformed similar to that described by Kelly and Hynes, EMBO J. 4:475-479 (1985)).

The *Actinomadura* xylanase producing transformants are then characterized similar to Example 16 with modification obvious to a person skilled in the art. The culture medium used may be *Aspergillus* complete medium. (Rowlands et al., Mol. Gen. Genet. 126: 201-216 (1973)).

### SEQUENCE LISTING

(1) GENERAL INFORMATION:
   (i) APPLICANT:
      (A) NAME: Primalco Ltd
      (B) STREET: Valta-akseli
      (C) CITY: Nurmijarvi
      (E) COUNTRY: Finland
      (F) POSTAL CODE (ZIP): FIN-05200
      (G) TELEPHONE: +358 9 133 11
      (H) TELEFAX: +358 9 133 1546
   (ii) TITLE OF INVENTION: Production and Secretion of Proteins of Bacterial Origin in Filamentous Fungi
   (iii) NUMBER OF SEQUENCES: 18
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 1375 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Actinomadura flexuosa
      (B) STRAIN: DSM43186
   (ix) FEATURE:
      (A) NAME/KEY: CDS
      (B) LOCATION:303..1337
      (D) OTHER INFORMATION:/product= "AM35 xylanase"
   (xi) SEQUENCE DESCRIPTION: SEQ ID NO: 1:
   CCCGGGTATT CATGTGAATG ATTAGCAACA GTTATGTTAC GGAGATATTT CTGAGAGTGT    60
   TGACAGGTCG TGAAGTCGGT CCGATACTTT CGAGCTAGCT CCGATAGTTT TCGATACGCC   120
   GGCACATCGA GCACGTCGGA CGAGTCACGC GCCACGTCGG TTTTCCGCCG CACGCCGCGC   180
   AGAGCGGCCG GAGAACCCCC GCGTGTCCGC GGCATCGGTG CCGGTCCGTC GTTCGCCGCC     240
   GACCGCGCGC CGGGTCGCGA CACGCCAGCC CCCATCGGCC CTTCTTCACG AGGAAGCCGT     300
   AC ATG AAC GAA CCC CTC ACC ATC ACG CAG GCC AGG CGC CGC AGA CGC        347
      Met Asn Glu Pro Leu Thr Ile Thr Gln Ala Arg Arg Arg Arg Arg
      1               5                   10                  15
   CTC GGC CTC CGG CGC ATC GTC ACC AGT GCC TTC GCC CTG GCA CTC GCC       395
   Leu Gly Leu Arg Arg Ile Val Thr Ser Ala Phe Ala Leu Ala Leu Ala
                   20                  25                  30
   ATC GCC GGT GCG CTG CTG CCC GGC ACG GCC CAC GCC GAC ACC ACC ATC       443
   Ile Ala Gly Ala Leu Leu Pro Gly Thr Ala His Ala Asp Thr Thr Ile
               35                  40                  45
   ACC CAG AAC CAG ACC GGG TAC GAC AAC GGC TAC TTC TAC TCG TTC TGG       491
   Thr Gln Asn Gln Thr Gly Tyr Asp Asn Gly Tyr Phe Tyr Ser Phe Trp
           50                  55                  60
   ACC GAC GCG CCC GGG ACC GTC TCC ATG ACC CTC CAC TCG GGC GGC AGC       539
   Thr Asp Ala Pro Gly Thr Val Ser Met Thr Leu His Ser Gly Gly Ser
       65                  70                  75
   TAC AGC ACC TCG TGG CGG AAC ACC GGG AAC TTC GTC GCC GGC AAG GGC       587
   Tyr Ser Thr Ser Trp Arg Asn Thr Gly Asn Phe Val Ala Gly Lys Gly
   80                  85                  90                  95
   TGG TCC ACC GGG GGA CGG CGG ACC GTG ACC TAC AAC GCC TCC TTC AAC       635
   Trp Ser Thr Gly Gly Arg Arg Thr Val Thr Tyr Asn Ala Ser Phe Asn
                   100                 105                 110
   CCG TCG GGT AAC GGC TAC CTC ACG CTC TAC GGC TGG ACC AGG AAC CCG       683
   Pro Ser Gly Asn Gly Tyr Leu Thr Leu Tyr Gly Trp Thr Arg Asn Pro
               115                 120                 125
   CTC GTC GAG TAC TAC ATC GTC GAG AGC TGG GGC ACC TAC CGG CCC ACC       731
   Leu Val Glu Tyr Tyr Ile Val Glu Ser Trp Gly Thr Tyr Arg Pro Thr
           130                 135                 140
   GGC ACC TAC AAG GGC ACC GTC ACC ACC GAC GGG GGA ACG TAC GAC ATC       779
   Gly Thr Tyr Lys Gly Thr Val Thr Thr Asp Gly Gly Thr Tyr Asp Ile
       145                 150                 155
   TAC GAG ACC TGG CGG TAC AAC GCG CCG TCC ATC GAG GGC ACC CGG ACC       827
   Tyr Glu Thr Trp Arg Tyr Asn Ala Pro Ser Ile Glu Gly Thr Arg Thr
   160                 165                 170                 175
   TTC CAG CAG TTC TGG AGC GTC CGG CAG CAG AAG CGG ACC AGC GGC ACC       875
   Phe Gln Gln Phe Trp Ser Val Arg Gln Gln Lys Arg Thr Ser Gly Thr
                   180                 185                 190
   ATC ACC ATC GGC AAC CAC TTC GAC GCC TGG GCC CGC GCC GGC ATG AAC        923
   Ile Thr Ile Gly Asn His Phe Asp Ala Trp Ala Arg Ala Gly Met Asn
               195                 200                 205
   CTG GGC AGC CAC GAC TAC CAG ATC ATG GCG ACC GAG GGC TAC CAG AGC        971
   Leu Gly Ser His Asp Tyr Gln Ile Met Ala Thr Glu Gly Tyr Gln Ser
           210                 215                 220
   AGC GGT AGC TCC ACC GTC TCC ATC AGC GAG GGT GGC AAC CCC GGC AAC       1019
   Ser Gly Ser Ser Thr Val Ser Ile Ser Glu Gly Gly Asn Pro Gly Asn
       225                 230                 235
   CCG GGT AAC CCC GGC AAC CCC GGC AAC CCC GGT AAC CCG GGT AAC CCC       1067
   Pro Gly Asn Pro Gly Asn Pro Gly Asn Pro Gly Asn Pro Gly Asn Pro
   240                 245                 250                 255
   GGC GGT GGC TGC GTC GCG ACC CTC TCC GCC GGC CAG CAG TGG AGC GAC       1115
   Gly Gly Gly Cys Val Ala Thr Leu Ser Ala Gly Gln Gln Trp Ser Asp
                   260                 265                 270
   CGC TAC AAC CTC AAC GTC TCG GTC AGC GGC TCG AAC AAC TGG ACG GTC       1163
   Arg Tyr Asn Leu Asn Val Ser Val Ser Gly Ser Asn Asn Trp Thr Val
               275                 280                 285
   CGG ATG GAC GTG CCC TAC CCG GCC CGC ATC ATC GCC ACC TGG AAC ATC       1211
   Arg Met Asp Val Pro Tyr Pro Ala Arg Ile Ile Ala Thr Trp Asn Ile
           290                 295                 300
   CAC GCC CAG TGG CCC GAG TCC CAG GTG CTC ATC GCC AGA CCC AAC GGC       1259
   His Ala Gln Trp Pro Glu Ser Gln Val Leu Ile Ala Arg Pro Asn Gly
       305                 310                 315
   AAC GGC AAC AAC TGG GGC GTG ACG ATC CAG CAC AAC GGC AAC TGG ACC       1307
   Asn Gly Asn Asn Trp Gly Val Thr Ile Gln His Asn Gly Asn Trp Thr
   320                 325                 330                 335
   TGG CCG ACG GTC ACC TGT ACC GCG AAC TGA GTTCCCGCCC CCAAAGGTGG         1357
   Trp Pro Thr Val Thr Cys Thr Ala Asn  *
                   340                 345
   CGCGGCGGCT CCCGGCCG                                                   1375
(2) INFORMATION FOR SEQ ID NO: 2:
   (i) SEQUENCE CHARACTERISTICS:
      (A) LENGTH: 345 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Asn Glu Pro Leu Thr Ile Thr Gln Ala Arg Arg Arg Arg Arg Leu
   1 5 10 15
   Gly Leu Arg Arg Ile Val Thr Ser Ala Phe Ala Leu Ala Leu Ala Ile
               20                  25                  30
   Ala Gly Ala Leu Leu Pro Gly Thr Ala His Ala Asp Thr Thr Ile Thr
           35                  40                  45
   Gln Asn Gln Thr Gly Tyr Asp Asn Gly Tyr Phe Tyr Ser Phe Trp Thr
       50                  55                  60
   Asp Ala Pro Gly Thr Val Ser Met Thr Leu His Ser Gly Gly Ser Tyr
   65                  70                  75                  80
   Ser Thr Ser Trp Arg Asn Thr Gly Asn Phe Val Ala Gly Lys Gly Trp
                   85                  90                  95
   Ser Thr Gly Gly Arg Arg Thr Val Thr Tyr Asn Ala Ser Phe Asn Pro
               100                 105                 110
   Ser Gly Asn Gly Tyr Leu Thr Leu Tyr Gly Trp Thr Arg Asn Pro Leu
           115                 120                 125
   Val Glu Tyr Tyr Ile Val Glu Ser Trp Gly Thr Tyr Arg Pro Thr Gly
       130                 135                 140
   Thr Tyr Lys Gly Thr Val Thr Thr Asp Gly Gly Thr Tyr Asp Ile Tyr
   145                 150                 155                 160
   Glu Thr Trp Arg Tyr Asn Ala Pro Ser Ile Glu Gly Thr Arg Thr Phe
                   165                 170                 175
   Gln Gln Phe Trp Ser Val Arg Gln Gln Lys Arg Thr Ser Gly Thr Ile
               180                 185                 190
   Thr Ile Gly Asn His Phe Asp Ala Trp Ala Arg Ala Gly Met Asn Leu
           195                 200                 205
   Gly Ser His Asp Tyr Gln Ile Met Ala Thr Glu Gly Tyr Gln Ser Ser
       210                 215                 220
   Gly Ser Ser Thr Val Ser Ile Ser Glu Gly Gly Asn Pro Gly Asn Pro
   225                 230                 235                 240
   Gly Asn Pro Gly Asn Pro Gly Asn Pro Gly Asn Pro Gly Asn Pro Gly
                   245                 250                 255
   Gly Gly Cys Val Ala Thr Leu Ser Ala Gly Gln Gln Trp Ser Asp Arg
               260                 265                 270
   Tyr Asn Leu Asn Val Ser Val Ser Gly Ser Asn Asn Trp Thr Val Arg
           275                 280                 285
   Met Asp Val Pro Tyr Pro Ala Arg Ile Ile Ala Thr Trp Asn Ile His
       290                 295                 300
   Ala Gln Trp Pro Glu Ser Gln Val Leu Ile Ala Arg Pro Asn Gly Asn
   305                 310                 315                 320
   Gly Asn Asn Trp Gly Val Thr Ile Gln His Asn Gly Asn Trp Thr Trp
                   325                 330                 335
   Pro Thr Val Thr Cys Thr Ala Asn  *
               340                 345
(2) INFORMATION FOR SEQ ID NO: 3:
   (i) SEQUENCE CHARACTERISTICS:
      (A) LENGTH: 1864 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Actinomadura flexuosa
      (B) STRAIN: DSM43186
   (ix) FEATURE:
      (A) NAME/KEY: CDS
      (B) LOCATION:194..1672
      (D) OTHER INFORMATION:/product= "AM50 xylanase"
   (xi) SEQUENCE DESCRIPTION: SEQ ID NO: 3:
   TTCGGCAGCC TATTGACAAA TTTCGTGAAT GTTTCCCACA CTTGCTCTGC AGACGGCCCC      60
   GCCGATCATG GGTGCACCGG TCGGCGGGAC CGTGCTCCGA CGCCATTCGG GGGTGTGCGC     120
   CTGCGGGCGC GGCGTCGATC CCGCGGGGAC TCCCGCGGTT CCCTTTCCGT GTCCCTCTAA     180
   TGGAGGCTCA GGC ATG GGC GTG AAC GCC TTC CCC AGA CCC GGA GCT CGG        229
                  Met Gly Val Asn Ala Phe Pro Arg Pro Gly Ala Arg
                                  350                 355
   CGG TTC ACC GGC GGG CTG TAC CGG GCC CTG GCC GCG GCC ACG GTG AGC       277
   Arg Phe Thr Gly Gly Leu Tyr Arg Ala Leu Ala Ala Ala Thr Val Ser
           360                 365                 370
   GTG GTC GGC GTG GTC ACG GCC CTG ACG GTG ACC CAG CCC GCC AGC GCC       325
   Val Val Gly Val Val Thr Ala Leu Thr Val Thr Gln Pro Ala Ser Ala
       375                 380                 385
   GCG GCG AGC ACG CTC GCC GAG GGT GCC GCG CAG CAC AAC CGG TAC TTC       373
   Ala Ala Ser Thr Leu Ala Glu Gly Ala Ala Gln His Asn Arg Tyr Phe
   390                 395                 400                 405
   GGC GTG GCC ATC GCC GCG AAC AGG CTC ACC GAC TCG GTC TAC ACC AAC      421
   Gly Val Ala Ile Ala Ala Asn Arg Leu Thr Asp Ser Val Tyr Thr Asn
                   410                     415                     420
   ATC GCG AAC CGC GAG TTC AAC TCG GTG ACG GCC GAG AAC GAG ATG AAG      469
   Ile Ala Asn Arg Glu Phe Asn Ser Val Thr Ala Glu Asn Glu Met Lys
               425                     430                     435
   ATC GAC GCC ACC GAG CCG CAG CAG GGG CGG TTC GAC TTC ACC CAG GCC      517
   Ile Asp Ala Thr Glu Pro Gln Gln Gly Arg Phe Asp Phe Thr Gln Ala
           440                     445                     450
   GAC CGG ATC TAC AAC TGG GCG CGC CAG AAC GGC AAG CAG GTC CGC GGC      565
   Asp Arg Ile Tyr Asn Trp Ala Arg Gln Asn Gly Lys Gln Val Arg Gly
       455                     460                     465
   CAC ACC CTG GCC TGG CAC TCG CAG CAG CCG CAG TGG ATG CAG AAC CTC      613
   His Thr Leu Ala Trp His Ser Gln Gln Pro Gln Trp Met Gln Asn Leu
   470                     475                     480                     485
   AGC GGC CAG GCG CTG CGC CAG GCG ATG ATC AAC CAC ATC CAG GGG GTC      661
   Ser Gly Gln Ala Leu Arg Gln Ala Met Ile Asn His Ile Gln Gly Val
                   490                     495                     500
   ATG TCC TAC TAC CGG GGC AAG ATC CCG ATC TGG GAC GTG GTG AAC GAG      709
   Met Ser Tyr Tyr Arg Gly Lys Ile Pro Ile Trp Asp Val Val Asn Glu
               505                     510                     515
   GCG TTC GAG GAC GGA AAC TCC GGC CGC CGG TGC GAC TCC AAC CTC CAG      757
   Ala Phe Glu Asp Gly Asn Ser Gly Arg Arg Cys Asp Ser Asn Leu Gln
           520                     525                     530
   CGC ACC GGT AAC GAT TGG ATC GAG GTC GCG TTC CGC ACC GCC CGC CAG      805
   Arg Thr Gly Asn Asp Trp Ile Glu Val Ala Phe Arg Thr Ala Arg Gln
       535                     540                     545
   GGG GAC CCC TCG GCC AAG CTC TGC TAC AAC GAC TAC AAC ATC GAG AAC      853
   Gly Asp Pro Ser Ala Lys Leu Cys Tyr Asn Asp Tyr Asn Ile Glu Asn
   550                     555                     560                     565
   TGG AAC GCG GCC AAG ACC CAG GCG GTC TAC AAC ATG GTG CGG GAC TTC      901
   Trp Asn Ala Ala Lys Thr Gln Ala Val Tyr Asn Met Val Arg Asp Phe
                   570                     575                     580
   AAG TCC CGC GGC GTG CCC ATC GAC TGC GTG GGC TTC CAG TCG CAC TTC      949
   Lys Ser Arg Gly Val Pro Ile Asp Cys Val Gly Phe Gln Ser His Phe
               585                     590                     595
   AAC AGC GGT AAC CCG TAC AAC CCG AAC TTC CGC ACC ACC CTG CAG CAG      997
   Asn Ser Gly Asn Pro Tyr Asn Pro Asn Phe Arg Thr Thr Leu Gln Gln
           600                     605                     610
   TTC GCG GCC CTC GGC GTG GAC GTC GAG GTC ACC GAG CTG GAC ATC GAG     1045
   Phe Ala Ala Leu Gly Val Asp Val Glu Val Thr Glu Leu Asp Ile Glu
       615                     620                     625
   AAC GCC CCG GCC CAG ACC TAC GCC AGC GTG ATC CGG GAC TGC CTG GCC      1093
   Asn Ala Pro Ala Gln Thr Tyr Ala Ser Val Ile Arg Asp Cys Leu Ala
   630                 635                 640                 645
   GTG GAC CGC TGC ACC GGC ATC ACC GTC TGG GGT GTC CGC GAC AGC GAC      1141
   Val Asp Arg Cys Thr Gly Ile Thr Val Trp Gly Val Arg Asp Ser Asp
                   650                 655                 660
   TCC TGG CGC TCG TAC CAG AAC CCG CTG CTG TTC GAC AAC AAC GGC AAC      1189
   Ser Trp Arg Ser Tyr Gln Asn Pro Leu Leu Phe Asp Asn Asn Gly Asn
               665                 670                 675
   AAG AAG CAG GCC TAC TAC GCG GTG CTC GAC GCC CTG AAC GAG GGC TCC      1237
   Lys Lys Gln Ala Tyr Tyr Ala Val Leu Asp Ala Leu Asn Glu Gly Ser
           680                 685                 690
   GAC GAC GGT GGC GGC CCG TCC AAC CCG CCG GTC TCG CCG CCG CCG GGT      1285
   Asp Asp Gly Gly Gly Pro Ser Asn Pro Pro Val Ser Pro Pro Pro Gly
       695                 700                 705
   GGC GGT TCC GGG CAG ATC CGG GGC GTG GCC TCC AAC CGG TGC ATC GAC      1333
   Gly Gly Ser Gly Gln Ile Arg Gly Val Ala Ser Asn Arg Cys Ile Asp
   710                 715                 720                 725
   GTG CCG AAC GGC AAC ACC GCC GAC GGC ACC CAG GTC CAG CTG TAC GAC      1381
   Val Pro Asn Gly Asn Thr Ala Asp Gly Thr Gln Val Gln Leu Tyr Asp
                   730                 735                 740
   TGC CAC AGC GGT TCC AAC CAG CAG TGG ACC TAC ACC TCG TCC GGT GAG      1429
   Cys His Ser Gly Ser Asn Gln Gln Trp Thr Tyr Thr Ser Ser Gly Glu
               745                 750                 755
   TTC CGC ATC TTC GGC AAC AAG TGC CTG GAC GCG GGC GGC TCC AGC AAC      1477
   Phe Arg Ile Phe Gly Asn Lys Cys Leu Asp Ala Gly Gly Ser Ser Asn
           760                 765                 770
   GGT GCG GTG GTC CAG ATC TAC AGC TGC TGG GGC GGC GCC AAC CAG AAG      1525
   Gly Ala Val Val Gln Ile Tyr Ser Cys Trp Gly Gly Ala Asn Gln Lys
       775                 780                 785
   TGG GAG CTC CGG GCC GAC GGC ACC ATC GTG GGC GTG CAG TCC GGG CTG      1573
   Trp Glu Leu Arg Ala Asp Gly Thr Ile Val Gly Val Gln Ser Gly Leu
   790                 795                 800                 805
   TGC CTC GAC GCG GTG GGT GGC GGC ACC GGC AAC GGC ACG CGG CTG CAG      1621
   Cys Leu Asp Ala Val Gly Gly Gly Thr Gly Asn Gly Thr Arg Leu Gln
                   810                 815                 820
   CTC TAC TCC TGC TGG GGC GGC AAC AAC CAG AAG TGG TCC TAC AAC GCC      1669
   Leu Tyr Ser Cys Trp Gly Gly Asn Asn Gln Lys Trp Ser Tyr Asn Ala
               825                 830                 835
   TGA TCCCCGGCTG ATCGACCCTA GTTGAGGCCG TCTCCGGTAC GGCACCGTCG 1722
    *
   GACCGGAGGC GGTCCCTTGT TCGTCCAGGA CGGAAGGACC GGTCTGAGCA GGCGCGGCGA 1782
   TCGGACACCA TGGTGGGAGG CACGAAAGCG GGAGGGGGTC GTATTCCGAG ACTCCGGGAA 1842
   GTGGAGGTGT TCCTCCACCT GA 1864
(2) INFORMATION FOR SEQ ID NO: 4:
   (i) SEQUENCE CHARACTERISTICS:
      (A) LENGTH: 493 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Gly Val Asn Ala Phe Pro Arg Pro Gly Ala Arg Arg Phe Thr Gly
    1               5                  10                  15
   Gly Leu Tyr Arg Ala Leu Ala Ala Ala Thr Val Ser Val Val Gly Val
               20                  25                  30
   Val Thr Ala Leu Thr Val Thr Gln Pro Ala Ser Ala Ala Ala Ser Thr
           35                  40                  45
   Leu Ala Glu Gly Ala Ala Gln His Asn Arg Tyr Phe Gly Val Ala Ile
       50                  55                  60
   Ala Ala Asn Arg Leu Thr Asp Ser Val Tyr Thr Asn Ile Ala Asn Arg
   65                  70                  75                  80
   Glu Phe Asn Ser Val Thr Ala Glu Asn Glu Met Lys Ile Asp Ala Thr
                   85                  90                  95
   Glu Pro Gln Gln Gly Arg Phe Asp Phe Thr Gln Ala Asp Arg Ile Tyr
               100                 105                 110
   Asn Trp Ala Arg Gln Asn Gly Lys Gln Val Arg Gly His Thr Leu Ala
           115                 120                 125
   Trp His Ser Gln Gln Pro Gln Trp Met Gln Asn Leu Ser Gly Gln Ala
       130                 135                 140
   Leu Arg Gln Ala Met Ile Asn His Ile Gln Gly Val Met Ser Tyr Tyr
   145                 150                 155                 160
   Arg Gly Lys Ile Pro Ile Trp Asp Val Val Asn Glu Ala Phe Glu Asp
                   165                 170                 175
   Gly Asn Ser Gly Arg Arg Cys Asp Ser Asn Leu Gln Arg Thr Gly Asn
               180                 185                 190
   Asp Trp Ile Glu Val Ala Phe Arg Thr Ala Arg Gln Gly Asp Pro Ser
           195                 200                 205
   Ala Lys Leu Cys Tyr Asn Asp Tyr Asn Ile Glu Asn Trp Asn Ala Ala
       210                 215                 220
   Lys Thr Gln Ala Val Tyr Asn Met Val Arg Asp Phe Lys Ser Arg Gly
   225                 230                 235                 240
   Val Pro Ile Asp Cys Val Gly Phe Gln Ser His Phe Asn Ser Gly Asn
                   245                 250                 255
   Pro Tyr Asn Pro Asn Phe Arg Thr Thr Leu Gln Gln Phe Ala Ala Leu
               260                 265                 270
   Gly Val Asp Val Glu Val Thr Glu Leu Asp Ile Glu Asn Ala Pro Ala
           275                 280                 285
   Gln Thr Tyr Ala Ser Val Ile Arg Asp Cys Leu Ala Val Asp Arg Cys
       290                 295                 300
   Thr Gly Ile Thr Val Trp Gly Val Arg Asp Ser Asp Ser Trp Arg Ser
   305                 310                 315                 320
   Tyr Gln Asn Pro Leu Leu Phe Asp Asn Asn Gly Asn Lys Lys Gln Ala
                   325                 330                 335
   Tyr Tyr Ala Val Leu Asp Ala Leu Asn Glu Gly Ser Asp Asp Gly Gly
               340                 345                 350
   Gly Pro Ser Asn Pro Pro Val Ser Pro Pro Pro Gly Gly Gly Ser Gly
           355                 360                 365
   Gln Ile Arg Gly Val Ala Ser Asn Arg Cys Ile Asp Val Pro Asn Gly
       370                 375                 380
   Asn Thr Ala Asp Gly Thr Gln Val Gln Leu Tyr Asp Cys His Ser Gly
   385                 390                 395                 400
   Ser Asn Gln Gln Trp Thr Tyr Thr Ser Ser Gly Glu Phe Arg Ile Phe
                   405                 410                 415
   Gly Asn Lys Cys Leu Asp Ala Gly Gly Ser Ser Asn Gly Ala Val Val
               420                 425                 430
   Gln Ile Tyr Ser Cys Trp Gly Gly Ala Asn Gln Lys Trp Glu Leu Arg
           435                 440                 445
   Ala Asp Gly Thr Ile Val Gly Val Gln Ser Gly Leu Cys Leu Asp Ala
       450                 455                 460
   Val Gly Gly Gly Thr Gly Asn Gly Thr Arg Leu Gln Leu Tyr Ser Cys
   465 470 475 480
   Trp Gly Gly Asn Asn Gln Lys Trp Ser Tyr Asn Ala *
   485 490
(2) INFORMATION FOR SEQ ID NO: 5:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (v) FRAGMENT TYPE: N-terminal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..6
      (D) OTHER INFORMATION:/label= AM35_N-term
   (xi) SEQUENCE DESCRIPTION: SEQ ID NO: 5:
   Asp Thr Thr Ile Thr Gln
   1 5
(2) INFORMATION FOR SEQ ID NO: 6:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (v) FRAGMENT TYPE: N-terminal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..14
      (D) OTHER INFORMATION:/label= AM50_1696_pep
   (xi) SEQUENCE DESCRIPTION: SEQ ID NO: 6:
   Ala Ala Ser Thr Leu Ala Glu Gly Ala Ala Gln His Asn Arg
   1               5                   10
(2) INFORMATION FOR SEQ ID NO: 7:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (v) FRAGMENT TYPE: internal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..10
      (D) OTHER INFORMATION:/label= AMSO_1697_pep
   (xi) SEQUENCE DESCRIPTION: SEQ ID NO: 7:
   Tyr Phe Gly Val Ala Ile Ala Ala Asn Arg
   1               5                   10
(2) INFORMATION FOR SEQ ID NO: 8:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (v) FRAGMENT TYPE: internal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..12
      (D) OTHER INFORMATION:/label= AM50_1698_pep
   (xi) SEQUENCE DESCRIPTION: SEQ ID NO: 8:
   Leu Asn Asp Ser Val Tyr Thr Asn Ile Ala Asn Arg
   1               5                   10
(2) INFORMATION FOR SEQ ID NO: 9:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (v) FRAGMENT TYPE: internal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..9
      (D) OTHER INFORMATION:/label= AM50_1699_pep
   (xi) SEQUENCE DESCRIPTION: SEQ ID NO: 9:
   Xaa Thr Gly Ile Thr Val Xaa Gly Val
   1               5
(2) INFORMATION FOR SEQ ID NO: 10:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (v) FRAGMENT TYPE: internal
   (vi) ORIGINAL SOURCE:
      (A) ORGANISM: Actinomadura flexuosa
      (B) STRAIN: DSM43186
   (ix) FEATURE:
      (A) NAME/KEY: Peptide
      (B) LOCATION:1..11
      (D) OTHER INFORMATION:/label= AM50_1704_pep
   (xi) SEQUENCE DESCRIPTION: SEQ ID NO: 10:
   Glu Phe Asn Ser Val Thr Ala Glu Asn Glu Met
    1               5                   10
(2) INFORMATION FOR SEQ ID NO: 11:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Trichoderma reesei and Actinomadura flexuosa
      (B) STRAIN: QM6a and DSM43186
   (ix) FEATURE:
      (A) NAME/KEY: CDS
      (B) LOCATION:1..18
      (D) OTHER INFORMATION:/product= "Mannanase-xylanase fusion protein" /note= "Partial sequence of the fusion in pALK945. Bases 1-5 are bases 1342-1346 of T.reesei man1 sequence, bases 6-9 synthetic and bases 10-18 are bases 432-440 of A.flexuosa AM35 sequence."
   (xi) SEQUENCE DESCRIPTION: SEQ ID NO: 11:
   TAT GGT CGC GAC ACC ACC                                            18
   Tyr Gly Arg Asp Thr Thr
       495
(2) INFORMATION FOR SEQ ID NO: 12:
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Tyr Gly Arg Asp Thr Thr
    1               5
(2) INFORMATION FOR SEQ ID NO: 13:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Trichoderma reesei and Actinomadura flexuosa
      (B) STRAIN: QM6a and DSM43186
   (ix) FEATURE:
      (A) NAME/KEY: CDS
      (B) LOCATION:1..27
      (D) OTHER INFORMATION:/product= "Mannanase-xylanase fusion protein" /note= "Partial sequence of the fusion in pALK948. Bases 1-5 are bases 1342-1346 of T.reesei man1 sequence, bases 6-18 are synthetic KEX2-linker, bases are 19-27 are bases 432-440 of A.flexuosa AM35 sequence."
   (xi) SEQUENCE DESCRIPTION: SEQ ID NO: 13:
   TAT GGT CGC GAC AAG CGC GAC ACC ACC                                27
   Tyr Gly Arg Asp Lys Arg Asp Thr Thr
               10                  15
(2) INFORMATION FOR SEQ ID NO: 14:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO: 14:
   Tyr Gly Arg Asp Lys Arg Asp Thr Thr
    1               5
(2) INFORMATION FOR SEQ ID NO: 15:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Trichoderma reesei and Actinomadura flexuosa
      (B) STRAIN: QM6a and DSM43186
   (ix) FEATURE:
      (A) NAME/KEY: CDS
      (B) LOCATION:1..39
      (D) OTHER INFORMATION:/product= "Mannanase-xylanase fusion protein" /note= "Partial sequence of the fusion in pALK1021. Bases 1-18 are bases 1342-1359 of T.reesei man1 sequence, and bases 19-39 are bases 432-452 of A.flexuosa AM35 sequence."
   (xi) SEQUENCE DESCRIPTION: SEQ ID NO: 15:
   TAT GGC CAG TGT GGA GGT GAC ACC ACC ATC ACC CAG AAC                    39
   Tyr Gly Gln Cys Gly Gly Asp Thr Thr Ile Thr Gln Asn
    10                 15                  20
(2) INFORMATION FOR SEQ ID NO: 16:
   (i) SEQUENCE CHARACTERISTICS:
      (A) LENGTH: 13 amino acids
      (B) TYPE: amino acid
      (D) TOPOLOGY: linear
   (ii) MOLECULE TYPE: protein
   (xi) SEQUENCE DESCRIPTION: SEQ ID NO: 16:
   Tyr Gly Gln Cys Gly Gly Asp Thr Thr Ile Thr Gln Asn
    1               5                  10
(2) INFORMATION FOR SEQ ID NO: 17:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Trichoderma reesei and Actinomadura flexuosa
      (B) STRAIN: QM6a and DSM43186
   (ix) FEATURE:
      (A) NAME/KEY: CDS
      (B) LOCATION:1..39
      (D) OTHER INFORMATION:/product= "Mannanase-xylanase fusion protein" /note= "Partial sequence of the fusion in pALK1022. Bases 1-18 are bases 1342-1359 of T.reesei man1 sequence, bases 19-30 are synthetic KEX2-linker, bases 31-39 are bases 432-440 of A.flexuosa AM35 sequence."
   (xi) SEQUENCE DESCRIPTION: SEQ ID NO: 17:
   TAT GGC CAG TGT GGA GGT CGC GAC AAG CGC GAC ACC ACC                39
   Tyr Gly Gln Cys Gly Gly Arg Asp Lys Arg Asp Thr Thr
        15                  20                  25
(2) INFORMATION FOR SEQ ID NO: 18:
   (i) SEQUENCE CHARACTERISTICS:
      (A) LENGTH: 13 amino acids
      (B) TYPE: amino acid
      (D) TOPOLOGY: linear
   (ii) MOLECULE TYPE: protein
   (xi) SEQUENCE DESCRIPTION: SEQ ID NO: 18:
   Tyr Gly Gln Cys Gly Gly Arg Asp Lys Arg Asp Thr Thr
     1               5                  10

## Claims

1. A recombinant expression vector for production of bacterial proteins secreted into the culture medium of a filamentous fungal host comprising a promoter operably linked to a DNA sequence, which encodes a signal sequence and a functional domain of a filamentous fungal secretable protein, which functional domain is a tail domain or a core and hinge domain, wherein the core domain contains an active site, the hinge domain a serine-threonine rich region and the tail a substrate binding site, and which is fused in frame with a DNA sequence encoding said bacterial protein, **characterized in that** the functional domain is a tail domain of a cellulase, or a core and hinge domain of a mannanase, wherein the cellulase or mannanase are homologous to the host *Trichoderma,* and the bacterial protein is an actinomycete xylanase.

2. The recombinant expression vector of claim 1, **characterized in that** the host is *Trichoderma reesei.*

3. The recombinant expression vector of claim 1, **characterized in that** the cellulase is a cellobiohydrolase or an endoglucanase.

4. The recombinant expression vector of claim 1 or claim 3, **characterized in that** the cellulase is CBHI.

5. The recombinant expression vector of claim 1 or claim 3, **characterized in that** the cellulase is CBHII.

6. The recombinant expression vector of claim 1 or claim 3, **characterized in that** the cellulase is EGI.

7. The recombinant expression vector of claim 1 or claim 3, **characterized in that** the cellulase is EGII.

8. The recombinant expression vector of claim 1, **characterized in that** the mannanase is MANI.

9. The recombinant expression vector of claim 1, **characterized in that** the promoter is a filamentous fungal promoter.

10. The recombinant expression vector of claim 1, **characterized in that** the promoter is a *Trichoderma reesei cbh1* promoter.

11. The recombinant expression vector of claim 1, **characterized in that** said expression vector comprises the plasmid constructs of Figure 17, wherein plasmid pALK1022 carries SEQ ID NO: 17, plasmid pALK945 carries SEQ ID NO: 11, plasmid pALK948 carries SEQ ID NO: 13, and plasmid pALK1021 carries SEQ ID NO: 15, or an equivalent thereof, which encodes the amino acid sequences SEQ ID NO: 12, SEQ ID NO: 14, SEQ ID NO: 16, and SEQ ID NO: 18, respectively.

12. The recombinant expression vector of claim 1, **characterized in that** the DNA sequence encoding an actinomycete xylanase comprises the DNA sequence SEQ ID NO: 1 encoding the amino acid sequence of SEQ ID NO: 2 or a DNA sequence encoding an amino acid sequence having at least 85 % identity at the amino acid level with SEQ ID NO: 2 and xylanolytic activity, or the DNA sequence SEQ ID NO: 3 encoding the amino acid sequence of SEQ ID NO: 4 or a DNA sequence encoding an amino acid sequence having at least 85 % identity at the amino acid level with SEQ ID NO: 4 and xylanolytic activity.

13. The recombinant expression vector of claim 1, **characterized in that** the DNA sequence encoding an actinomycete xylanase is comprised in a plasmid selected from the group consisting of pALK923 (DSM9322), pALK938 (DSM9899), pALK939 (DSM9900), pALK940 (DSM9901), pALK941 (DSM9902) and pALK1056 (DSM9903) that encodes *Actinomadura flexuosa* AM35 xylanase, or in a plasmid selected from a group consisting of plasmid pALK927 (DSM9447) or plasmid pALK928 (DSM9448) that encodes the *Actinomadura flexuosa* AM50 xylanase.

14. A filamentous fungus host, **characterized in that** it is transformed with at least one of the expression vectors of claims 1-13.

15. The use of the recombinant expression vector of claims 1-13 for the production of a preparation comprising an actinomycete xylanase, which is secreted into a culture medium during the cultivation of the filamentous fungus host.

16. The use according to claim 15, **characterized in that** the preparation is a spent culture medium essentially free from host cells.

17. The use of the preparation produced in claims 15 or 16 for enzyme-aided bleaching in a processing temperature 50-90 °C, or 70-80 °C.

18. The use of the preparation produced in claims 15 or 16 for enzymatic treatment of plant biomass, or pulp in paper making.

## Patentansprüche

1. Rekombinanter Expressionsvektor zur Herstellung bakterieller Proteine, sezerniert in das Kulturmedium eines Fadenpilz-Wirts, umfassend einen Promotor, funktionell verknüpft mit einer DNA-Sequenz, welche eine Signalsequenz und eine funktionale Domäne eines sezernierbaren Fadenpilz-Proteins codiert, wobei die funktionale Domäne eine Tail-Domäne oder eine Core- und Hinge-Domaine ist, wobei die Core-Domäne ein aktives Zentrum enthält, die Hinge-Domäne eine Serin-Threonin-reiche Region enthält und der Tail eine Substratbindungsstelle enthält, und welche im Leserahmen fusioniert ist mit einer DNA-Sequenz, codierend das bakterielle Protein, **dadurch gekennzeichnet, dass** die funktionale Domäne eine Tail-Domäne einer Cellulase oder eine Core- und Hinge-Domäne einer Mannanase ist, wobei die Cellulase oder Mannanase zu dem Wirt *Trichoderma* homolog sind und das bakterielle Protein eine Actinomycetenxylanase ist.

2. Rekombinanter Expressionsvektor nach Anspruch 1, **dadurch gekennzeichnet, dass** der Wirt *Trichoderma reesei* ist.

3. Rekombinanter Expressionsvektor nach Anspruch 1, **dadurch gekennzeichnet, dass** die Cellulose eine Cellobiohydrolase oder eine Endoglucanase ist.

4. Rekombinanter Expressionsvektor nach Anspruch 1 oder Anspruch 3, **dadurch gekennzeichnet, dass** die Cellulase CBHI ist.

5. Rekombinanter Expressionsvektor nach Anspruch 1 oder Anspruch 3, **dadurch gekennzeichnet, dass** die Cellulase CBHII ist.

6. Rekombinanter Expressionsvektor nach Anspruch 1 oder Anspruch 3, **dadurch gekennzeichnet, dass** die Cellulase EGI ist.

7. Rekombinanter Expressionsvektor nach Anspruch 1 oder Anspruch 3, **dadurch gekennzeichnet, dass** die Cellulase EGII ist.

8. Rekombinanter Expressionsvektor nach Anspruch 1, **dadurch gekennzeichnet, dass** die Mannanase MANI ist.

9. Rekombinanter Expressionsvektor nach Anspruch 1, **dadurch gekennzeichnet, dass** der Promotor ein Fadenpilz-Promotor ist.

10. Rekombinanter Expressionsvektor nach Anspruch 1, **dadurch gekennzeichnet, dass** der Promotor ein *Trichoderma* reesei-cbhl-Promotor ist.

11. Rekombinanter Expressionsvektor nach Anspruch 1, **dadurch gekennzeichnet, dass** der Expressionsvektor die Plasmidkonstrukte von Figur 17 umfasst, wobei Plasmid pALK1022 SEQ ID NO: 17 trägt, Plasmid pALK945 SEQ ID NO: 11 trägt, Plasmid pALK948 SEQ ID NO: 12 trägt und Plasmid pALK1021 SEQ ID NO: 15 trägt, oder ein Äquivalent davon, welches die Aminosäuresequenzen SEQ ID NO: 12, SEQ ID NO: 14, SEQ ID NO: 16 bzw. SEQ ID NO: 18 codiert.

12. Rekombinanter Expressionsvektor nach Anspruch 1, **dadurch gekennzeichnet, dass** die DNA-Sequenz, codierend eine Actinomycetenxylanase, die DNA-Sequenz SEQ ID NO: 1, codierend die Aminosäuresequenz von SEQ ID NO: 2, oder eine DNA-Sequenz, codierend eine Aminosäuresequenz, die wenigstens 85% Identität auf dem Aminosäurelevel mit SEQ ID NO: 2 aufweist und xylanolytische Aktivität aufweist, oder die DNA-Sequenz SEQ ID NO: 3, codierend die Aminosäuresequenz von SEQ ID NO: 4, oder eine DNA-Sequenz, codierend eine Aminosäuresequenz, die wenigstens 85% Identität auf dem Aminosäurelevel mit SEQ ID NO: 4 aufweist und xylanolytische Aktivität aufweist, umfasst.

13. Rekombinanter Expressionsvektor nach Anspruch 1, **dadurch gekennzeichnet, dass** die DNA-Sequenz, codierend eine Actinomycetenxylanase, in einem Plasmid enthalten ist, das ausgewählt ist aus der Gruppe, bestehend aus pALK923 (DSM9322), pALK938 (DSM9899), pALK939 (DSM9900), pALK940 (DSM9901), pALK941 (DSM9902) und pALK1056 (DSM9903), das Ac*tinomadura flexuosa*-AM35-Xylanase codiert, oder in einem Plasmid, das ausgewählt ist aus einer Gruppe, bestehend aus Plasmid pALK927 (DSM9447) oder Plasmid pALK928 (DSM9448), das die *Actinomadura flexuosa*-AM50-Xylanase codiert.

14. Fadenpilz-Wirt, **dadurch gekennzeichnet, dass** er mit wenigstens einem der Expressionsvektoren der Ansprüche 1-13 transformiert ist.

15. Verwendung des rekombinanten Expressionsvektors nach Anspruch 1-13 zur Herstellung einer Zubereitung, umfassend eine Actinomycetenxylanase, welche während der Kultivierung des Fadenpilz-Wirts in ein Kulturmedium sezerniert wird.

16. Verwendung nach Anspruch 15, **dadurch gekennzeichnet, dass** die Zubereitung ein verbrauchtes Kulturmedium, im Wesentlichen frei von Wirtszellen, ist.

17. Verwendung der Zubereitung, hergestellt in Anspruch 15 oder 16, zum Enzym-unterstützten Bleichen bei einer Prozessierungstemperatur von 50-90°C oder 70-80°C.

18. Verwendung der Zubereitung, hergestellt in Anspruch 15 oder 16, zur enzymatischen Behandlung von Pflanzenbiomasse, oder Pulpe bei der Papierherstellung.

## Revendications

1. Vecteur d'expression recombinant utilisé pour la production de protéines bactériennes sécrétées dans le milieu de culture d'un hôte de type champignon filamenteux comprenant un promoteur en liaison fonctionnelle avec une séquence d'ADN, codant pour une séquence signal et un domaine fonctionnel d'une protéine sécrétable par un champignon filamenteux, ledit domaine fonctionnel étant un domaine de type terminal ou de type central et charnière, le domaine central contenant un site actif, le domaine charnière contenant une région riche en sérine-thréonine et le domaine terminal contenant un site de liaison du substrat, et ledit domaine fonctionnel étant fusionné en phase avec une séquence d'ADN codant pour ladite protéine bactérienne, **caractérisé en ce que** le domaine fonctionnel est un domaine de type terminal d'une cellulase, ou un domaine de type central et charnière d'une mannanase, la cellulase ou la mannanase étant homologues à celles du *Trichoderma* hôte, et la protéine bactérienne étant une xylanase d'actinomycète.

2. Vecteur d'expression recombinant selon la revendication 1, **caractérisé en ce que** l'hôte est *Trichoderma reesei.*

3. Vecteur d'expression recombinant selon la revendication 1, **caractérisé en ce que** la cellulase est une cellobiohydrolase ou une endoglucanase.

4. Vecteur d'expression recombinant selon la revendication 1 ou la revendication 3, **caractérisé en que** la cellulase est la CBHI.

5. Vecteur d'expression recombinant selon la revendication 1 ou la revendication 3, **caractérisé en ce que** la cellulase est la CBHII.

6. Vecteur d'expression recombinant selon la revendication 1 ou la revendication 3, **caractérisé en que** la cellulase est l'EGI.

7. Vecteur d'expression recombinant selon la revendication 1 ou la revendication 3, **caractérisé en ce que** la cellulase est l'EGII.

8. Vecteur d'expression recombinant selon la revendication 1, **caractérisé en ce que** la mannanase est la MANI.

9. Vecteur d'expression recombinant selon la revendication 1, **caractérisé en ce que** le promoteur est un promoteur de champignon filamenteux.

10. Vecteur d'expression recombinant selon la revendication 1, **caractérisé en ce que** le promoteur est un promoteur du gène *cbh1* de *Trichoderma reesei.*

11. Vecteur d'expression recombinant selon la revendication 1, **caractérisé en ce que** ledit vecteur d'expression comprend les constructions plasmidiques de la figure 17, le plasmide pALK1022 étant porteur de SEQ ID NO : 17, le plasmide pALK945 de SEQ ID NO : 11, le plasmide pALK948 de SEQ ID NO : 13 et le plasmide pALK1021 de SEQ ID NO : 15, ou d'un équivalent de celles-ci, codant, respectivement, pour les séquences d'acides aminés SEQ ID NO : 12, SEQ ID NO : 14, SEQ ID NO : 16 et SEQ ID NO : 18.

12. Vecteur d'expression recombinant selon la revendication 1, **caractérisé en ce que** la séquence d'ADN codant pour une xylanase d'actinomycète comprend la séquence d'ADN SEQ ID NO : 1 codant pour la séquence d'acides aminés SEQ ID NO : 2 ou une séquence d'ADN codant pour une séquence d'acides aminés présentant au moins 85 % d'identité, au niveau de ses acides aminés, avec SEQ ID NO : 2 et ayant une activité xylanolytique, ou la séquence d'ADN SEQ ID NO : 3 codant pour la séquence d'acides aminés SEQ ID NO : 4 ou pour une séquence d'ADN codant pour une séquence d'acides aminés présentant au moins 85 % d'identité, au niveau de ses acides aminés, avec SEQ ID NO : 4 et ayant une activité xylanolytique.

13. Vecteur d'expression recombinant selon la revendication 1, **caractérisé en ce que** la séquence d'ADN codant pour une xylanase d'actinomycète est contenue dans un plasmide, choisi dans le groupe constitué de pALK923 (DSM9322), pALK938 (DSM9899), pALK939 (DSM9900), pALK940 (DSM9901), pALK941 (DSM9902) et pALK1056 (DSM9903), codant pour la xylanase AM35 d'*Actinomadura flexuosa,* ou dans un plasmide, choisi dans le groupe constitué du plasmide pALK927 (DSM9447) ou du plasmide pALK928 (DSM9448), codant pour la xylanase AM50 d*'Actinomadura flexuosa.*

14. Hôte de type champignon filamenteux, **caractérisé en ce qu'**il est transformé au moyen d'au moins l'un des vecteurs d'expression des revendications 1 à 13.

15. Utilisation du vecteur d'expression recombinant des revendications 1 à 13 pour l'obtention d'une préparation comprenant une xylanase d'actinomycète, qui est sécrétée dans un milieu de culture lors de la culture de l'hôte de type champignon filamenteux.

16. Utilisation selon la revendication 15, **caractérisée en ce que** la préparation correspond à un milieu de culture épuisé essentiellement exempt de cellules hôtes.

17. Utilisation de la préparation obtenue selon les revendications 15 ou 16 pour le blanchiment enzymatique à une température de traitement de 50 à 90 °C ou de 70 à 80 °C.

18. Utilisation de la préparation obtenue selon les revendications 15 ou 16 pour le traitement enzymatique de la biomasse végétale, ou de la pâte à papier dans le cadre de la fabrication du papier.
